# EUROPEAN PATENT SPECIFICATION

(11) **EP 0 466 137 B1**
(45) Date of publication and mention of the grant of the patent: **17.04.1996**
(21) Application number: 91111506.1
(22) Date of filing: 10.07.1991
(51) Int. Cl.: C07D 251/70, C08K 5/3492, C07F 9/38, C07F 9/6574, C07F 9/48, C07F 9/142

(54) **Salts of triazine derivatives with oxygenated acids of phosphorus and their use in self-extinguishing polymeric compositions**
Salze von Triazinderivaten mit oxygenierter Phosphorsäure und ihre Verwendung in selbstlöschenden Polymerzusammensetzungen
Sels de dérivés de triazine avec des acides oxygénés de phosphore et leur utilisation dans des compositions polymériques anti-extinctrices

(30) Priority: 11.07.1990 IT 2091990
(43) Date of publication of application: 15.01.1992
(73) Proprietor: MINISTERO DELL' UNIVERSITA' E DELLA RICERCA SCIENTIFICA E TECNOLOGICA, I-00196 Roma (IT)
(72) Inventor: Cipolli, Roberto, I-28100 Novara (IT); Masarati, Enrico, I-29010 Castelnuovo Valtidone, Piacenzy (IT); Nucida, Gilberto, I-20098 San Giuliano Milanese, Milan (IT); Oriani, Roberto, I-20137 Milan (IT); Pirozzi, Mario, I-20097 San Donato Milanese, Milan (IT)
(74) Representative: Barz, Peter, Dr.

(56) References cited:
- EP-A- 0 149 480
- EP-A- 0 286 478
- EP-A- 0 415 371
- US-A- 4 201 705

## Description

The present invention relates to salts of triazine derivatives with oxygenated acids of phosphorus. More specifically the present invention relates to salts of triazine derivatives with oxygenated acids of phosphorus and their use in the preparation of self-extinguishing polymeric compositions based on thermoplastic polymers or polymers having elastomeric properties, particularly olefinic polymers or copolymers.

Several methods of reducing or eliminating the combustibility of polymers are known in the art. Some of said methods are based on the use of metal compounds, in particular on antimony, bismuth or arsenic, in combination with partially halogenated, thermally unstable organic compounds, such as chlorinated paraffin waxes.

Other methods are based on the use of substances capable of yielding intumescence. The formulations of the intumescent type generally are composed of the polymer and at least three main additives, i.e., an essentially phosphorus-containing additive, whose purpose is to form, during the combustion, an impermeable, semi-solid and vitreous layer essentially composed of polyphosphoric acid and to activate the process of formation of intumescence; a second additive, containing nitrogen, which is to serve as foaming agent; and a third, carbon-containing additive, which acts as a carbon donor in order to allow an insulating cellular carbonaceous layer ("char") to be formed between the polymer and the flame.

Examples of intumescent formulations of said type may be found in US-A-3 810 862 (Phillips Petroleum Co.; based on melamine, pentaerythritol and ammonium polyphosphate); US-A-4 727 102 (Vamp S.r.l.; based on melamine cyanurate, a hydroxyalkyl derivative of isocyanuric acid and ammonium polyphosphate); and WO-85/05626 (Plascoat U.K. Limited; on the basis of various phosphorus and nitrogen compounds among which, in particular, a combination of melamine phosphate, pentaerythritol and ammonium polyphosphate may be mentioned).

In more recent formulations, together with an organic or inorganic phosphorus compound, a nitrogen-containing organic compound was used, generally consisting of an aminoplastic resin obtained by means of condensation of urea, melamine or dicyandiamide with formaldehyde.

Examples of formulations containing two additives are those described in US-A-4 504 610 (Montedison S.p.A.; based on oligomeric derivates of 1,3,5-triazine and ammonium polyphosphate) and EP-A-14 463 (Montedison S.p.A.; based on organic compounds selected from benzylguanamine and reaction products of aldehydes and several cyclic nitrogen compounds, in particular, benzylguanamine-formaldehyde copolymers, and ammonium polyphosphate).

Self-extinguishing compositions can also be obtained by using single-component additives which, in their organic molecule, contain both nitrogen and phosphorus atoms, as disclosed in US-A-4 201 705 (Borg-Wagner Corp.).

These intumescent flame retardant systems endow the polymers to which they are added with the property of forming a carbonaceous residue when they burn or are exposed to a flame. This kind of flame-retardant system offers numerous advantages, i.e., absence of corrosion phenomena in the machinery in which the polymers are processed, a lower emission of smokes as compared with systems containing metal compounds and halogenated hydrocarbons and, above all, the possibility of endowing the polymers with satisfactory flame-proof properties with a smaller amount of total additive and, therefore, without excessively impairing the mechanical properties thereof.

It has now, surprisingly, been found that it is possible to confer satisfactory antiflame (flame-retardant) properties to the above polymers by using mono-component additives, resulting in polymeric compositions free of ammonium phosphate or amine phosphate, or to impart very good antiflame properties to said polymers by using, together with the above additives, a quantity of ammonium phosphate and/or amine phosphate which is much lower than that used in the prior art.

Furthermore, it has been found that it is possible to obtain said very good results by using phosphorus-nitrogen compounds having a very simple structure, i.e., based on derivatives of 2,4,6-triamino-1,3,5-triazine salified with a phosphorus-containing acid. Said salts also show a good heat stability and, therefore, retain a high activity as flame-retardants, also when the polymeric compositions containing them are heat-processed.

As mentioned above, salified derivatives of 2,4,6-triamino-1,3,5-triazine (melamine) containing phosphorus are known in the art which are useable as co-additives for self-extinguishing compositions in many polymeric matrices, mainly polyolefinic matrices (see also EP-A-286 478). These compounds, such as, for instance, melamine phosphate and melamine pyrophosphate, require the presence of other additives, in particular, a component containing the carbon necessary for the char formation, such as a polyol (pentaerythritol, dipentaerythritol, tripentaerythritol) in order to be effective flame-retardants.

In contrast thereto, the compounds used according to the present invention are employed in formulations of polymeric materials as flame-retardant additives of the intumescent type, i.e., they are "char-forming" without the aid of other co-additives.

Furthermore, the compositions of the present invention show the advantage of giving a very moderate and not darkening smoke emission.

The present invention, consequently, provides salts of general formula (I): 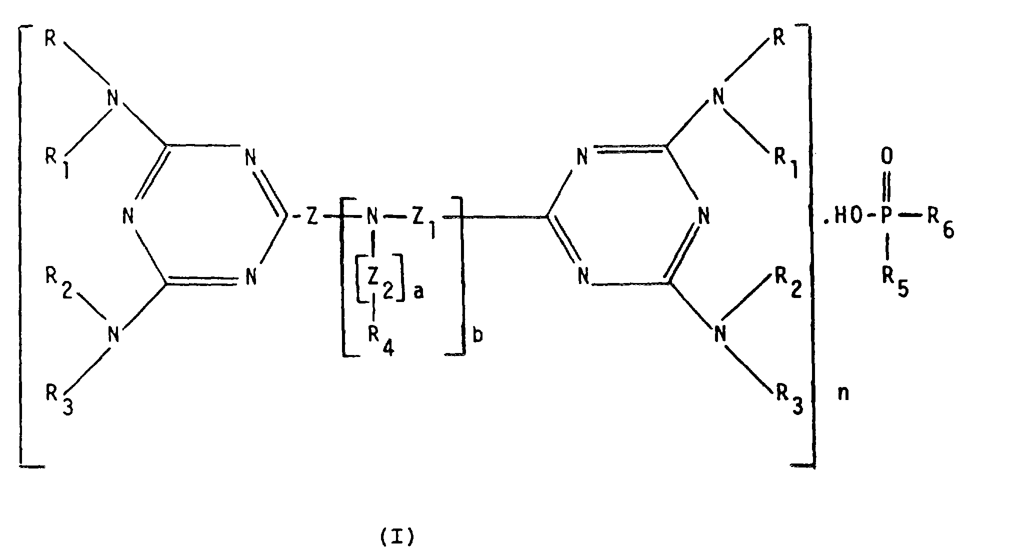 wherein
the groups R, R₁, R₂ and R₃, alike or different from each other and having the same or different meanings for each triazine ring, are selected from H; C₁-C₁₈ (and, preferably, C₁-C₈) alkyl; C₂-C₈ (and, preferably, C₂-C₄) alkenyl; C₆-C₁₆ (and, preferably, C₆-C₈) cycloalkyl or alkyl-cycloalkyl, optionally substituted with one or more hydroxy or C₁-C₄ hydroxyalkyl groups (e.g., hydroxymethyl or hydroxyethyl); provided that when the bivalent or polyvalent radicals defined below belong to those of general formulae (III) and (XII), respectively, the radical pair R₅/R₆, defined below, is different from H/OH; 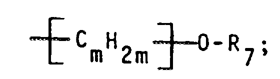 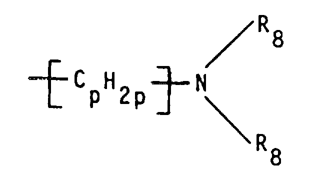 wherein
m = integer of from 2 to 8, preferably from 2 to 4;
p = integer of from 2 to 6; preferably from 2 to 4;
R₇ = H; C₁-C₈ (and, preferably, C₁-C₄) alkyl; C₂-C₆ (and, preferably, C₂-C₄) alkenyl;

   ⁅C_{q}H_{2q}⁆O-R₉,

   q being an integer of from 1 to 4 (e.g., 1 or 2) and R₉ being H or a C₁-C₄ alkyl group; C₆-C₁₂ (and, preferably, C₆-C₈) cycloalkyl or alkyl-cycloalkyl; the radicals R₈, alike or different from each other, are selected from H; C₁-C₈ (and, preferably, C₁-C₄) alkyl; C₂-C₆ (and, preferably, C₂-C₄) alkenyl; C₆-C₁₂ (and, preferably, C₆-C₈) cycloalkyl or alkylcycloalkyl; and C₁-C₄ hydroxyalkyl;
provided that when the bivalent or polyvalent radicals defined below belong to those of general formulae (III) and (XII), respectively, the radical pair R₅/R₆, defined below, is different from H/OH; or the moiety N(R₈)₂ is replaced by an N-heterocyclic radical as specified below which is linked to the alkyl chain through the nitrogen atom;
or in the general formula (I) at least one of the groups NRR₁ and NR₂R₃ is replaced by an N-heterocyclic radical as specified below which is linked to the triazine ring through the nitrogen atom;
a is 0 or 1
b is 0 or an integer of from 1 to 5;
R₄ is hydrogen or a group of general formula 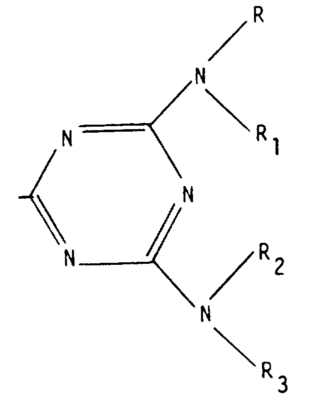 and its meaning can be different in each repeating unit;
when b is 0:
Z is a divalent radical of one of the following formulae: 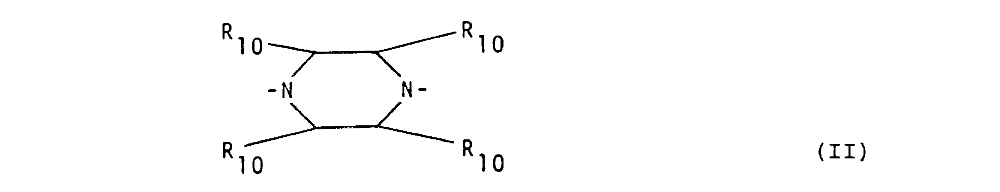 where the groups R₁₀, alike or different from each other, are hydrogen or C₁-C₄ alkyl; 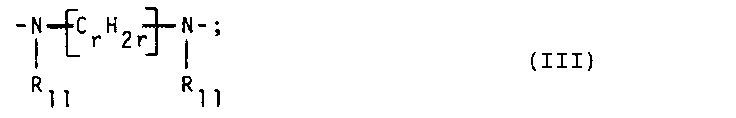 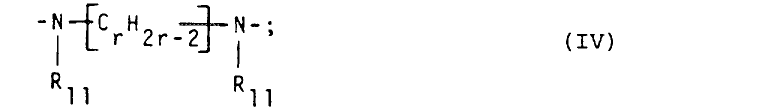 where r is an integer of from 2 to 14 and R₁₁ is hydrogen; C₁-C₄ alkyl; C₂-C₆ alkenyl; or C₁-C₄ hydroxyalkyl; 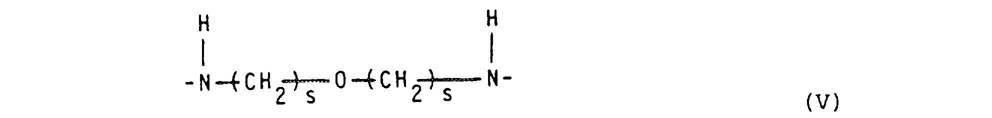 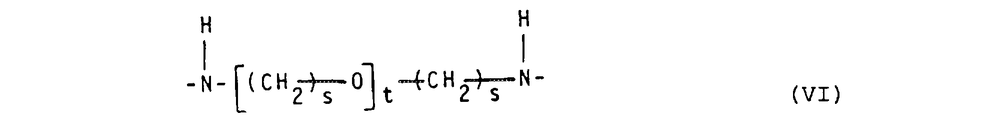 where s is an integer of from 2 to 5 and t is an integer of from 1 to 3; 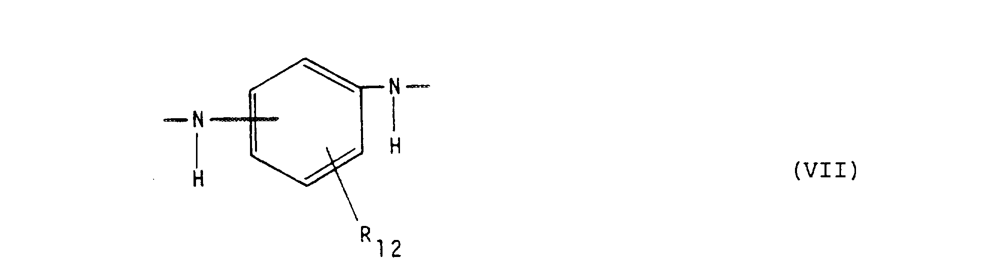 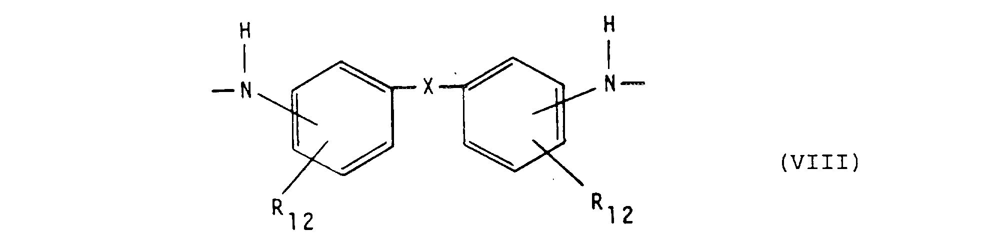 where:
X represents a C-C bond; O; S; S-S; SO; SO₂; NH; NHSO₂; NHCO; N=N; or CH₂; R₁₂ is hydrogen; hydroxy;
   C₁-C₄ alkyl; or C₁-C₄ alkoxy; 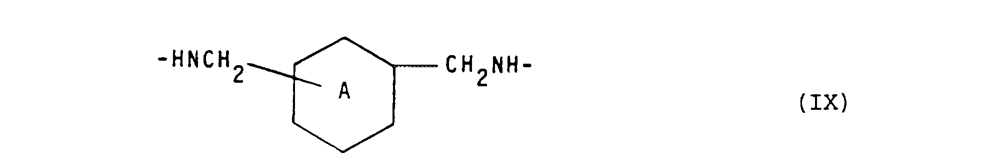 where A is a saturated or unsaturated ring; 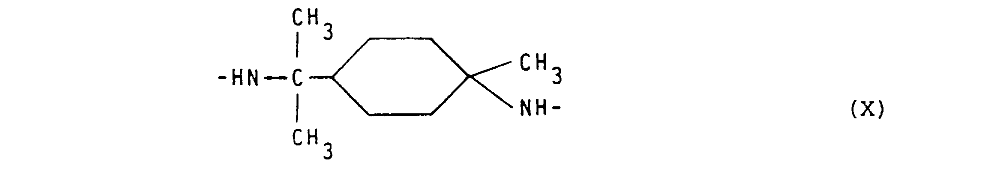 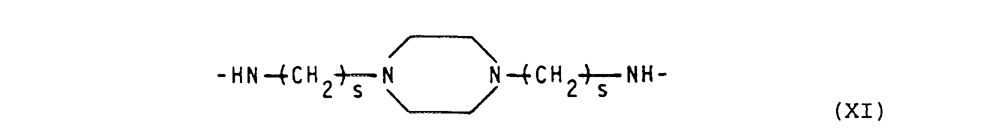
where s has the meaning previously defined;
when b is an integer of from 1 to 5:
the group 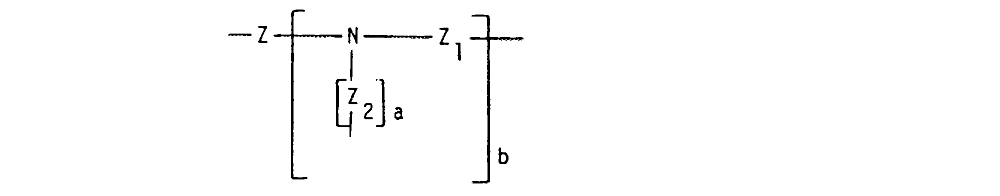 is a polyvalent radical selected from one of the following formulae: 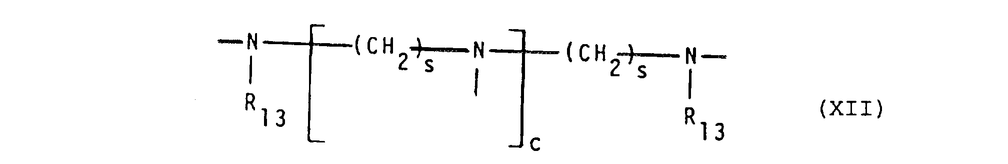 where:
R₁₃ is hydrogen or C₁-C₄ alkyl;
c is an integer of from 1 to 5;
the subscripts s, alike or different from each other, have the meaning defined above; 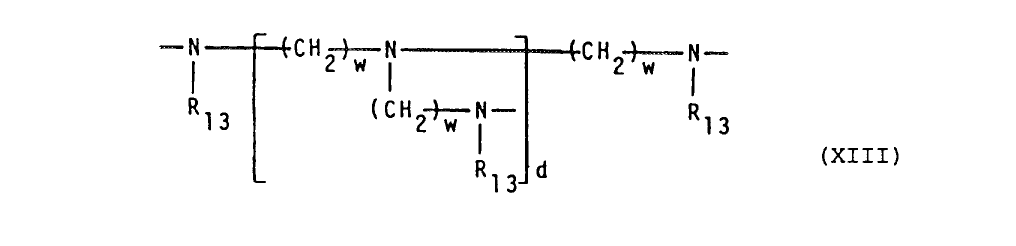 where:
R₁₃ has the meaning previously defined;
w is an integer of from 2 to 4;
d is 1 or 2;
n is a number of from greater than 0 to 3, particularly from 0.2 to 2.5;
R₅ is selected from H; OH; C₁-C₈ (preferably C₁-C₄) alkoxy; C₆-C₁₂ (preferably C₆-C₁₀) aryloxy, optionally substituted by a C₁-C₈ (preferably C₁-C₄) alkyl group; C₇-C₁₂ aralkyl, optionally substituted by a C₁-C₄ alkyl group; C₁-C₄ alkyl, optionally substituted by a carboxylic group; and C₆-C₁₂ (preferably C₆-C₁₀) aryl;
R₆ is selected from H; OH; C₁-C₈ (preferably C₁-C₄) alkoxy; C₆-C₁₂ (preferably C₆-C₁₀) aryloxy; C₁-C₄ alkyl; C₆-C₁₂ (preferably C₆-C₁₀) aryl;
a group of formula 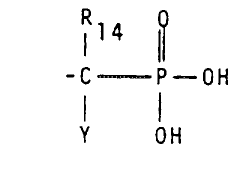 wherein:
R₁₄ is H or C₁-C₁₂ (and, preferably, C₁-C₆) alkyl; and
Y is OH or R₁₄;
a group of formula 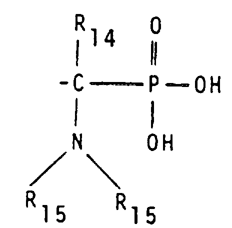 wherein:
R₁₄ has the meaning previously defined, and the radicals
R₁₅, alike or different from each other, are H or C₁-C₄ alkyl (e.g., methyl or ethyl);
or the moiety N(R₁₅)₂ is replaced by an N-heterocyclic radical which, optionally, contains another heteroatom (preferably selected from O, N and S) and is linked to the carbon atom through the nitrogen atom;
a group of formula 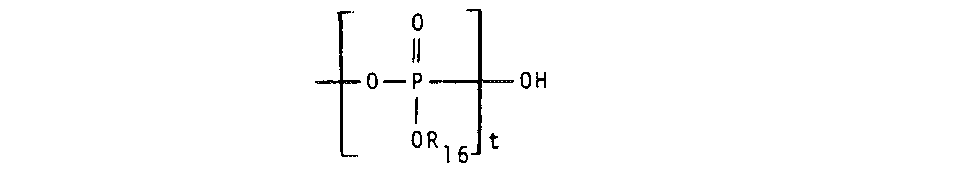 wherein:
R₁₆ is H or C₁-C₈ (and, preferably, C₁-C₄) alkyl; and t is an integer of from 1 to 3 (e.g., 1 or 2);
a group of formula: 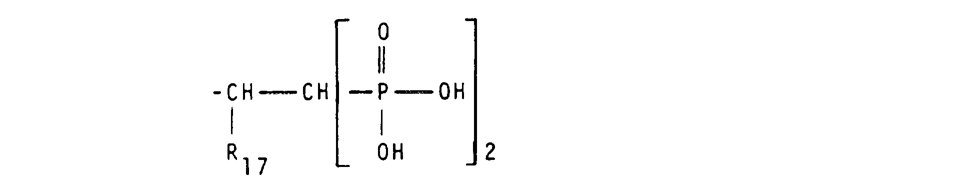 wherein:
R₁₇ is H or OH;
and groups of formulae: 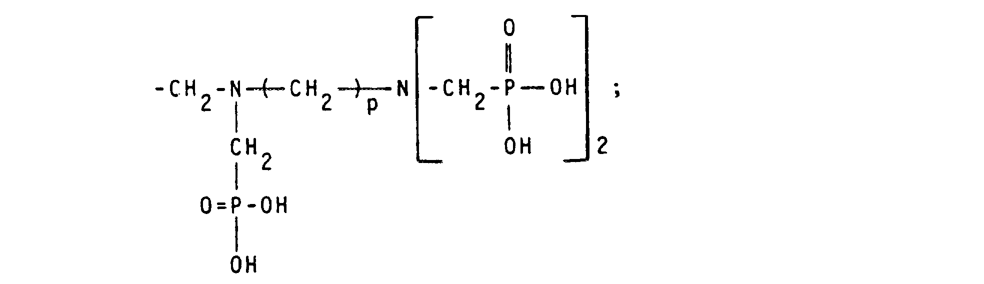 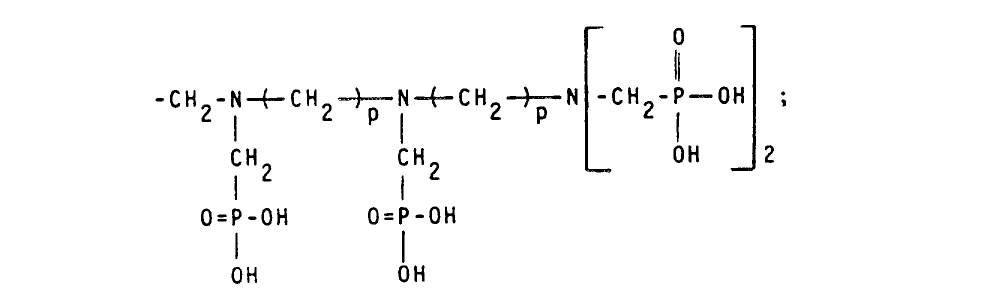 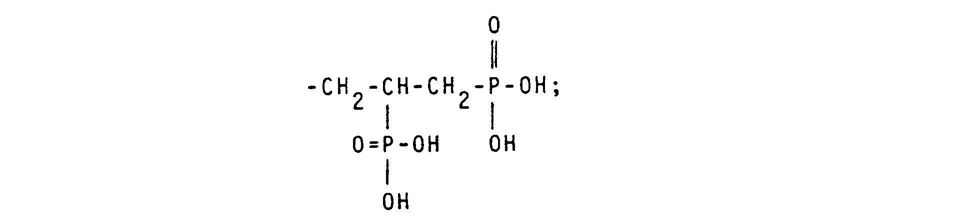 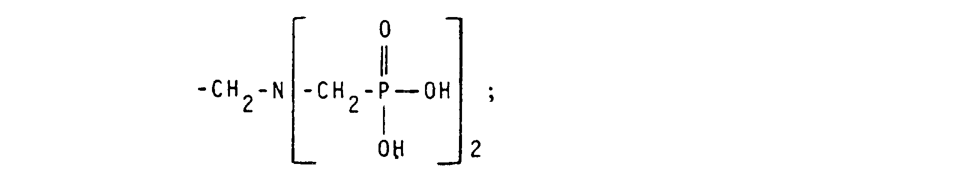 wherein:
p is an integer of from 2 to 6 (e.g., from 2 to 4); or
R₅ and R₆ together form a cyclic structure of one of the following formulae: 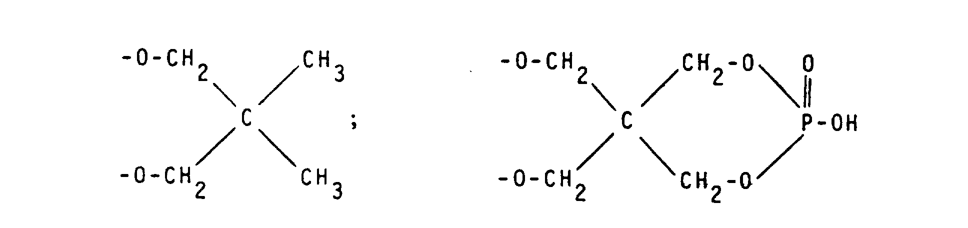 Compounds having an asymmetric structure, in the sense that any of the radicals R, R₁, R₂ and R₃ has a different meaning in different triazine rings, are also comprised by general formula (I).

Specific examples of the radicals R, R₁, R₂ and R₃ in formula (I) are
methyl; ethyl; propyl; isopropyl; n-butyl; isobutyl; tert-butyl; n-pentyl; isopentyl; n-hexyl; tert-hexyl;n-octyl; tert-octyl; decyl; dodecyl; octadecyl; ethenyl; propenyl; butenyl; isobutenyl; hexenyl; octenyl; cyclohexyl; propylcyclohexyl; butylcyclohexyl; decylcyclohexyl; hydroxycyclohexyl; hydroxyethylcyclohexyl; 2-hydroxyethyl; 2-hydroxypropyl; 3-hydroxypropyl; 3-hydroxybutyl; 4-hydroxybutyl; 3-hydroxypentyl; 5-hydroxypentyl; 6-hydroxyhexyl; 3-hydroxy-2,5-dimethylhexyl; 7-hydroxyheptyl; 7-hydroxyoctyl; 2-methoxyethyl; 2-methoxypropyl; 3-methoxypropyl; 4-methoxybutyl; 6-methoxyhexyl; 7-methoxyheptyl; 7-methoxyoctyl; 2-ethoxyethyl; 3-ethoxypropyl; 4-ethoxybutyl; 3-propoxypropyl; 3-butoxypropyl; 4-butoxybutyl; 4-isobutoxybutyl; 5-propoxypentyl; 2-cyclohexyloxyethyl; 2-ethenyloxyethyl; 2-(N,N-dimethylamino)ethyl; 3-(N,N-dimethylamino)propyl; 4-(N,N-dimethylamino)butyl; 5-(N,N-dimethylamino)pentyl; 4-(N,N-diethylamino)butyl; 5-(N,N-diethylamino)pentyl; 5-(N,N-diisopropylamino)pentyl; 3-(N-ethylamino)propyl; 4-(N-methylamino)butyl; 4-(N,N-dipropylamino)butyl; 2-(N,N-diisopropylamino)ethyl; 6-(N-hexenylamino)hexyl; 2-(N-ethenylamino)ethyl; 2-(N-cyclohexylamino)ethyl; 2-(N-2-hydroxyethyloamino)ethyl; 2-(2-hydroxyethoxy)ethyl; 2-(2-methoxyethoxy)ethyl; 6-(N-propylamino)hexyl, etc.

The heterocyclic radicals which can replace the moieties NRR₁ and NR₂R₃ are aziridinyl; pyrrolidinyl; piperidinyl; morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl, 2-ethylpiperazinyl; and 2,5-diethylpiperazinyl.

The heterocyclic radicals which can replace the moiety N(R₈)₂ are aziridinyl; pyrrolidinyl; piperidinyl; morpholinyl; thiomorpholinyl; piperazinyl; 4-methylpiperazinyl; and 4-ethylpiperazinyl.

Specific examples of divalent -Z- radicals are those obtained from the following diamino compounds by eliminating one hydrogen atom from any amino group:
piperazine; 2-methylpiperazine; 2,5-dimethylpiperazine; 2,3,5,6-tetramethylpiperazine; 2-ethylpiperazine; 2,5-diethylpiperazine; 1,2-diaminoethane; 1,3-diaminopropane; 1,4-diaminobutane; 1,5-diaminopentane; 1,6-diaminohexane; 1,8-diaminooctane; 1,10-diaminodecane; 1,12-diaminododecane; N,N'-dimethyl-1,2-diaminoethane; N-methyl-1,3-diaminopropane; N-ethyl-1,2-diaminoethane; N-isopropyl-1,2-diaminoethane; N-(2-hydroxyethyl)-1,2-diaminoethane; N,N'-bis(2-hydroxyethyl)-1,2-diaminoethane; N-(2-hydroxyethyl)-1,3-diaminopropane; N-hexenyl-1,6-diaminohexane; N,N'-diethyl-1,4-diamino-2-butene; 2,5-diamino-3-hexene; 2-aminoethylether; (2-aminoethoxy)methylether; 1,2-bis(2-aminoethoxy)ethane; 1,3-diaminobenzene; 1,4-diaminobenzene; 2,4-diaminotoluene; 2,4-diaminoanisole; 2,4-diaminophenol; 4-aminophenylether; 4,4'-methylene dianiline; 4,4'-diaminobenzanilide; 3-aminophenylsulfone; 4-aminophenylsulfone; 4-aminophenylsulfoxide; 4-aminophenyldisulfide; 1,3-bis(aminomethyl)benzene; 1,4-bis(aminomethyl)benzene; 1,3-bis(aminomethyl)cyclohexane; 1,8-diamino-p-menthane; 1,4-bis(2-aminoethyl)piperazine; 1,4-bis(3-aminopropyl)piperazine; 1,4-bis(4-aminobutyl)piperazine; 1,4-bis(5-aminopentyl)piperazine; etc.

Specific examples of polyvalent radicals: 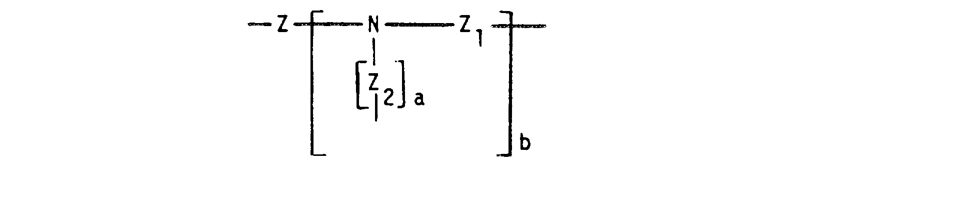 are those obtained from the following polyamino compounds by eliminating one hydrogen atom from any reacted amino group:
bis(2-aminoethyl)amine; bis(3-aminopropyl)amine; bis(4-aminobutyl)amine; bis(5-aminopentyl)amine; bis[2(N-methylamino)ethyl]amine; 2-N-butyl-bis(2-aminoethyl)amine; bis[3-(N-methylamino)propyl]amine; N-(3-aminopropyl)-1,4-diaminobutane; N-(3-aminopropyl)-1,5-diaminopentane; N-(4-aminobutyl)-1,5-diaminopentane; tris(2-aminoethyl)amine; tris(3-aminopropyl)amine; tris(4-aminobutyl)amine; tris[2-(N-ethylamino)ethyl]amine; N,N'-bis(3-aminopropyl)-1,3-diaminopropane; N,N'-bis(2-aminoethyl)-1,3-diaminopropane; N,N'-bis(3-aminopropyl)-1,2-diaminoethane; N,N'-bis(3-aminopropyl)-1,4-diaminobutane; bis[2-(2-aminoethyl)aminoethyl]amine; N,N'-bis[2-(2-aminoethyl)aminoethyl]-1,2-diaminoethane; N,N'-bis[3-(2-aminoethyl)aminopropyl]-1,2-diaminoethane; N,N,N',N'-tetrakis(2-aminoethyl)-1,2-diaminoethane; etc.

Specific examples of phosphorus-containing acids are hypophosphorous acid; phosphorous acid; phosphoric acid; pyrophosphoric acid; tripolyphosphoric acid; ethane-1,1,2-triphosphonic acid; 2-hydroxyethane-1,1,2-triphosphonic acid; propane-1,2,3-triphosphonic acid; isopropylphosphoric acid; n-butylphosphoric acid; di-isopropylphosphoric acid; d-n-butylphosphoric acid; di-n-pentylphosphoric acid; isooctylphosphoric acid; hexylphosphoric acid; 2-ethylhexylphosphoric acid; ethylphosphoric acid; methylphosphonic acid; ethylphosphonic acid; n-propylphosphonic acid; n-butylphosphonic acid; aminomethylphosphonic acid; phenylphosphoric acid; phenylphosphonic acid; phenylphosphinic acid; di-n-butylpyrophosphoric acid; di(2-ethylhexyl)pyrophosphoric acid; octylphenylphosphoric acid; 2-methylbenzylphosphonic acid; 1-aminoethane-1,1-diphosphonic acid; 1-hydroxyethane-1,1-diphosphonic acid; 1-hydroxydodecane-1,1-diphosphonic acid; 1-(N-methylamino)ethane-1,1-diphosphonic acid; N,N-dimethylaminomethane-1,1-diphosphonic acid; N-butylaminomethane-1,1-diphosphonic acid; phosphonacetic acid; 2-phosphonopropionic acid; 3-phosphonopropionic acid; 2-phosphonobutyric acid; 4-phosphonobutyric acid; 2-hydroxy-5,5-dimethyl-2-oxo-1,3,2-dioxophosphorinane; 3,9-dihydroxy-2,4,8,10-tetroxo-3,9-diphosphaspiro[5.5]undecano-3,9-dioxide; amino-tris(methylene phosphonic) acid; ethylene diaminotetra(methylene phosphonic) acid; hexamethylene diaminotetra(methylene phosphonic) acid; diethylene triaminopenta(methylene phosphonic) acid; etc.

Specific compounds included in formula (I) are indicated in the examples which follow.

The salts of general formula (I) can be synthesized, e.g., by reacting n moles of a derivative of
2,4,6-triamino-1,3,5-triazine of general formula (XIV): 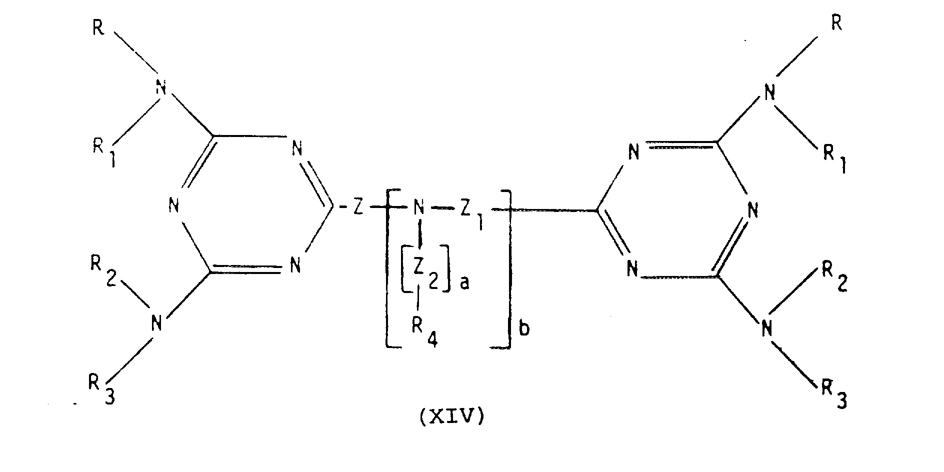 wherein n and the groups R, R₁, R₂, R₃ and R₄ and the radical: 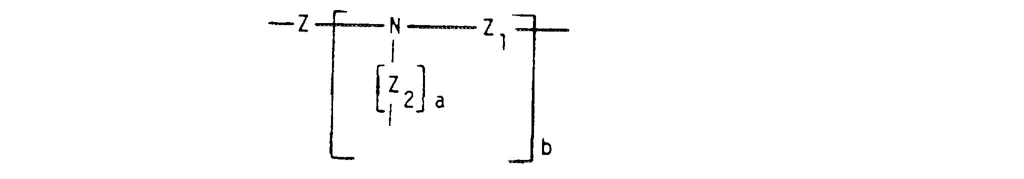 have the meaning previously defined, with one mole of a phosphorus-containing acid of general formula (XV): 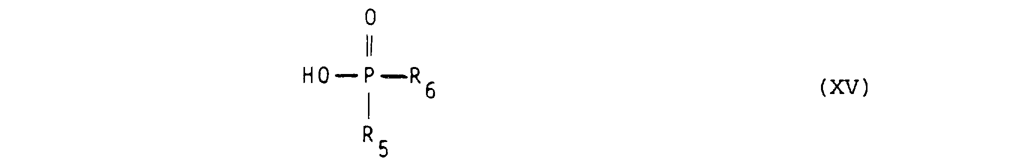 wherein R₅ and R₆ have the meaning previously defined, in the presence of a suitable solvent (for example, water, methyl alcohol, ethyl alcohol, acetonitrile, etc.) at a temperature of from 0°C and the boiling point of the solvent used, or without any solvent and with an excess of phosphorus-containing acid, if the latter can act as solvent, at a temperature of from about 0 to about 150°C:

The salts obtained can easily be prepared from the reaction mass, e.g., by filtration or by distillation of the solvent.

High quality products of general formula (I) are generally obtained in white, crystalline powder form, which can be used in self-extinguishing polymeric compositiions without further purification.

Some of the intermediates of general formula (XIV) are known. They can, however, also easily be synthesized according to the general method outlined below: 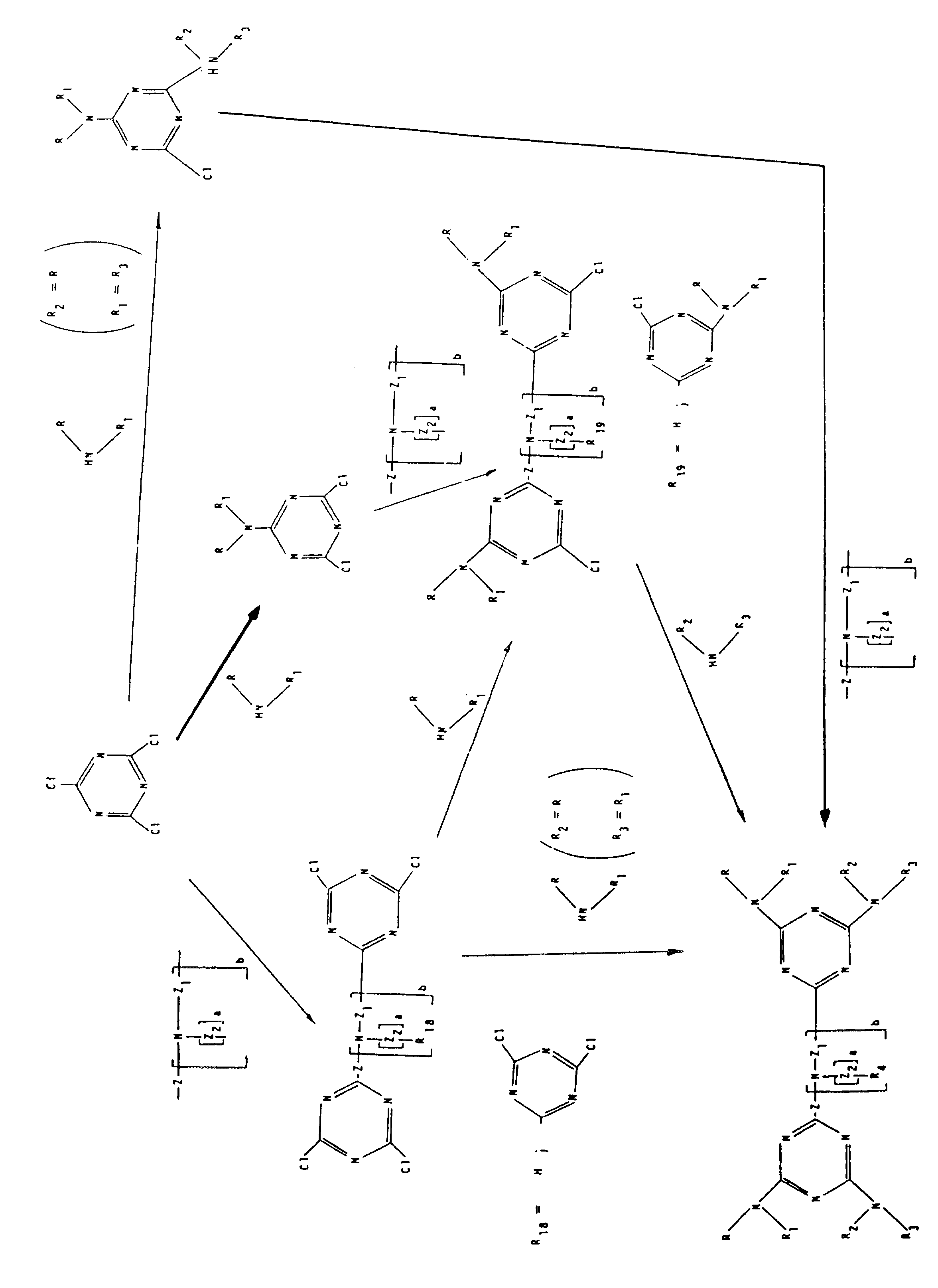 or as described in EP-A-415 371 and IT-A-18839/90.

The phosphorus-containing acids of general formula (XV) are also known and many of them are commercially available.

The present invention, moreover, provides self-extinguishing polymeric compositions, comprising:
(a) from 90 to 40 parts by weight of a thermoplastic polymer and/or a polymer having elastomeric properties;
(b) from 10 to 60 parts by weight, preferably from 12 to 40 parts by weight, of one or more derivatives of 2,4,6-triamino-1,3,5-triazine salified with an oxygenated acid of phosphorus, said derivatives of 2,4,6-triamino-1,3,5-triazine having the general formula (XIV) as specified above.

Preferably component (b) is selected from the salts of general formula (I) as specified above.

It is particularly preferable to use the salts of general formula (I) in which one or two of the moieties NRR₁ and NR₂R₃ represent NH₂ radicals.

If the self-extinguishing properties of the above polymeric compositions are to be further increased, from 1 to 25 parts by weight of one or more compounds selected from ammonium and/or amine phosphates and/or phosphonates may be added thereto in place of an equal amount of component (b).

Among the phosphates which can be used in addition to component (b) it is preferable to use ammonium polyphosphates of general formula (NH₄)ₙ₊₂PₙO₃ₙ₊₁, where n represents an integer equal to or higher than 2; preferably, the molecular weight of said polyphosphates is sufficiently high as to show a low solubility in water and, therefore, n preferably ranges from 5 to 500.

The composition of polyphosphates having the above formula, where n is a sufficiently high number, preferably from 5 to 500, corresponds essentially to the formula of metaphosphates of formula (NH₄PO₃)ₙ.

An example of said polyphosphates is that known by the trade name Exolit^{(R)} 422 (Hoechst), having the composition (NH₄PO₃)ₙ, in which n is greater than 50. Another example is the product known by the trade name Phos-Chek^{(R)} P/30 (Monsanto Chemical), which has a similar composition.

Another polyphosphate which can be advantageously used, especially because of its low solubility in water, is that known by the trade name Exolit^{(R)} 462 (Hoechst), which is Exolit^{(R)} 422 microencapsulated in melamine-formaldehyde resin.

Other phosphates which can be used are those deriving from amines, for example, dimethylammonium or diethylammonium phosphate, ethylenediamine phosphate, melamine ortho- or pyrophosphate.

Among the polymers which can be used in the compositions of the present invention, polymers or copolymers of olefins of general formula R'-CH=CH₂ are preferred, where R' is hydrogen, C₁-C₈ (preferably C₁-C₄) alkyl or (preferably C₆-C₁₀) aryl, in particular:
(1) Isotactic, or prevailingly isotactic polypropylene;
(2) HDPE, LLDPE and LDPE polyethylene;
(3) Crystalline copolymers of propylene and minor amounts of ethylene and/or other alpha-olefins, such as e.g., 1-butene, 1-hexene, 1-octene and 4-methyl-1-pentene;
(4) Heterophasic compositions comprising (A) a homopolymeric fraction of propylene and/or one of the copolymers indicated under (3), and (B) a copolymeric fraction composed of elastomeric copolymers of ethylene with an alpha-olefin, optionally containing minor amounts of a diene, wherein the alpha-olefin is, preferably, selected from propylene and 1-butene;
(5) Elastomeric copolymers of ethylene and alpha-olefins, optionally containing minor amounts of one or more dienes. Examples of dienes which are most commonly present in the above elastomeric copolymers are butadiene, ethylidene-norbornene and hexadiene-1,4. Among the polymers of olefins of general formula R'-CH=CH₂, where R' is an aryl radical, "crystal" and shock-resistant polystyrene are preferred.
   Other examples of commonly used polymers are ABS terpolymers and SAN copolymers; polyurethanes (particularly those derived from polyesters and polyethers); poly(ethylene terephthalate); poly(butylene terephthalate); polyamides; etc.

The self-extinguishing compositions of the present invention can be prepared according to known methods. For example, the ammonium and/or amine phosphate and/or phosphonate, if used, is first intimately mixed with one or more finely ground salts of general formula (I), (preferably the particle size is below 70 µm) and the mixture thus obtained is then added to the polymer in a turbomixer in order to form a homogeneous mixture which can be extruded and granulated. The granular product thus obtained can be transformed into various articles according to the well-known moulding techniques.

The fire-retardant additives of the present invention are also suitable for use in the field of flame-resistant paints.

The following examples serve to illustrate the present invention without limiting it in any way.

The salification reactions between the intermediates of general formula (XIV) and the acids of general formula (XV) were confirmed by IR spectroscopic analysis using a Perkin Elmer 580 B IR spectrophotometer.

It was found that an excellent reference signal is the peak caused by the deformation out of the triazine ring plane; the triazine ring yields a peak at approximately 830-800 cm⁻¹, whereas, when the ring is salified at the amino groups, the peak is shifted to 795-760 cm⁻¹.

### EXAMPLE 1

184.5 g of cyanuric acid chloride and 1300 ml of methylene chloride are charged into a 3 l reactor equipped with stirrer, thermometer, feeding funnel, reflux condenser and cooling bath.

At the same time, 75 g of 2-methoxyethylamine and 40 g of sodium hydroxide, dissolved in 150 ml of water, are charged, while cooling externally over 3 hours, the pH being maintained at between 5 and 7 and the temperature being maintained at between 0 and 3°C.

The whole mixture is kept at a temperature of from 0 to 3°C for a further 3 hours and then the aqueous phase is separated.

The organic solution is treated with two 200 ml portions of water, and the water phase is separated each time.

217.5 g of the intermediate product of formula (XVI): 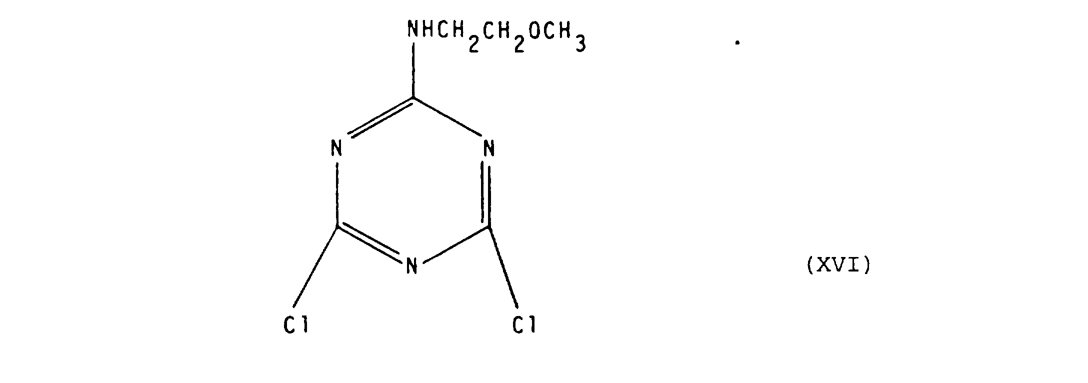 are obtained by the distillation of the methylene chloride, as a white, crystalline powder having an m.p. of 73-75°C (m.p. = melting point) and a chlorine content of 31.68% (theoretical value: 31.84%).

400 ml of acetone and 133.8 g of the intermediate (XVI) are charged into a 1 l reactor equipped with stirrer, thermometer, feeding funnel, reflux condenser and heating bath.

The mixture is stirred and heated to 40°C until a solution is obtained and then 102 g of an aqueous solution of ammonia (30% by weight) are added over 30 minutes during which the temperature is kept at 40°C.

The temperature is then raised to 45°C and maintained for 4 hours.

After cooling to 10°C, the resulting cake is filtered and washed on the filter with cold water.

After drying in an oven at 100°C, 114 g of the intermediate product of formula (XVII): 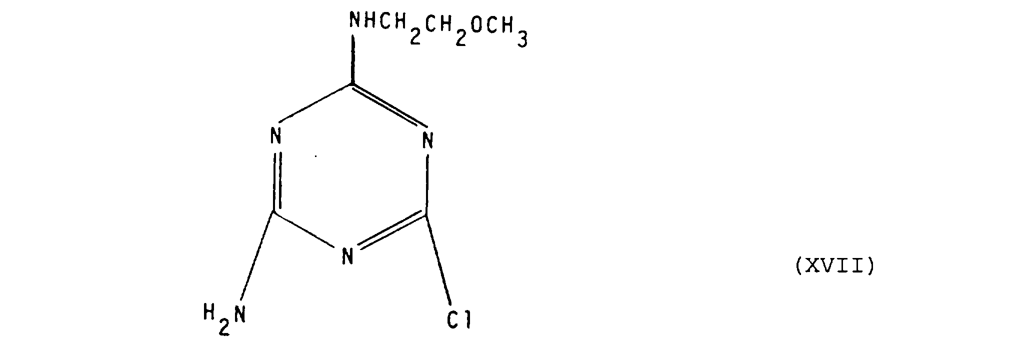 are obtained as white, crystalline powder having an m.p. of 195-197°C and a chlorine content of 17.18% (theoretical 17.44%).

The structure of the intermediates (XVI) and (XVII) was confirmed by IR-spectroscopic analysis.

500 ml of xylene, 81.4 g of the intermediate (XVII) and 17.2 g of piperazine are charged into the above 1 l reactor.

The mixture is heated to 100°C and this temperature is maintained for 2 hours.

16 g of sodium hydroxide are then added and the mixture is brought to reflux, keeping it in said condition for about 20 hours and, thereafter, cooling it to room temperature, followed by filtration.

The cake is thoroughly washed with water and dried.

74.2 g of the intermediate of formula (XVIII): 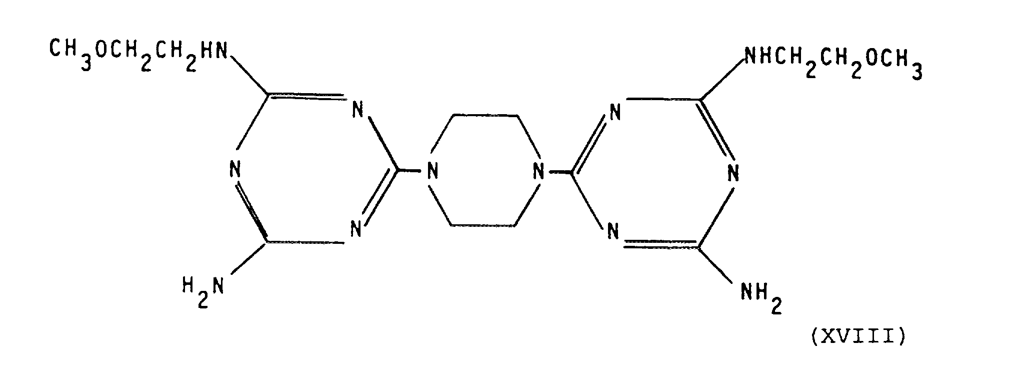 are obtained, having an m.p. of 212-215°C.

63 g of the intermediate (XVIII), 400 ml of acetonitrile and, under agitation, 34.6 g of an 85% by weight phosphoric acid are charged into the above 1 l reactor.

The resulting mixture is heated to the boiling point and is kept under reflux for 8 hours.

After cooling to room temperature, the resulting cake is filtered and washed on the filter with acetonitrile.

Upon drying the cake in an oven at 100°C, 89.2 g of product of the following formula: 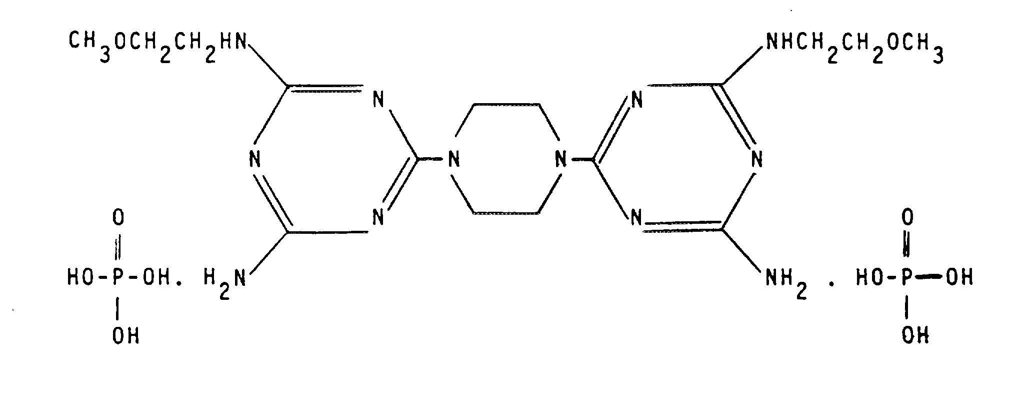 are obtained as a white crystalline powder having an m.p. of 265-268°C and a phosphorus content of 9.97%
(theoretical: 10.06%).

### EXAMPLE 2

184.5 g of cyanuric acid chloride and 1300 ml of methylene chloride are charged into the 3 l reactor of example 1.

Following the same procedure as in example 1 but using 87.2 g of morpholine, 230 g of the intermediate of formula (XIX): 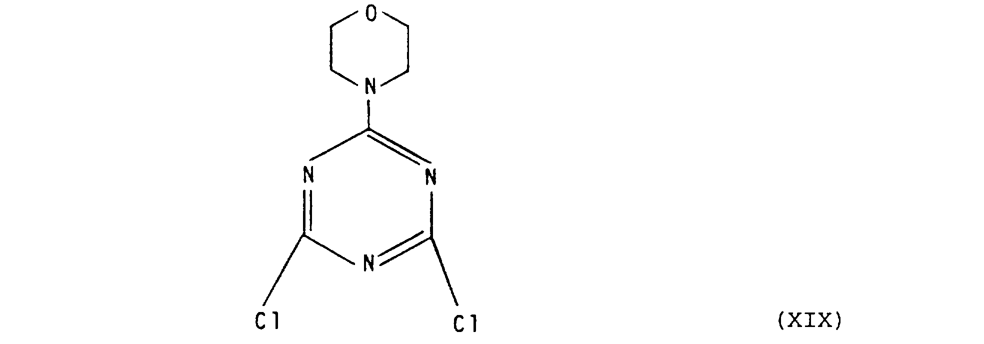 are obtained as white, crystalline powder having an m.p. of 155-157°C and a chlorine content of 29.87%
(theoretical: 30.21%).

100 g of a 30% by weight aqueous solution of ammonia, 100 ml of water and 70.5 g of the intermediate (XIX) are charged into a 0.5 l reactor equipped as described in example 1.

The above mixture is heated to 50°C and kept at this temperature for 7 hours. It is left to cool to room temperature and the resulting product is filtered and washed with water.

Upon drying of the resulting product, 58 g of the intermediate of formula (XX): 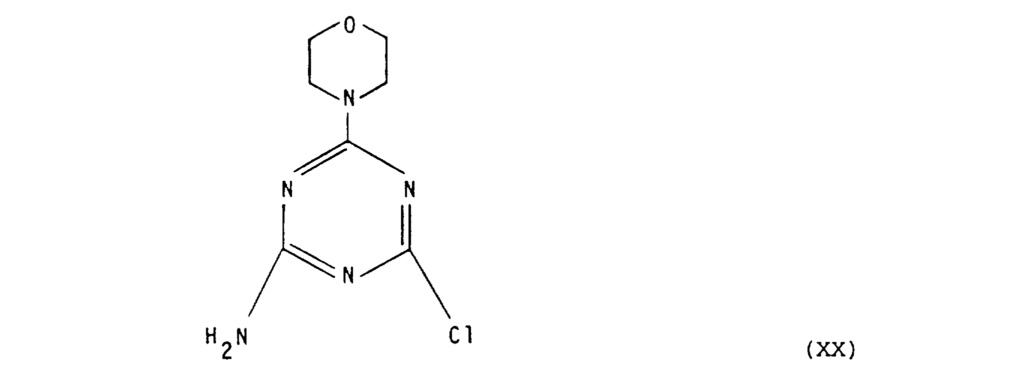 are obtained as white, crystalline powder having an m.p. of 189-191°C and a chlorine content of 16.28%
(theoretical: 16.47%).

The structure of compounds (XIX) anbd (XX) was confirmed by IR-spectroscopic analysis.

400 ml of ortho-dichlorobenzene, 53.9 g of intermediate (XX) and 14.5 g of hexamethylene diamine are charged into a 1 l reactor equipped as described above.

The mixture is heated to 100°C and kept at this temperature for 2 hours. 10 g of sodium hydroxide are then added and the mixture is heated to 140°C, keeping it at this temperature for 16 hours and then cooling it to room temperature. The resulting product is filtered and thoroughly washed with water.

After drying, 62.3 g of the intermediate of formula (XXI): 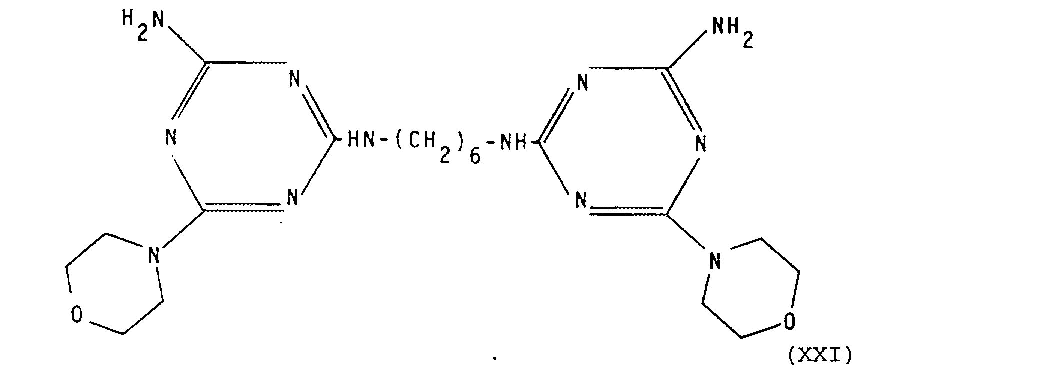 are obtained as white, crystalline powder having an m.p. of 267-269°C.

300 ml of acetonitrile, 47.4 g of the intermediate (XXI) and, under stirring, 24.2 g of an 85% by weight phosphoric acid are charged into a 0.5 l reactor equipped as described above.

The mixture is heated to the boiling point and kept under reflux for 12 hours.

After cooling to room temperature, the resulting product is filtered and washed on the filter with acetonitrile.

After drying, 65.8 g of the following product: 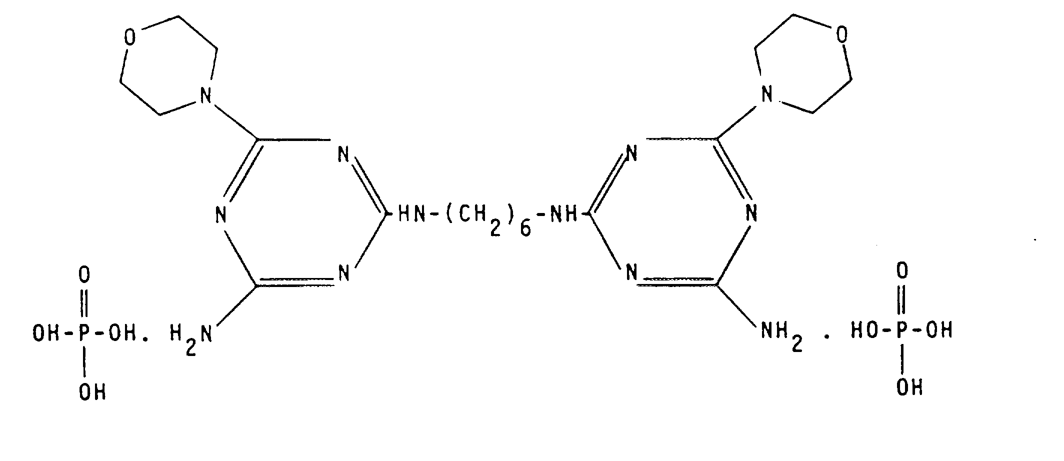 are obtained as a white, crystalline powder having an m.p. of 265-268°C and a phosphorus content of 9.18%
(theoretical: 9.25%).

### EXAMPLE 3

184.5 g of cyanuric acid chloride and 800 ml of acetone are charged into a 3 l reactor equipped with stirrer, thermometer, feeding funnel, reflux condenser and heating bath.

The mixture is heated, under agitation, to 40°C until a solution is obtained, and 284 g of a 30% by weight solution of aqueous ammonia are then added over a period of 30 minutes during which the temperature is maintained at 40°C.

The mixture is then further heated to 44°C and kept at this temperature for 4 hours.

After cooling, the resulting product is filtered and washed on the filter with water.

After drying under vacuum, in an oven at 50-60°C, 113 g of the intermediate of formula (XXII): 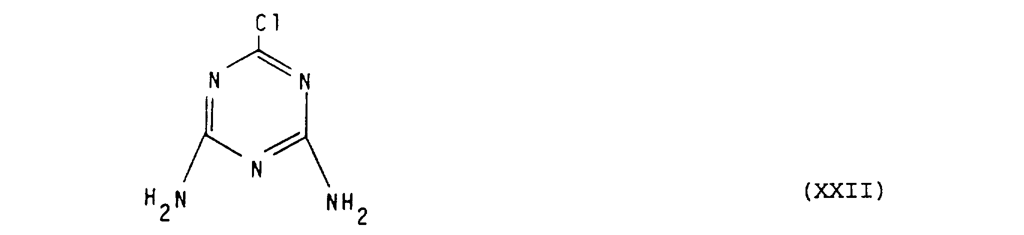 are obtained as white, crystalline powder having an m.p. of higher than 300°C and a chlorine content of 24.2%
(theoretical: 24.4%).

The structure of the above compound was confirmed by IR-spectroscopic analysis.

400 ml of xylene, 58.2 g of the intermediate (XXII) and 17.2 g of piperazine are charged into a 1 l reactor (equipped as described above).

The mixture is heated to 100°C and kept at this temperature for 2 hours.

16 g of solid sodium hydroxide are then added and the mixture is brought to reflux, keeping it under said condition for about 20 hours and then cooling it to room temperature, followed by filtration.

The cake is thoroughly washed with water and dried. 54.2 g of the intermediate of formula (XXIII): 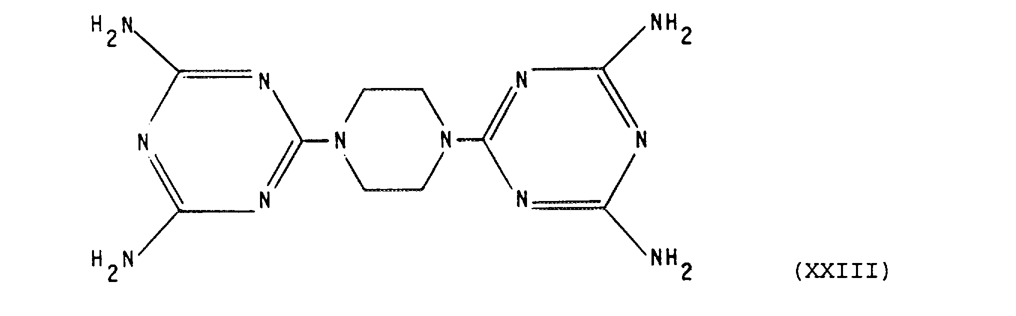 are obtained as white, crystalline powder, having an m.p. of higher than 300°C.

328 g of phosphorous acid and 82 g of acetonitrile are charged into a 1 l reactor equipped as described above.

The reaction mixture is slowly heated, over a period of 6 hours, to 160°C.

A white, crystalline powder is obtained.

The mixture is then cooled to 80°C, 500 ml of water are added under strong agitation and the resulting mass left to cool to room temperature.

The resulting product is separated by filtration and washed on the filter with a small amount of water.

After drying of the cake, 290 g of 1-aminoethane-1,1-diphosphonic acid are obtained as white, crystalline powder having an m.p. of 265-270°C (with decomposition) and a phosphorus content of 29.4%
(theoretical: 30.24%).

600 ml of water and 45.6 g of the intermediate (XXIII) are charged into the above 1 l reactor.

The mixture is heated to 80°C and 61.6 g of 1-aminoethane-1,1-diphosphonic acid are added under agitation.

The resulting mixture is brought to the boiling point and is maintained under reflux for about 8 hours.

After cooling to room temperature, the resulting product is filtered and washed on the filter with water.

After drying the cake, 102.5 g of the following product: 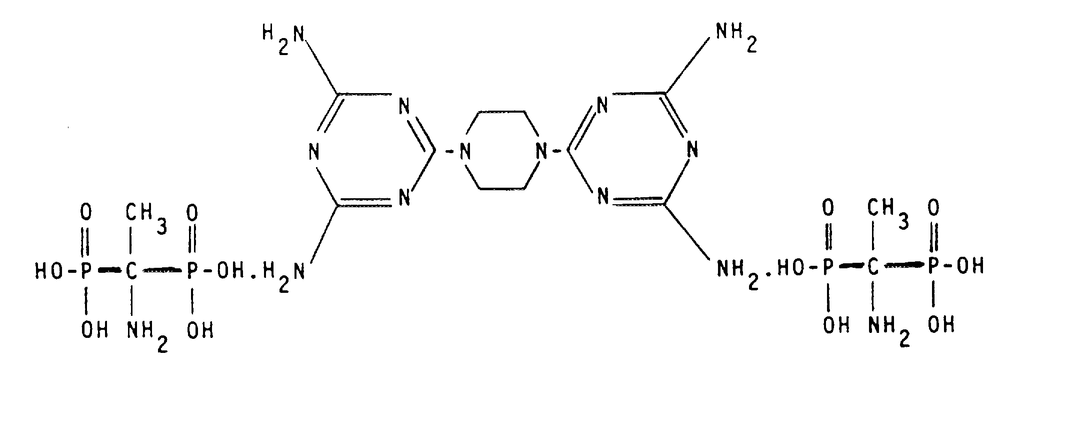 are obtained as white, crystalline powder havin an m.p. of 273-275°C and a phosphorus content of 16.97%
(theoretical: 17.36%).

### EXAMPLE 4

600 ml of xylene, 107.8 g of the intermediate (XX) and 15 g of ethylene diamine are charged into a 1 l reactor (equipped as described in example 2).

Following the same procedure as in example 2, 99.6 g of the intermediate of formula (XXIV): 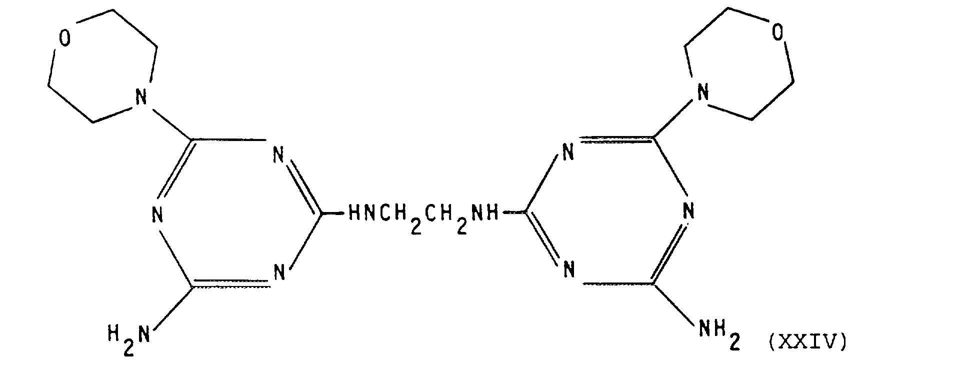 are obtained as white, crystalline powder having an m.p. of 265-268°C.

53.2 g of tetrasodium pyrophosphate and 400 ml of water are charged into the above 1 l reactor, equipped with a cooling bath.

The mixture is cooled from the outside to 5°C and 78.7 g of hydrochlorid acid (37% by weight) are then added, whereby a solution is obtained.

83.6 g of the intermediate of formula (XXIV) are added to this solution at a constant temperature of 5°C.

The above solution is then kept under agitation for 2 hours at a temperature of 5°C, is then left to reach room temperature and is further stirred for an additional 3 hours.

After cooling again to 2-5°C, the resulting product is separated by filtration and washed on the filter with cold water.

After drying the cake, 108.1 g of the following product: 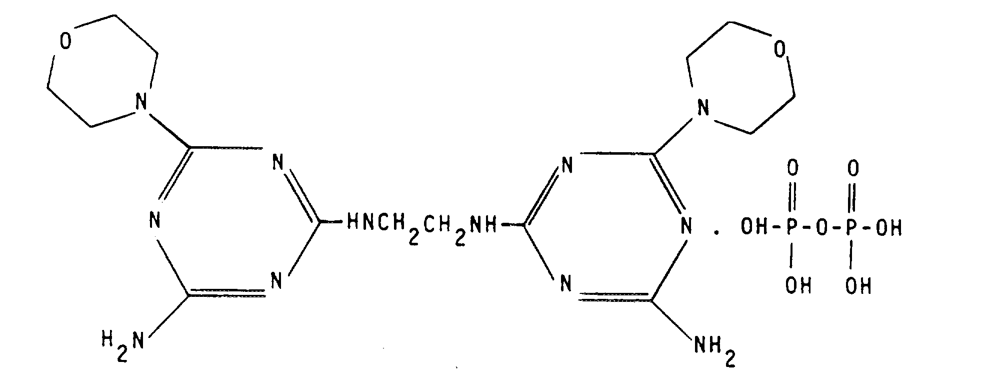 are obtained as white, crystalline powder having an m.p. of 277-282°C and a phosphorus content of 9.97%
(theoretical: 10.40%).

### EXAMPLE 5

400 ml of xylene, 64.8 g of the intermediate (XX) and 10.3 g of diethylene triamine are charged into a 1 l reactor (equipped as described in the preceding examples).

The mixture is heated to 100°C and kept at this temperature for 2 hours. 12 g of sodium hydroxide are then added and the whole mixture is brought to reflux.

The mass is maintained under reflux for 24 hours, is then cooled to room temperature and the resulting product is filtered and the cake is thoroughly washed with water.

After drying in an oven at 100°C, 56.7 g of the intermediate of formula (XXV): 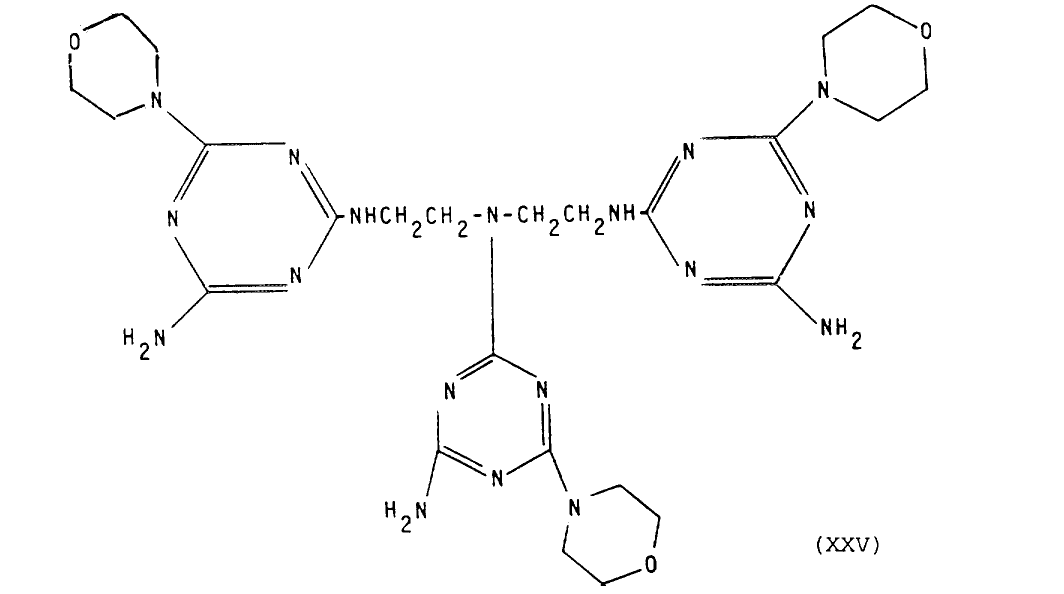 are obtained as white, crystalline powder having an m.p. of 207-208°C.

150 ml of acetonitrile, 32.0 g of the intermediate (XXV) and, under agitation, 18.2 g of phosphoric acid (85% by weight) are charged into a 0.5 1 reactor (equipped as described above).

The mass is heated to boiling temperature and is maintained under reflux for 16 hours.

After cooling to room temperature, the resulting product is filtered and washed on the filter with acetonitrile.

Upon drying of the cake, 46.4 g of the following product: 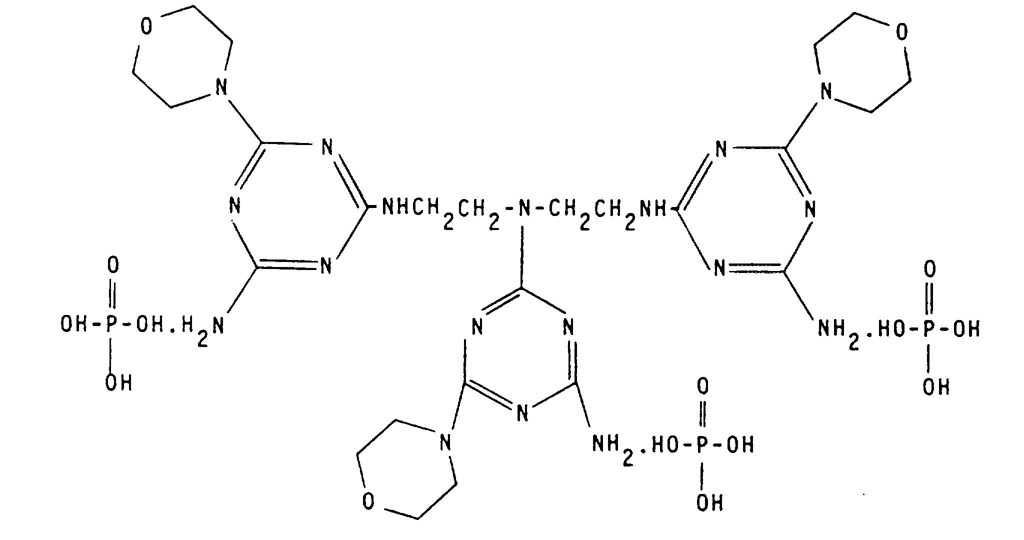 are obtained as white, crystalline powder having an m.p. of 99-101°C and a phosphorus content of 9.77%
(theoretical: 9.96%).

### EXAMPLE 6

129 g of cyanuric acic chloride and 100 ml of methylene chloride are charged into a 2 l reactor (equipped as indicated in the previous examples).

40 g of 3-amino-1-propene, dissolved in 150 g of water, are added to the solution (which is kept at 0-2°C with external cooling), over a period of 90 minutes.

At a constant temperature of 0-2°C, 28 g of sodium hydroxide in 100 ml of water are added over a period of 2 hours. The solution is left under agitation for a further 2 hours at a temperature of 3-5°C and the water phase is then separated.

By distillation of the methylene chloride, 137 g of the intermediate of formula (XXVI): 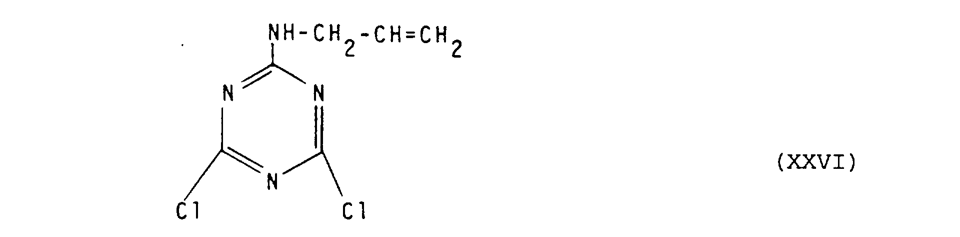 are obtained as white, crystalline powder having an m.p. of 70-72°C and a chlorine content of 34.37%
(theoretical: 34.63%).

200 g of a 30% by weight aqueous ammonia solution and 500 ml of water are charged into the above reactor.

The solution is heated to 40°C and 123 g of the intermediate fo formula (XXVI) are added over a period of 30 minutes, the temperature being maintained at 40°C.

The temperature is raised to 45°C and maintained for about 6 hours.

Thereafter, the solution is cooled to room temperature and the resulting product is filtered, washed with water and dried.

104 g of the intermediate of formula (XXVII): 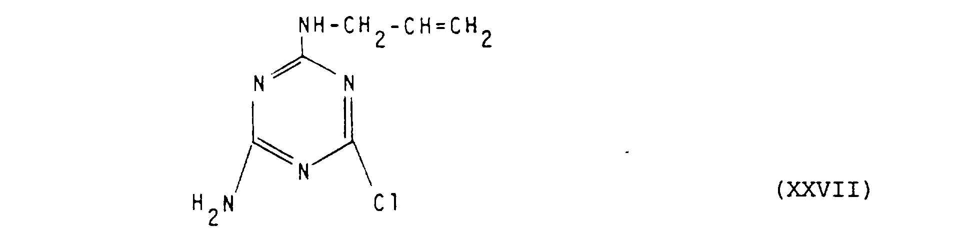 are obtained as white, crystalline powder having an m.p. of 168-170°C and a chlorine content of 18.82%
(theoretical: 19.14%).

The structures of the intermediates (XXVI) and (XXVII) were confirmed by means of NMR-analysis.

450 ml of xylene, 55.7 g of the intermediate (XXVII) and 17.1 g of 2,5-dimethylpiperazine are charged into a 1 l reactor (equipped as described above).

The resulting mass is heated to 100°C for 2 hours, whereafter 12 g of solid sodium hydroxide are added and the mixture is caused to boil.

The mixture is kept under reflux for 18 hours and then the procedure described in the preceding examples is followed.

56.3 g of the intermediate of formula (XXVIII): 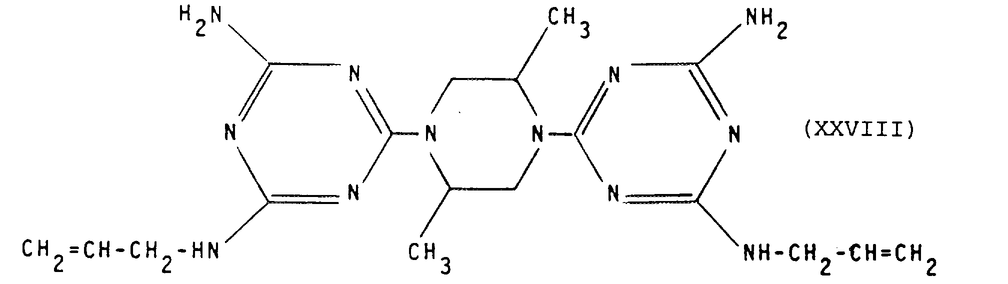 are obtained as white, crystalline powder having an m.p. of 192-194°C.

400 ml of acetonitrile, 48.4 g of phosphoric acid (85% by weight) and, under agitation, 82.4 g of the intermediate (XXVIII) are charged into the above 1 l reactor.

The resulting mixture is heated to the boiling point and is kept under reflux for 10 hours, whereafter it is cooled to room temperature and the product obtained is filtered and washed on the filter with acetonitrile.

Upon drying of the cake 114.2 g of the following product: 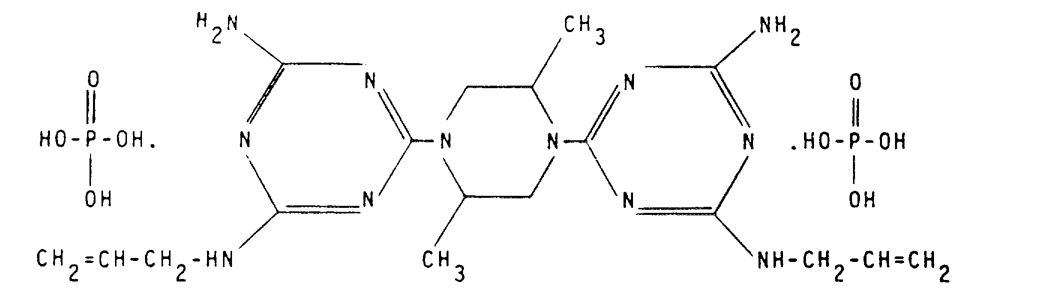 are obtained as white, crystalline powder having an m.p. of 197-200°C and a phosphorus content of 9.87%
(theoretical: 10.20%).

### EXAMPLE 7

92.2 g of cyanuric acid chloride and 300 ml of acetone are charged into a 1 l reactor (equipped as described in example 1).

21.5 g of piperazine, dissolved in 200 ml of acetone, are added to the mixture over a period of 1 hour, cooling externally to 0-5°C.

20 g of sodium hydroxide in 100 ml of water are added at a constant temperature of 0-5°C.

The whole mixture is kept for a further 4 hours under agitation at 5°C, whereafter 200 ml of cold water are added and the precipitate formed is filtered and washed on the filter with water.

After drying, 88.7 g of the intermediate of formula (XXIX): 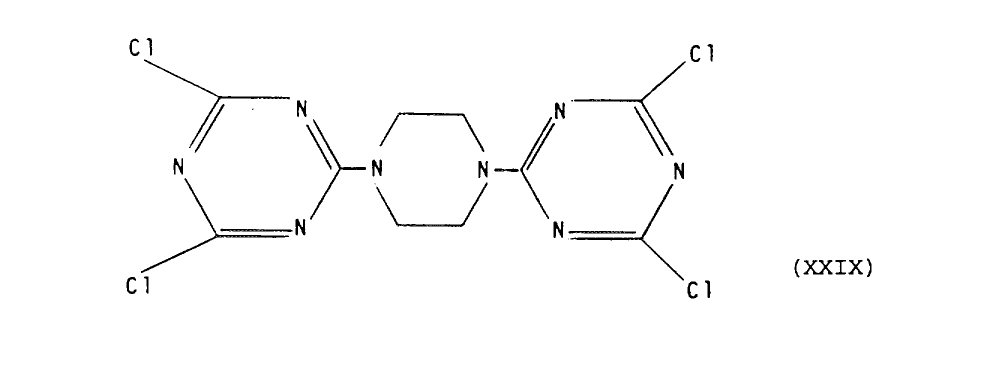 are obtained as white, crystalline powder, having an m.p. of higher than 300°C and a chlorine content of 37.4%
(theoretical: 37.2%).

The structure of the intermediate (XXIX) was also confirmed by IR-spectroscopic analysis.

400 ml of xylene and 76.4 g of the intermediate (XXIX) are charged into the above 1 l reactor, equipped with heating bath.

The mixture is heated to a temperature of 80°C and then 60 g of 2-methoxyethylamine, followed by 32 g of sodium hydroxide in 50 ml of water, are added over a period of 4 hours.

The temperature is gradually raised and the water is removed by means of azeotropic distillation until the boiling point of the solvent is reached.

The mixture is kept under reflux for 8 hours and then is cooled to room temperature, filtered and thoroughly washed with water.

After drying, 93.2 g of the intermediate of formula (XXX): 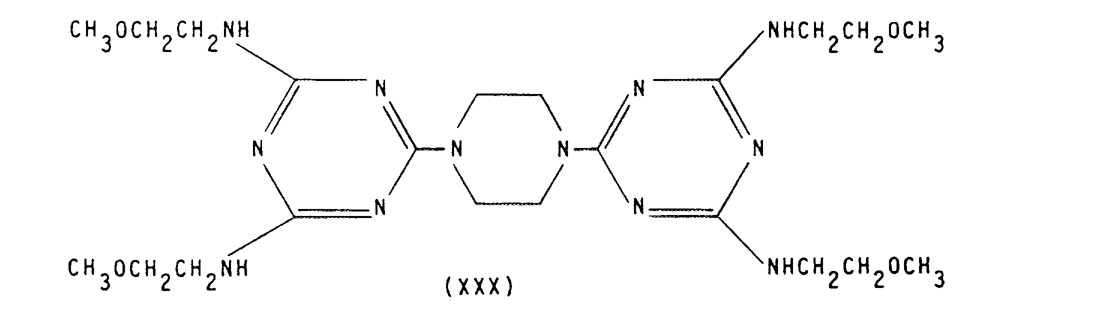 are obtained as a white, crystalline powder having an m.p. of 170-172°C.

400 ml of ethyl alcohol, 53.6 g of the intermediate (XXX) and, under agitation, 24.2 g of phosphoric acid (85% by weight) are charged into the above 1 l reactor.

The mixture is left under agitation at room temperature for about 14 hours, whereafter the resulting product is filtered and washed on the filter with a small amount of solvent.

By drying the cake in an oven at 100°C, 70.9 g of the following product: 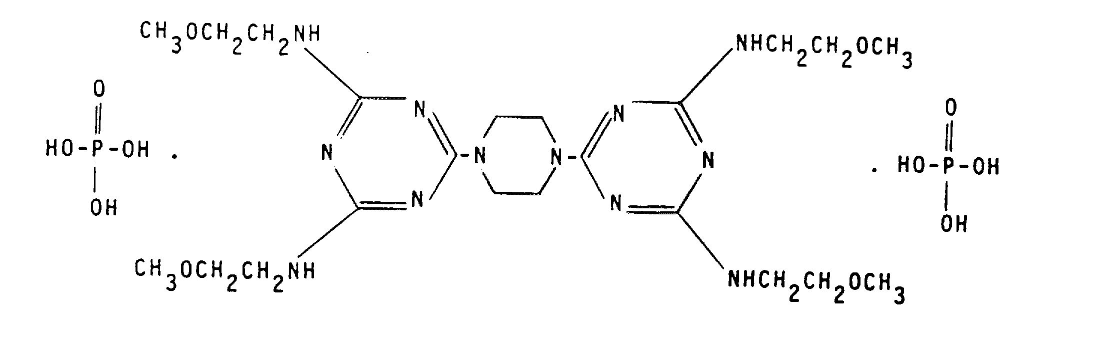 are obtained as white, crystalline powder having an m.p. of 242-245°C and a phosphorus content of 8.43%
(theoretical. 8.47%).

### EXAMPLE 8

400 ml of acetone, 500 ml of water and 94 g of the intermediate (XIX) are charged into a 2 l reactor (equipped as described in example 1).

The mixture is cooled from the outside to 5-10°C and then 49.8 g of 2-hydroxyethylamine are added over a period of 1 hour.

The temperature is raised to room temperature and than the mixture is left under agitation for 1 hour, whereafter it is heated to 40°C and kept at this temperature for a further 2 hours.

Upon cooling again to 10°C, the resulting product is filtered and washed with a small amount of cold water.

After drying the cake, 89.4 g of the intermediate of formula (XXXI): 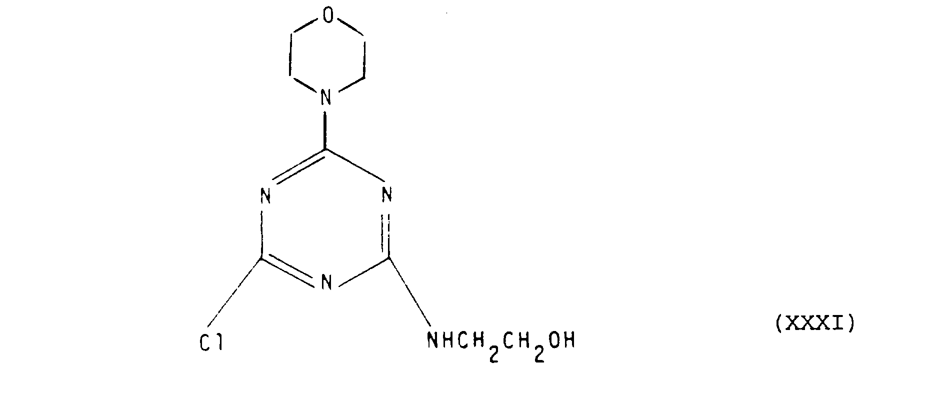 are obtained as white, crystalline powder, having an m.p. of 168-170°C and a chlorine content of 13.59%
(theoretical: 13.68%).

The structure of the intermediate (XXXI) was also confirmed by means of NMR analysis.

400 ml of xylene, 77.9 g of the intermediate (XXXI) and 12.9 g of piperazine are charged into a 1 l reactor, equipped as indicated in the preceding examples.

The mixture is heated to 100°C for 2 hours, 12 g of solid sodium hydroxide are then added and the whole mixture is caused to boil, keeping it under reflux for 16 hours, whereafter the procedure described in the preceding examples is followed.

65.4 g of the intermediate of formula (XXXII): 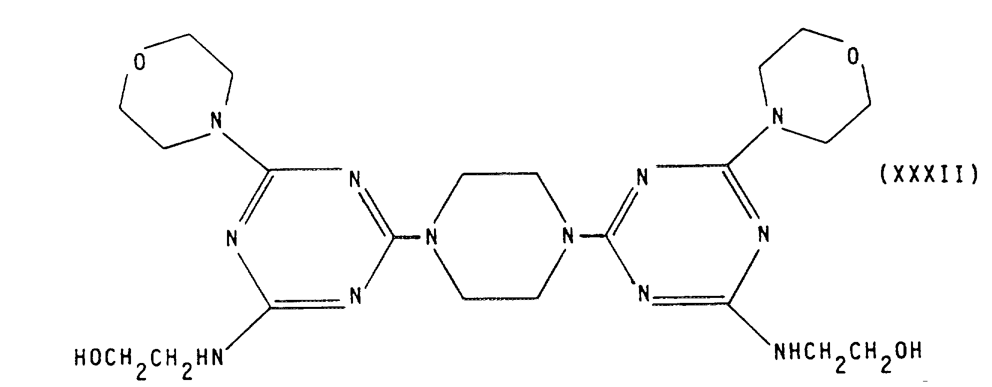 are obtained as white, crystalline powder having an m.p. of 260-262°C.

400 ml of acetonitrile, 53.2 g of the intermediate (XXXII) and, under agitation, 16.8 g of phosphorous acid are charged into the above 1 l reactor.

The mixture is brought to the boiling point and kept under reflux for 8 hours, whereafter it is cooled to room temperature and the resulting product is filtered and washed on the filter with acetonitrile.

By drying the cake in an oven at 100°C, 68.9 g of the following product: 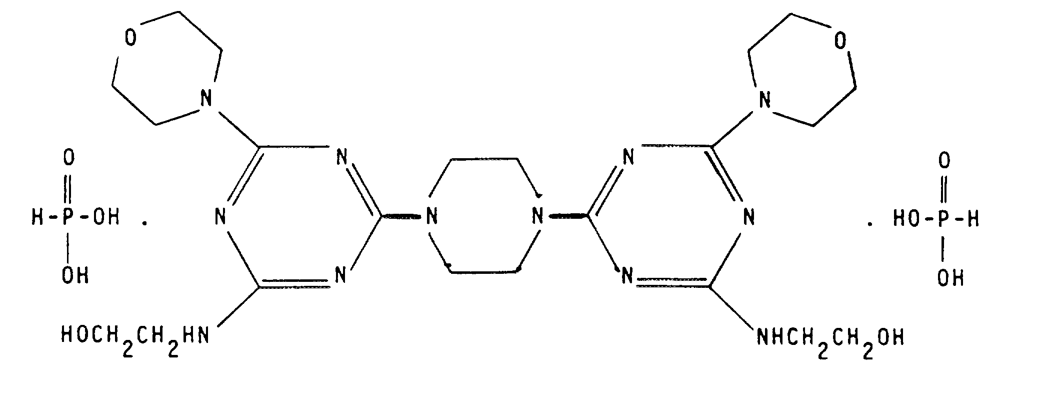 are obtained as white crystalline powder having an m.p. of 176-178°C and a phosphorus content of 8.84%
(theoretical: 8.91%).

### EXAMPLE 9

500 ml of xylene, 86.2 g of the intermediate (XX) and 15.1 g of tetraethylene pentamine are charged into a 1 l reactor, equipped as in the preceding examples.

The mixture is heated to 80°C and is kept at this temperature for 2 hours.

16 g of sodium hydroxide are then added and the temperature is raised to 110°C.

The mixture is kept at 110°C for 18 hours and is then cooled to room temperature, whereafter the resulting product is filtered and thoroughly washed on the filter with water.

By drying the cake in an oven at 100°C, 82.6 g of the intermediate of formula (XXXIII): 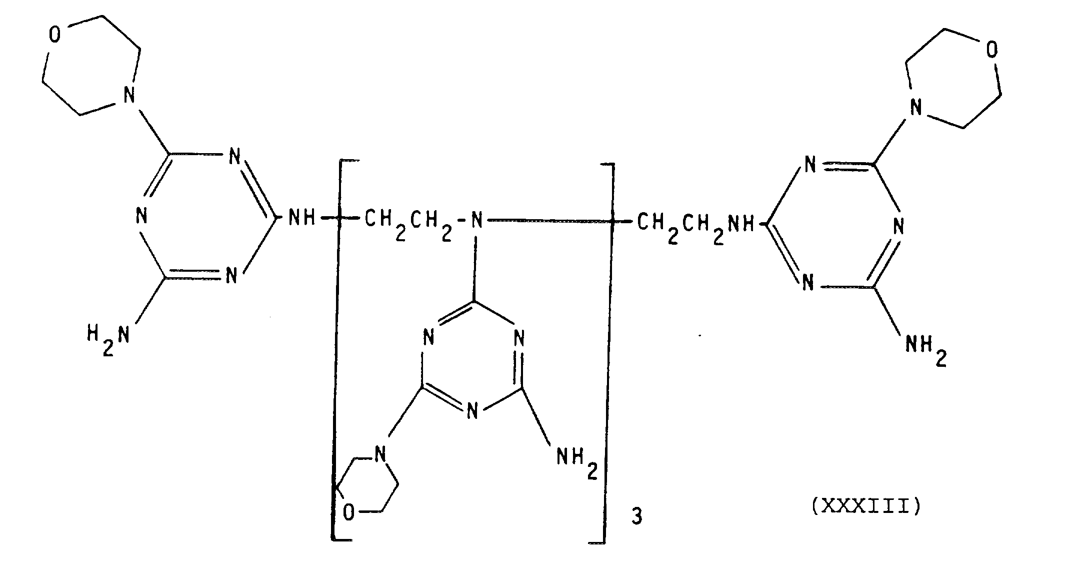 are obtained as white, crystalline powder having an m.p. of 178-183°C.

350 ml of acetonitrile, 54.2 g of the intermediate (XXXIII) and, under agitation, 20.5 g of phosphorous acid are charged into the above 1 l reactor.

The mixture is brought to the boiling point and kept in a reflux condensing system for about 12 hours.

After cooling to room temperature, the resulting product is filtered and washed on the filter with acetonitrile.

By drying in an oven, 72.7 g of the following product: 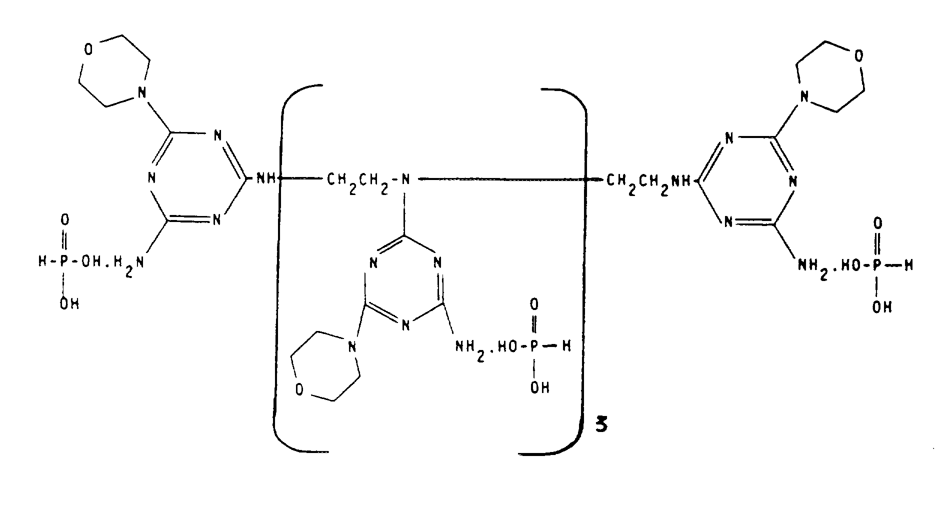 are obtained as white, crystalline powder having an m.p. of 129-132°C and a phosphorus content of 10.61%
(theoretical: 10.37%).

### EXAMPLE 10

450 ml of water, 91.6 g of the intermediate (XVII) and, under agitation, 21.9 g of tris(2-aminoethyl)amine are charged into a 1 l reactor (equipped as described in the preceding examples).

The mixture is heated to 80°C and kept at this temperature for 3 hours.

18 g of sodium hydroxide, dissolved in 30 ml of water, are then added and the resulting mixture is brought to the boiling temperature, keeping it under reflux for 16 hours.

After cooling to 10°C, the resulting product is filtered and washed on the filter with cold water.

By drying the cake in an oven at 100°C, 85.4 g of the intermediate of formula (XXXIV): 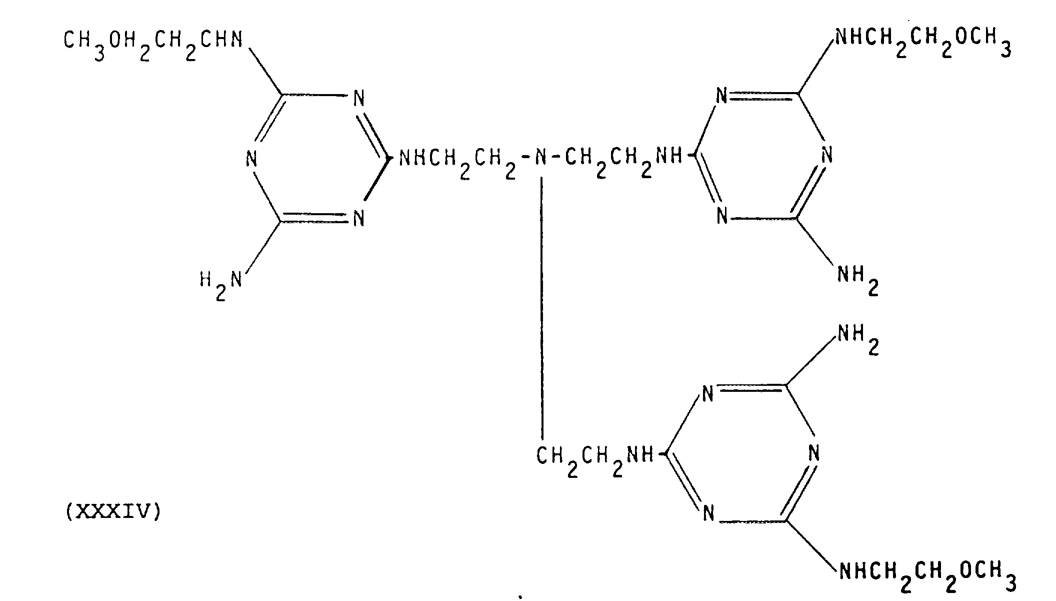 are obtained as white, crystalline powder having an m.p. of 190-195°C.

400 ml of acetonitrile, 64.7 g of the intermediate (XXXIV) and, under agitation, 36.3 g of phosphoric acid (85% by weight) are charged into the above 1 l reactor.

The mixture is heated to the boiling temperature and is kept under reflux for about 14 hours, whereafter it is cooled to room temperature and the resulting product is filtered and washed on the filter with acetonitrile.

By drying the cake in an oven, 82.1 g of the following product: 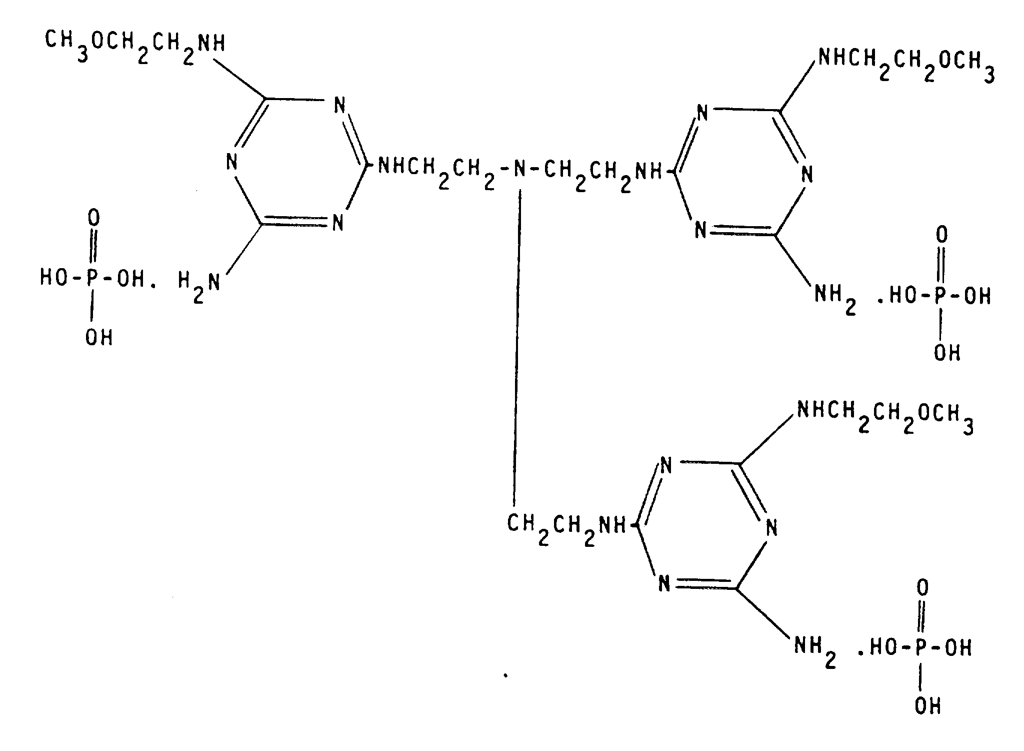 are obtained as white, crystalline powder having an m.p. of 107-111°C and a phosphorus content of 10.15%
(theoretical: 9.88%).

### EXAMPLE 11

400 ml of water, 86.2 g of the intermediate (XX) and 20.6 g of diethylene triamine are charged into a 1 l reactor with the equipment described in the preceding examples.

The mixture is heated to 80°C for 2 hours, whereafter 16 g of sodium hydroxide, dissolved in 30 ml of water, are added and the whole mixture is heated to the boiling temperature, keeping it under reflux for about 14 hours. Then the procedure described in the preceding examples is followed. 86.2 g of the intermediate of formula (XXXV): 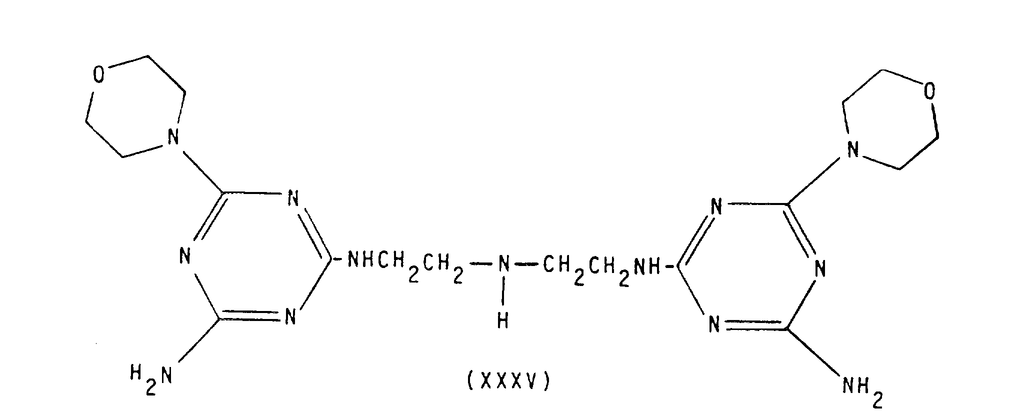 are obtained as white, crystalline powder having an m.p. of 198-201°C.

450 ml of acetonitrile, 69.1 g of the intermediate (XXXV) and, under agitation, 36.3 g of phosphoric acid (85% by weight) are charged into the above 1 l reactor.

The mixture is heated to the boiling temperature and kept under reflux for about 10 hours.

Using the procedure described in the preceding examples, 95.2 g of the following product: 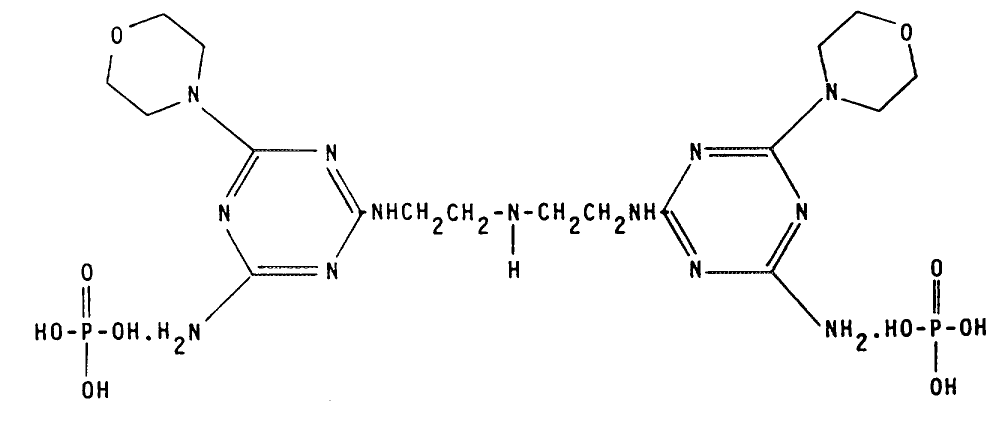 are obtained as white, crystalline powder having an m.p. of 120-124°C and a phosphorus content of 9.21%
(theoretical: 9.44%).

### EXAMPLES 12-52

The products of general formula (I), shown in table 1 below, are synthesized under the same conditions as those described in examples 1 to 11.

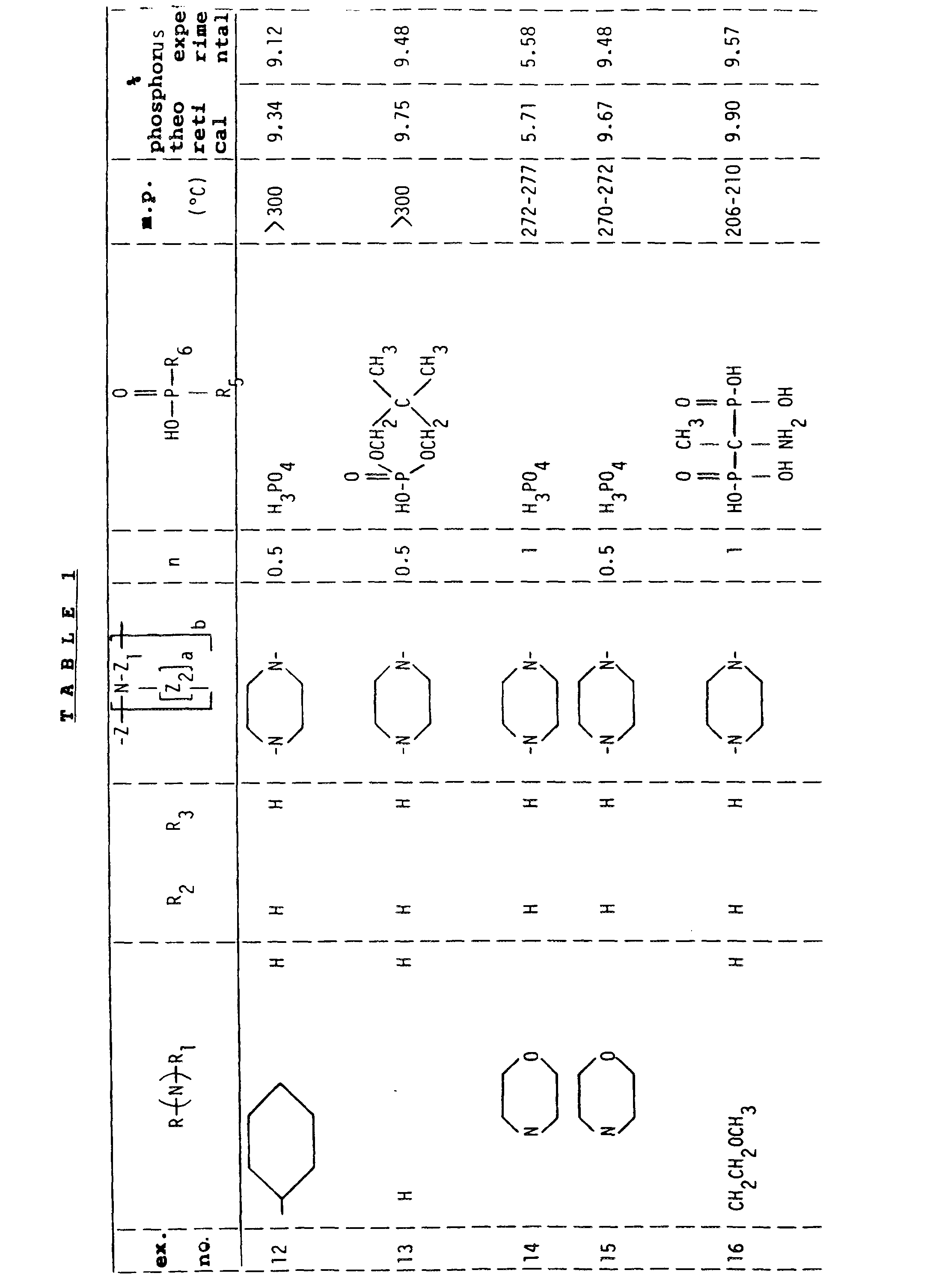

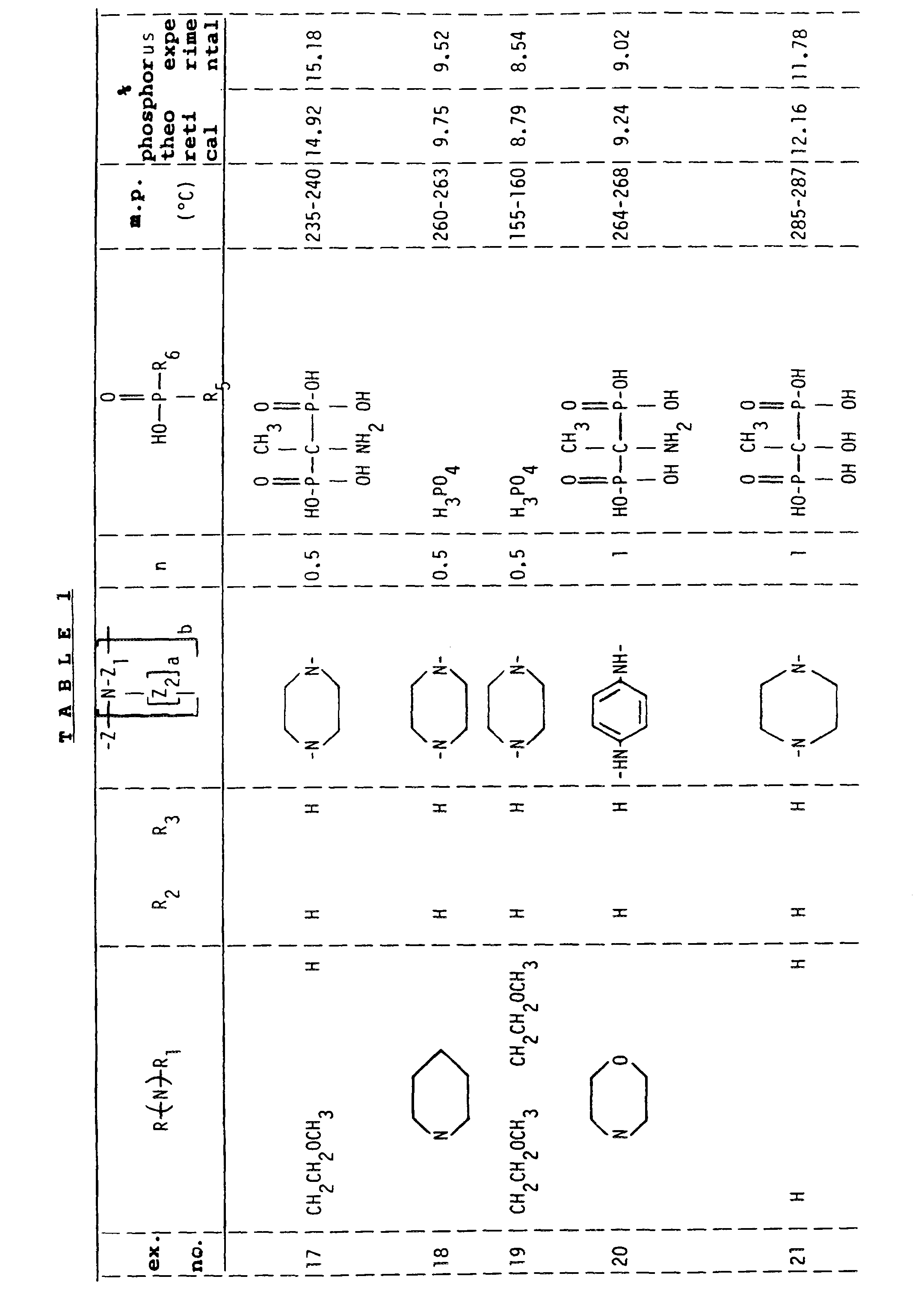

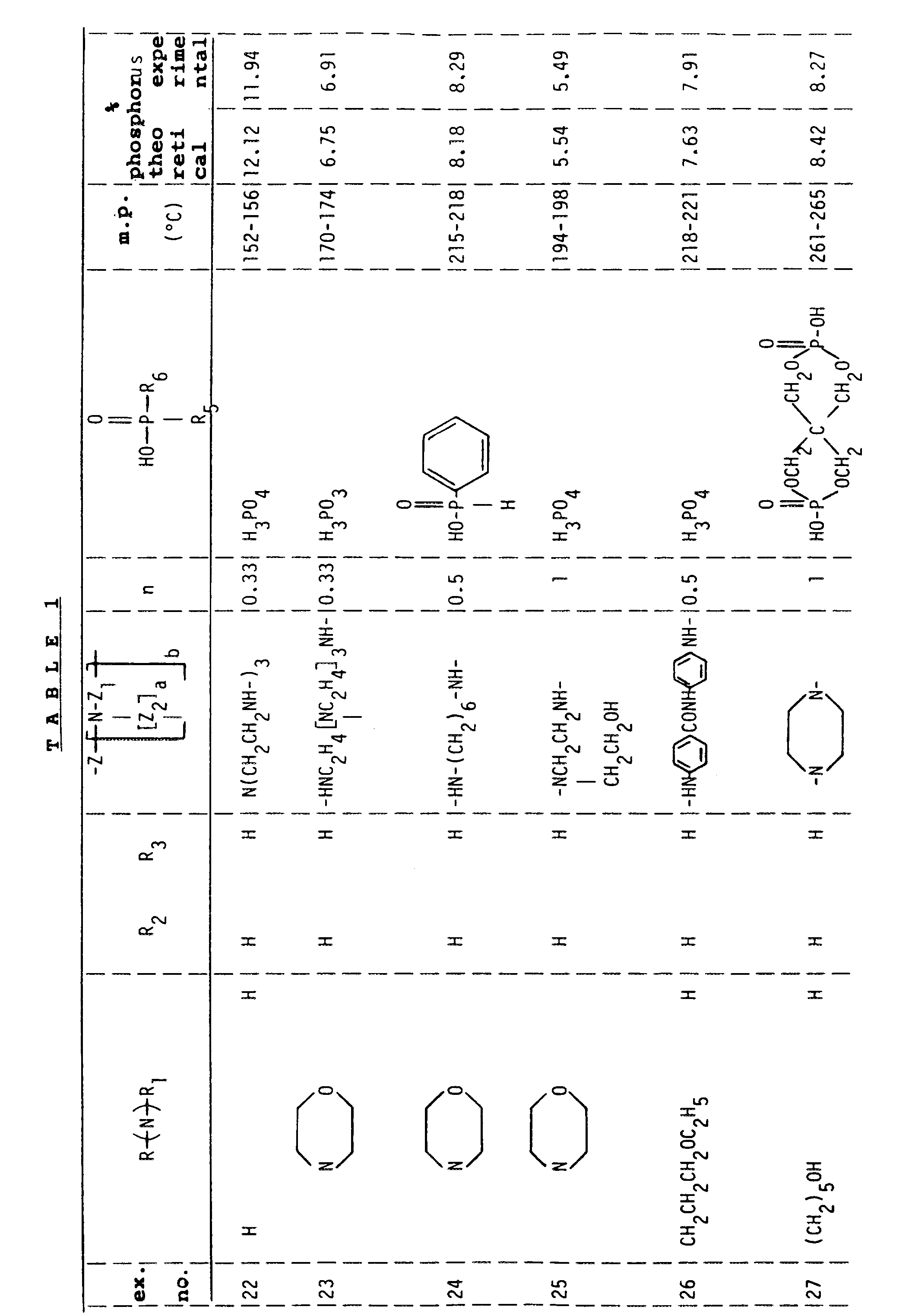

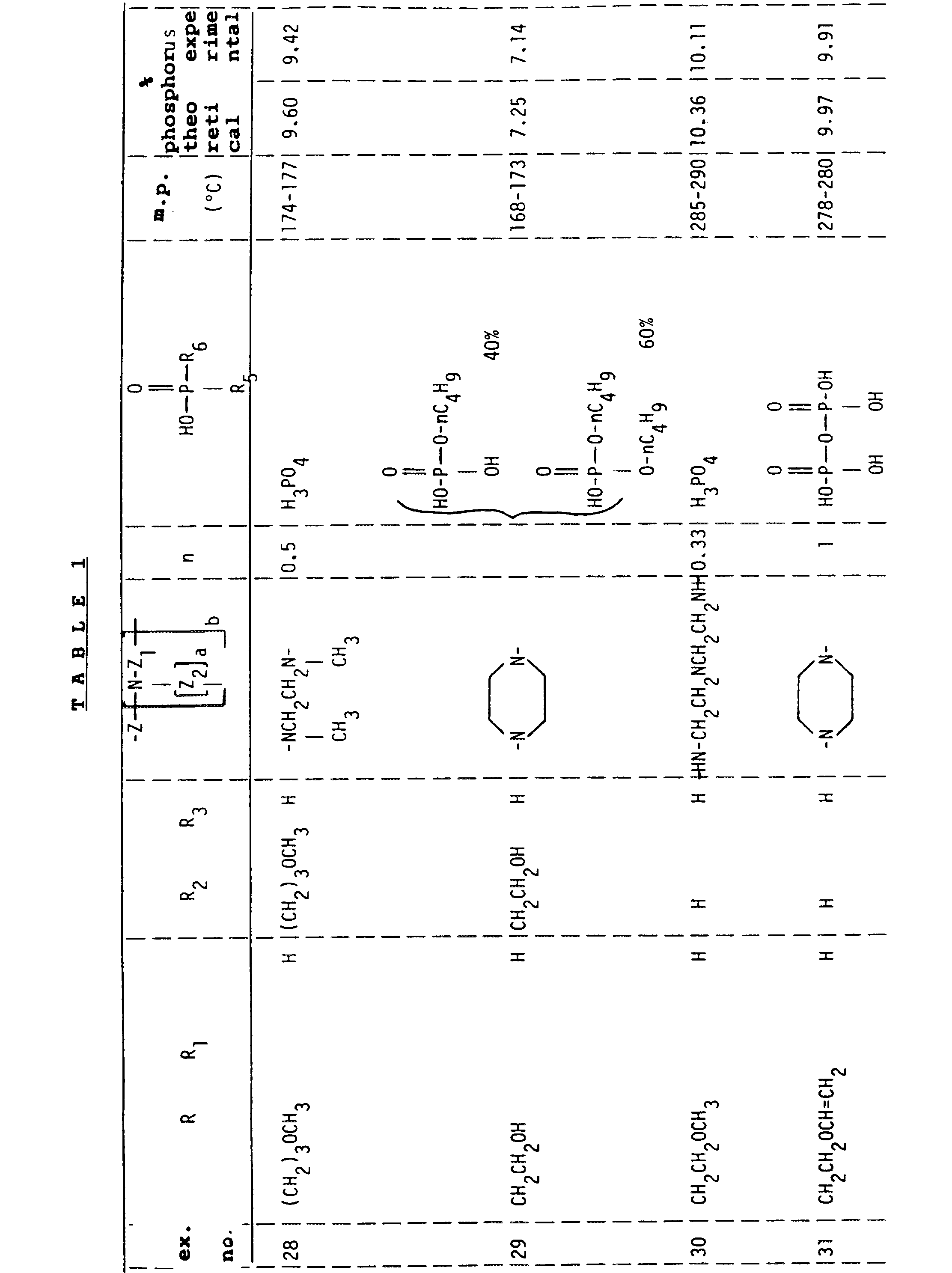

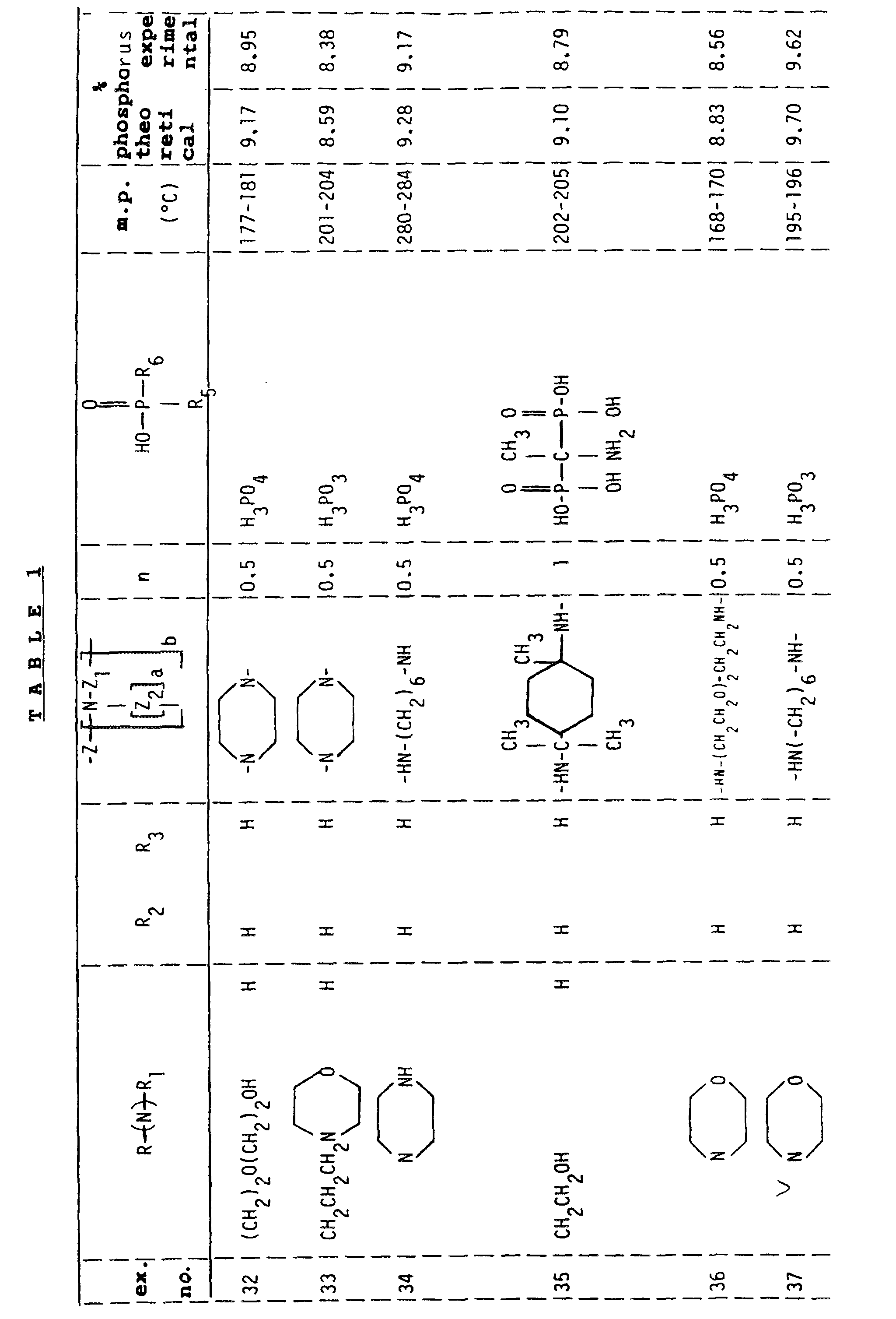

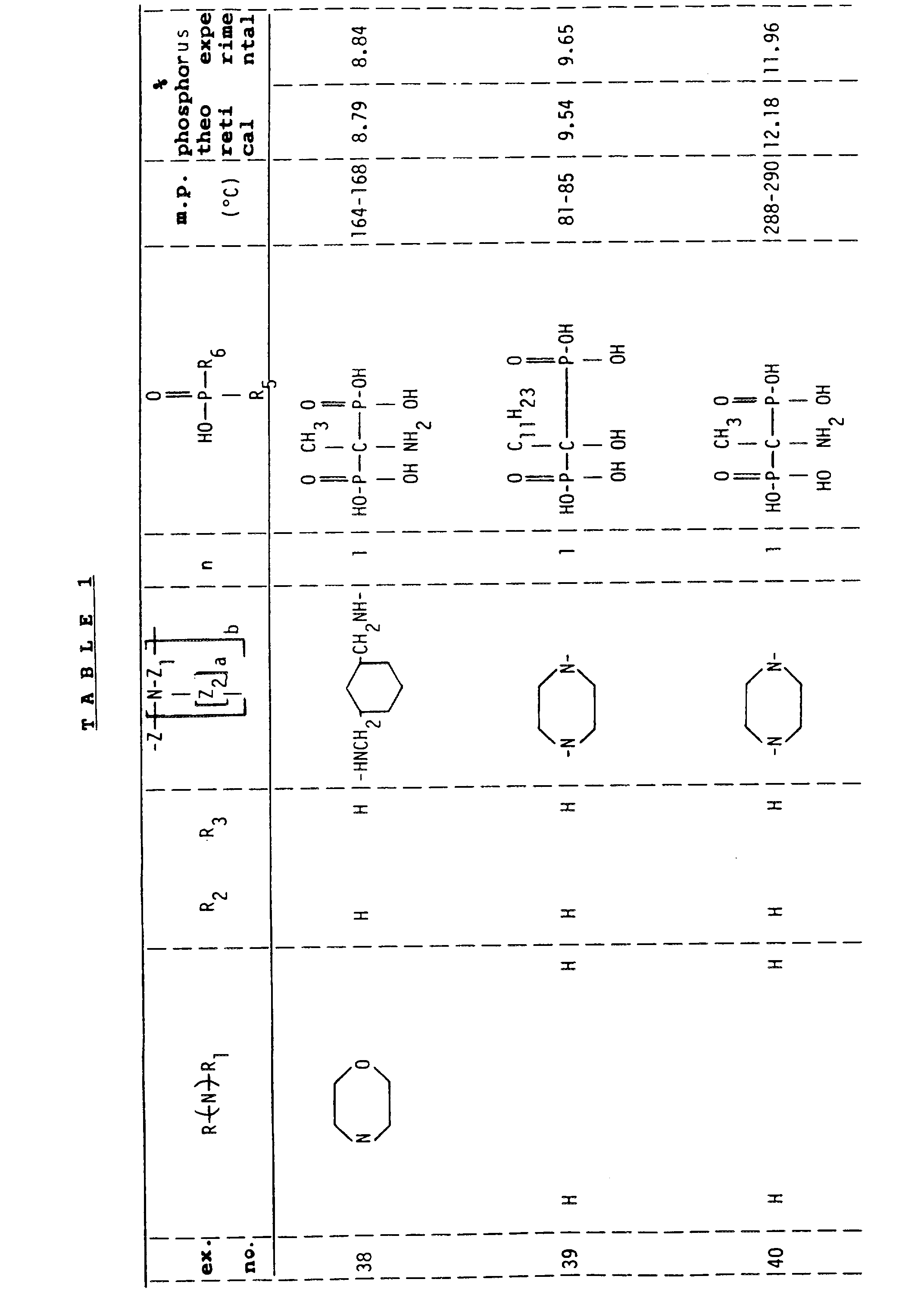

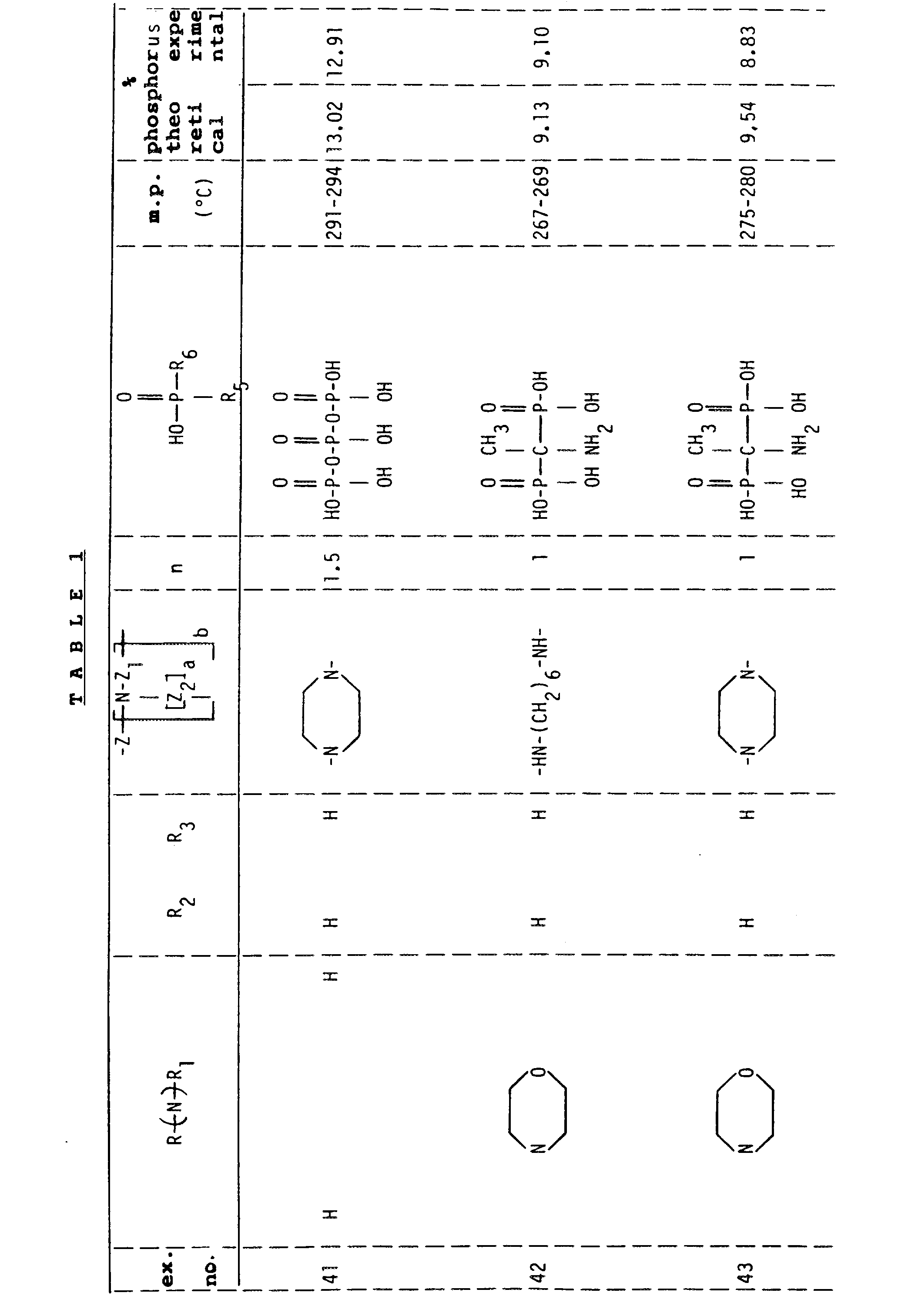

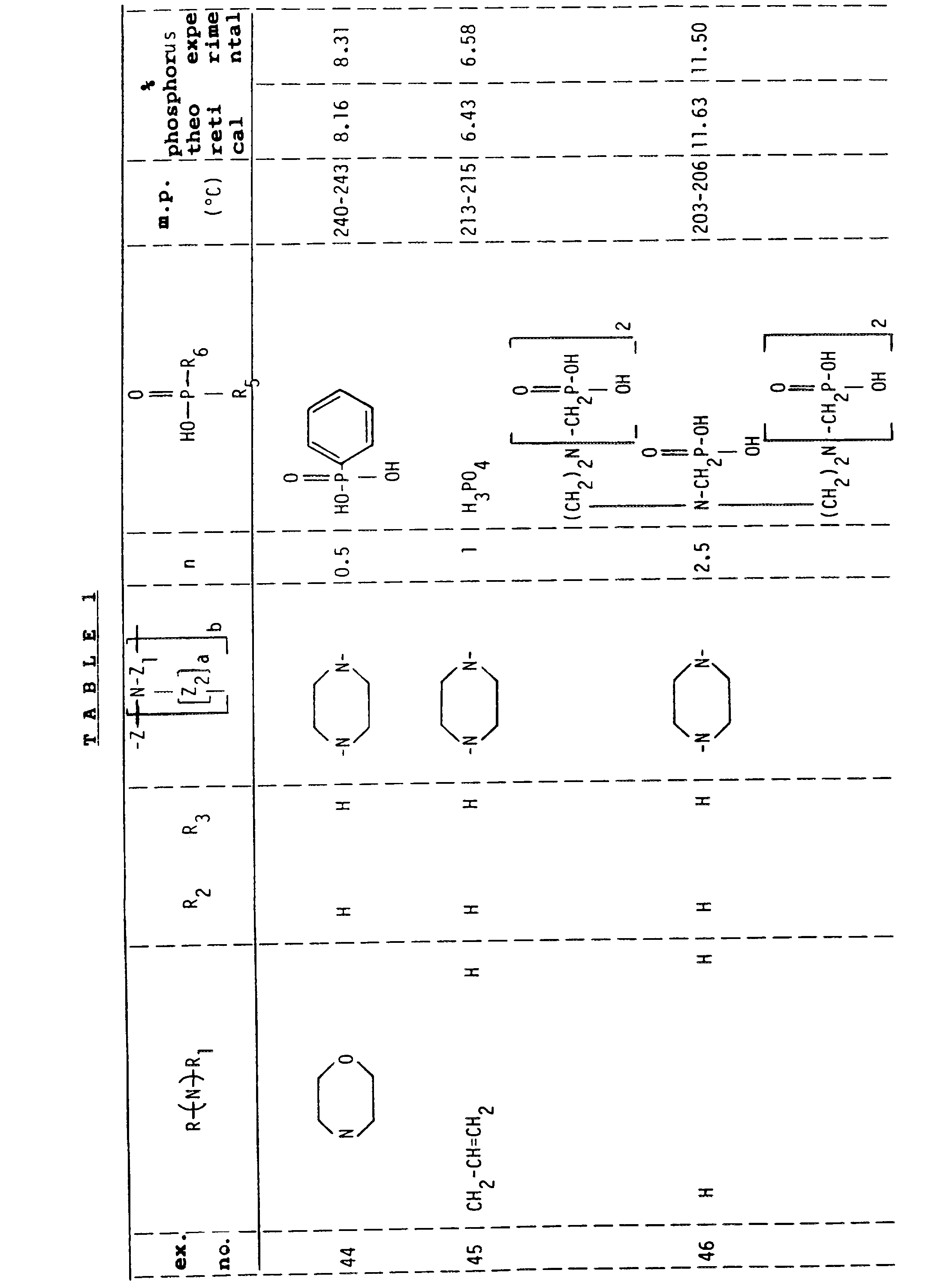

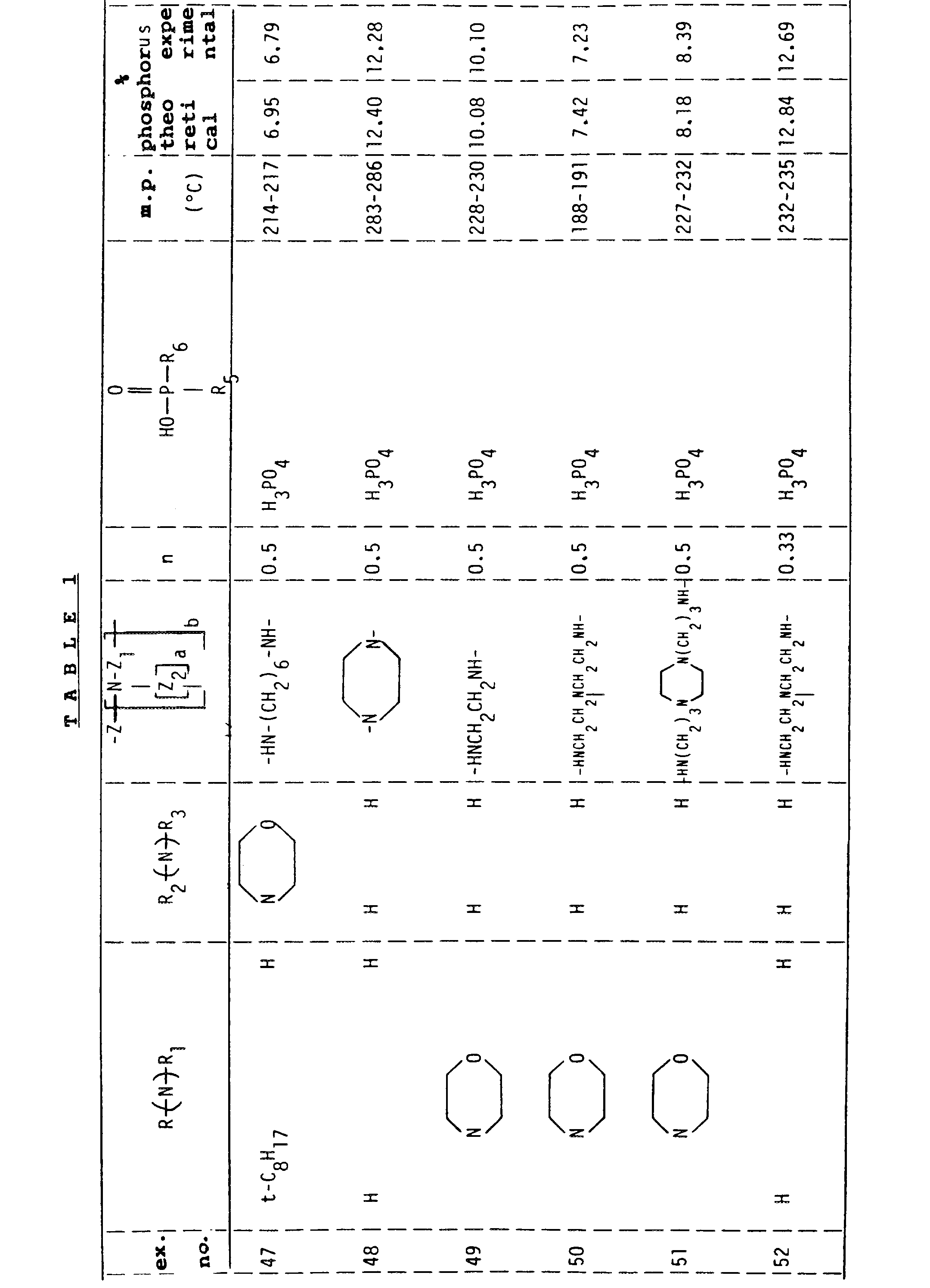

### EXAMPLES 53 to 136

The test results shown in the following tables 2 and 3 refer to polymeric compositions containing products of general formula (I), prepared according to the preceding examples.

Specimens were prepared, having a thickness of about 3 mm, by moulding mixtures of granular polymer and additives in a MOORE platen press, operating for 7 minutes at a pressure of 40 kg/cm.

The self-extinguishing level was determined one the above specimens by measuring the oxygen index (L.O.I. according to ASTM D-2863/77) in a Stanton Redcroft Instrument, and applying the "Vertical Burning Test", which allows classification of the material according to three ratings (V-0, V-1 and V-2, according to the UL 94 standard, issued by "Underwriters Laboratories", U.S.A.).

Table 2 shows the results obtained by using isotactic polypropylene in flake form and having a melt flow index (M.F.I.) of 12 and an insoluble fraction in boiling n-heptane of 96% by weight.

Table 3 shows the results obtained by using low density polyethylene in chip form and having an M.F.I. of 7; polystyrene in chip form, containing 5% by weight of butadiene and having an M.F.I. of 9; thermoplastic polyurethane, derived from both polyester (ESTANE 54600^{(R)} by Goodrich) and polyether (ESTANE 58300^{(R)} by Goodrich) in chip form and having a specific weight of 1.19 and 1.10 g/ml respectively; an elastomeric ethylene-propylene copolymer having a 45% by weight content of propylene; and an acrylonitrile-butadiene-styrene terpolymer having a specific weight of 1.06 g/ml and an M.F.I. of 1.6 and containing about 40% of each of acrylonitrile and styrene and 20% of butadiene.

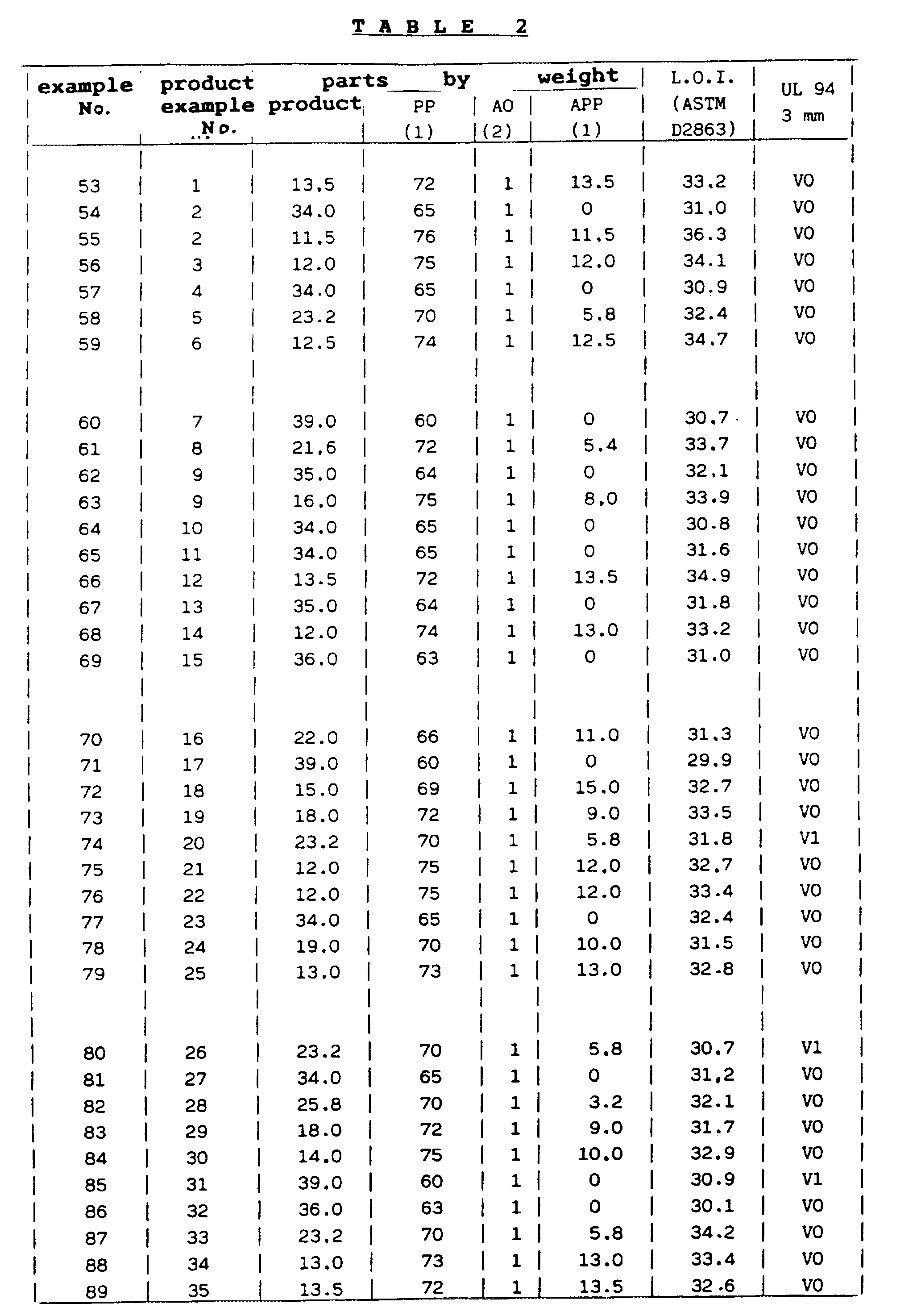

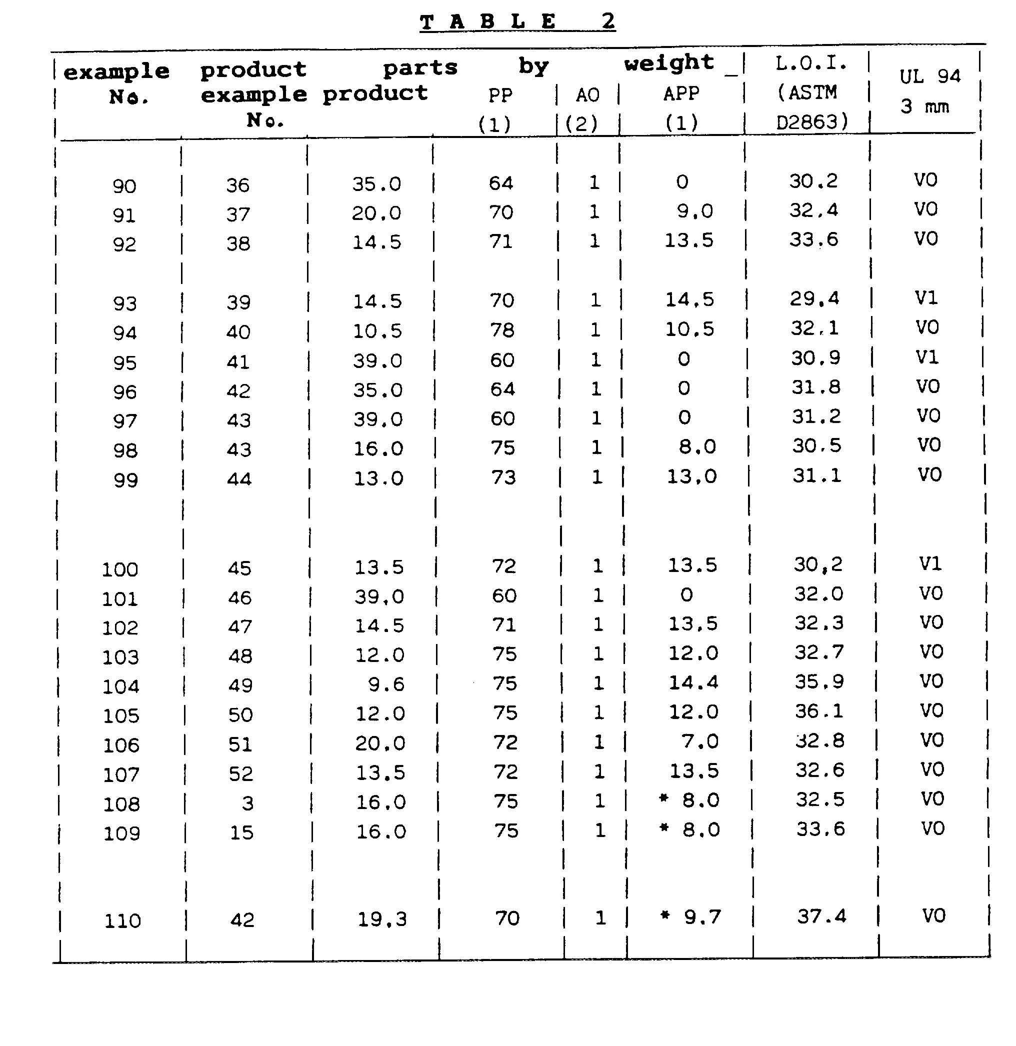

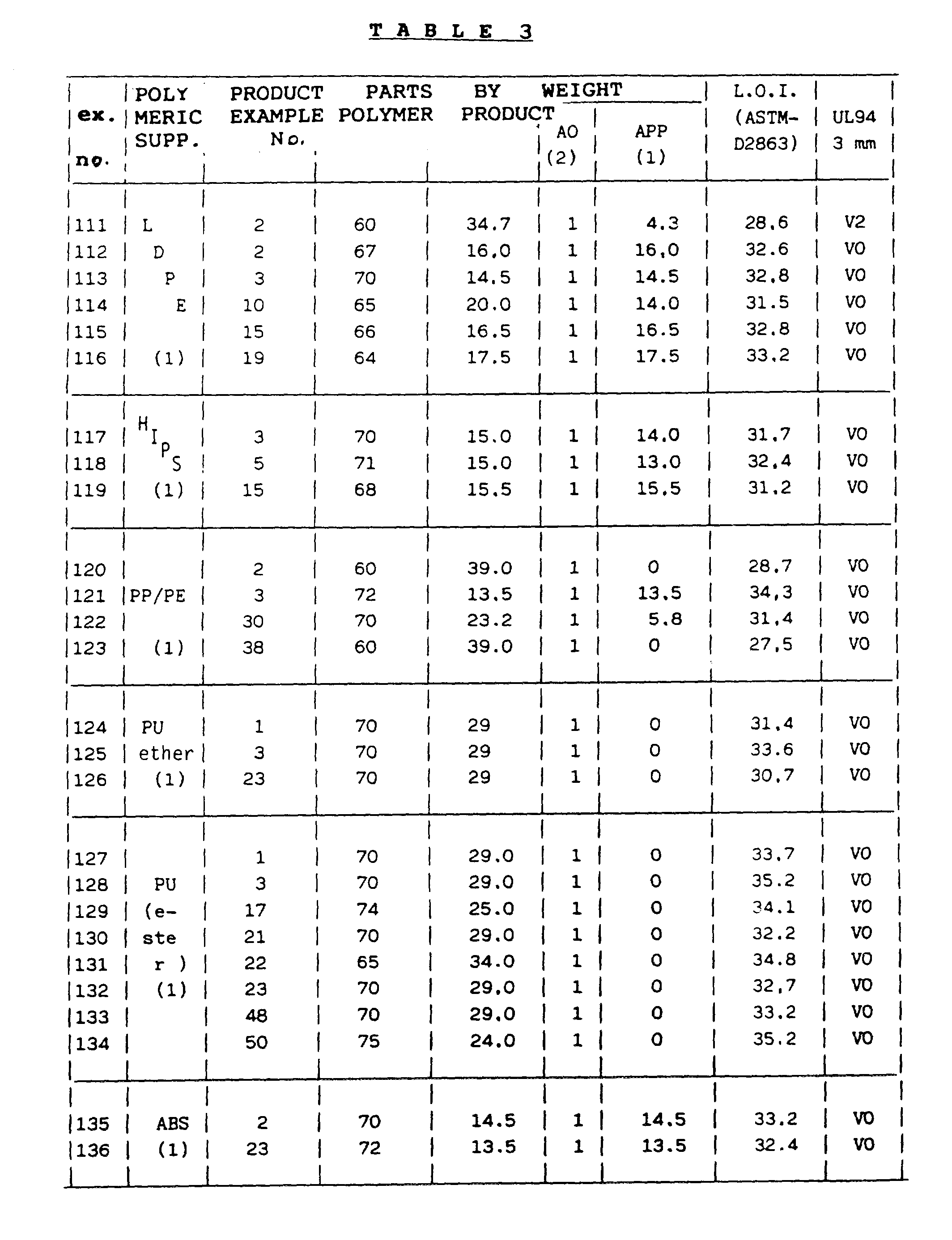

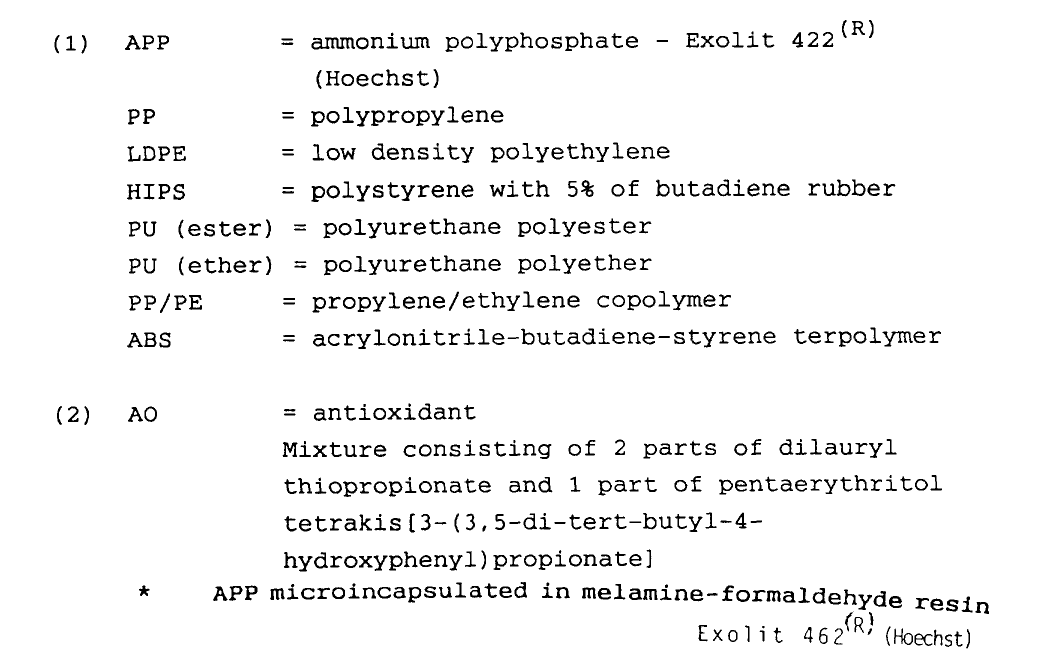

### EXAMPLE 137 (Comparison)

Following the procedure used in examples 53 to 110, but employing 2,4,6-triamino-1,3,5-triazine phosphate (1:1) as nitrogen-containing compound, the following composition was prepared:

| | |
|---|---|
| Polypropylene: | 65 parts by weight |
| Antioxidant: | 1 part by weight |
| 2,4,6-triamino-1,3,5-triazine phosphate (1:1): | 34 parts by weight |

Specimens were prepared, using the above composition, and self-extinguishing tests were carried out on these samples, according to the procedure previously described.

The following results were obtained:
- L.O.I. =: 23.5
- UL 94 = (3 mm) =: class B (the specimen burns).

### EXAMPLE 138 (Comparison)

Following the procedure described in example 137, the following composition was prepared:

| | |
|---|---|
| Polypropylene: | 73 parts by weight |
| Antioxidant: | 1 part by weight |
| Ammonium polyphosphate: | 13 parts by weight |
| 2,4,6-triamino-1,3,5-triazine phosphate (1:1): | 13 parts by weight |

Specimens were prepared, using the above composition and self-extinguishing tests were carried out on these samples, according to the procedure previously described.

The following results were obtained:
- L.O.I. =: 22.5
- UL 94 (3 mm) =: class B (the specimen burns).

## Claims

1. Salts of triazine derivatives with oxygenated acids of phosphorus, having general formula (I): 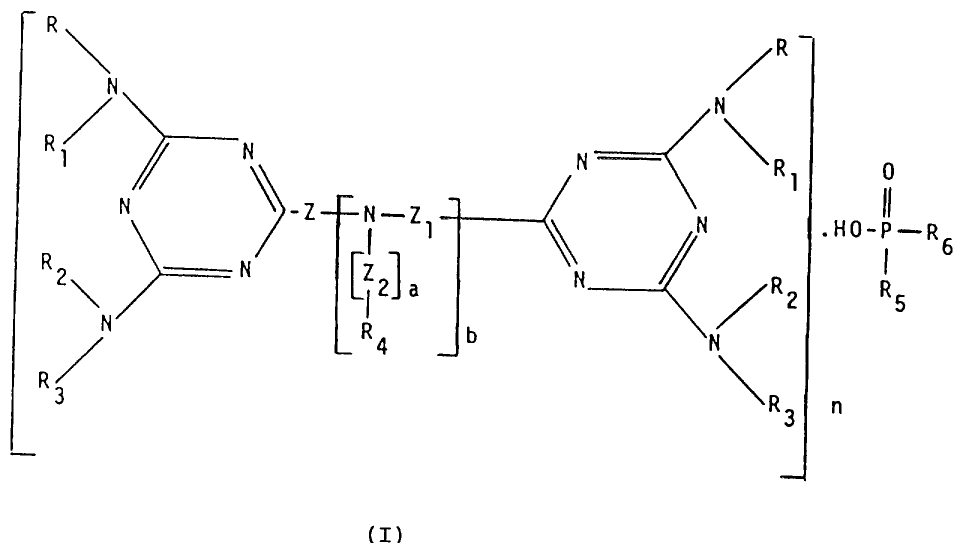 wherein:,
the radicals R, R₁, R₂ and R₃, the same of different from each other and having the same or different meanings for each triazine ring, are selected from H; C₁-C₁₈ alkyl; C₂-C₈ alkenyl; C₆-C₁₆ cycloalkyl and C₆-C₁₆ alkylcycloalkyl, optionally substituted with one or more hydroxy and/or C₁-C₄ hydroxyalkyl groups; provided that when the bivalent or polyvalent radicals defined below belong to those of general formulae (III) and (XII), respectively, the radical pair R₅/R₆ defined below is different from H/OH; 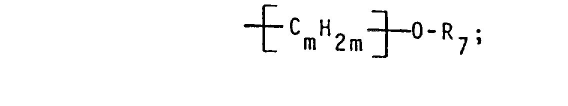 and 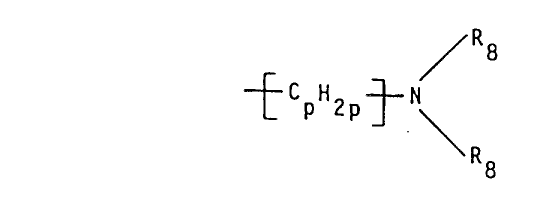 wherein:
m = an integer of from 2 to 8;
p = an integer of from 2 to 6;
R₇ = H; C₁-C₈ alkyl; C₂-C₆ alkenyl;
⁅C_{q}H_{2q}⁆O-R₉, q being an integer of from 1 to 4 and
R₉ being H or a C₁-C₄ alkyl group; C₆-C₁₂ cycloalkyl or C₆-C₁₂ alkyl cycloalkyl;
the R₈ radicals, the same or different from each other, are selected from H; C₁-C₈ alkyl; C₂-C₆ alkenyl; C₆-C₁₂ cycloalkyl or C₆-C₁₂ alkyl cycloalkyl; and C₁-C₄ hydroxyalkyl; provided that when the bivalent or polyvalent radicals defined below belong to those of general formulae (III) and (XII), respectively, the radical pair R₅/R₆ defined below is different from H/OH;
or the moiety N(R₈)₂ is replaced by an N-heterocyclic radical selected from aziridinyl; pyrrolidinyl; piperidinyl; morpholinyl; thiomorpholinyl; piperazinyl; 4-methylpiperazinyl; and 4-ethylpiperazinyl which is linked to the alkyl chain through the nitrogen atom;
or, in general formula (I), at least one of the moieties NRR₁ and NR₂R₃ is replaced by an N-heterocyclic radical selected from aziridinyl; pyrrolidinyl; piperidinyl; morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl; 2-ethylpiperazinyl;
and 2,5-diethylpiperazinyl which is linked to the triazine ring through the nitrogen atom;
a is 0 or 1
b is 0 or an integer of from 1 to 5;
R₄ is hydrogen or a group of general formula 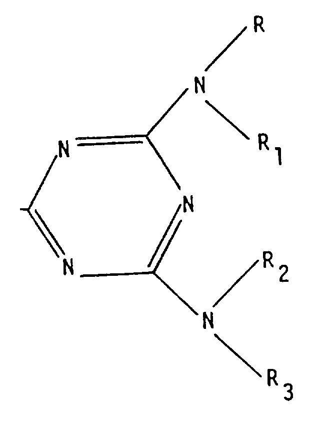 and its meaning can be different in each repeating unit;
when b is 0:
Z is selected from divalent radicals of the following formulae: 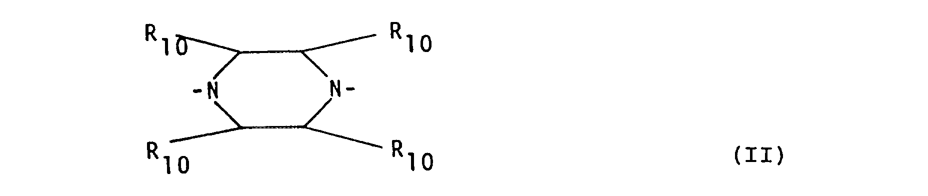 where the groups R₁₀, the same or different from each other, represent hydrogen or C₁-C₄ alkyl; 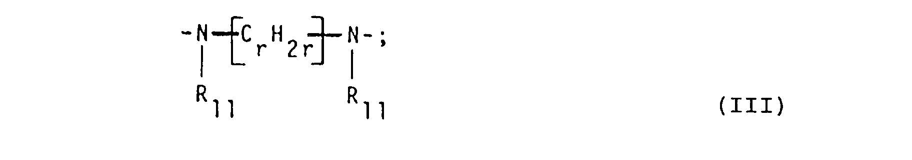 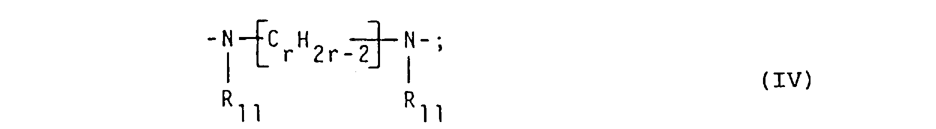 where r is an integer of from 2 to 14 and R₁₁ is hydrogen;
C₁-C₄ alkyl; C₂-C₆ alkenyl; or C₁-C₄ hydroxyalkyl; 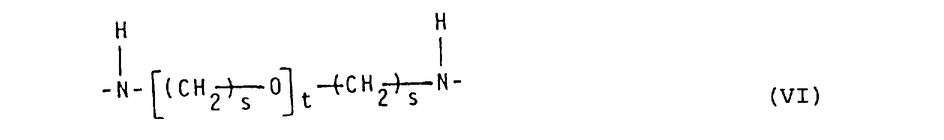 where s is an integer of from 2 to 5 and t is an integer of from 1 to 3; 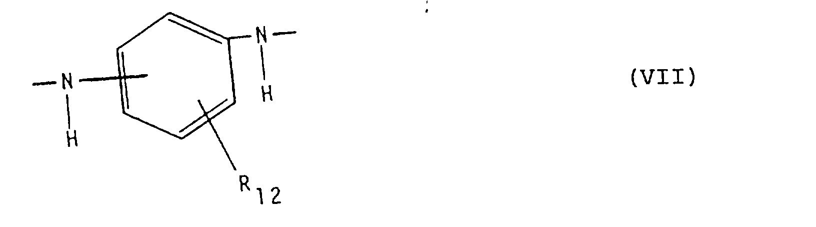 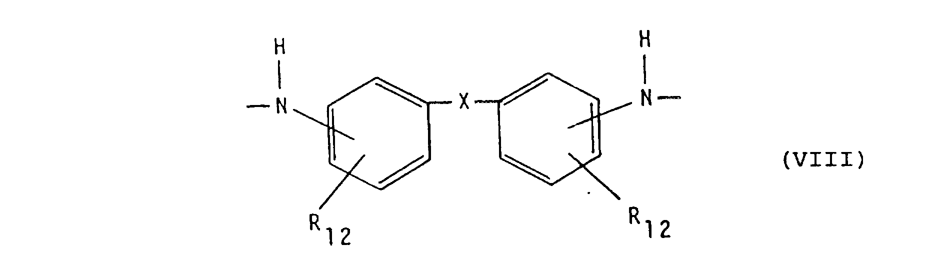 where
X represents a direct bond; O; S; S-S; SO; NH; NHSO₂; NHCO; N=N; or CH₂;
R₁₂ is hydrogen; hydroxy; C₁-C₄ alkyl; or
C₁-C₄ alkoxy; 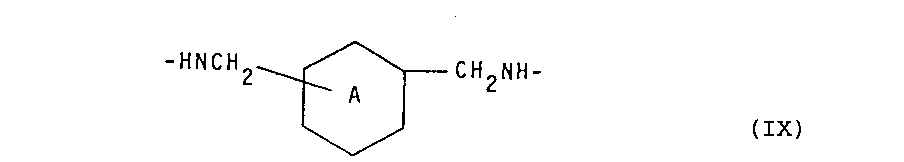
where A is a saturated or unsaturated ring; 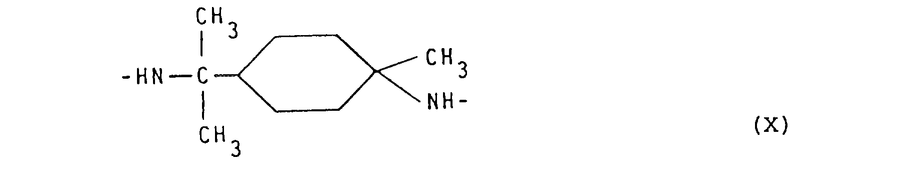 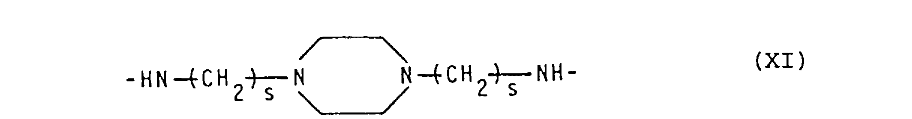 where s has the meaning previously defined;
when b is an integer of from 1 to 5:
the group 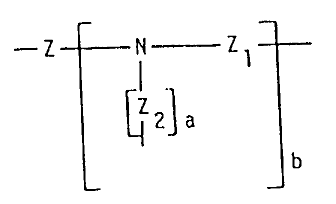 is a polyvalent radical represented by one of the following formulae: 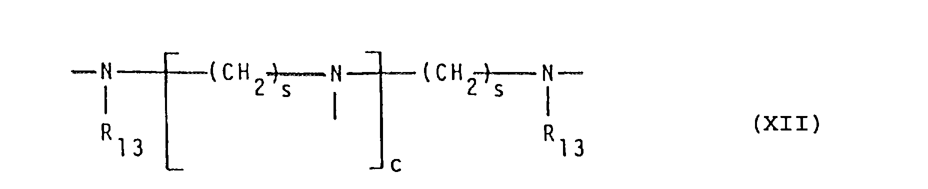 where:
R₁₃ is hydrogen or C₁-C₄ alkyl;
c is an integer of from 1 to 5;
the subscripts s, alike or different from each other,
have the meaning defined above; 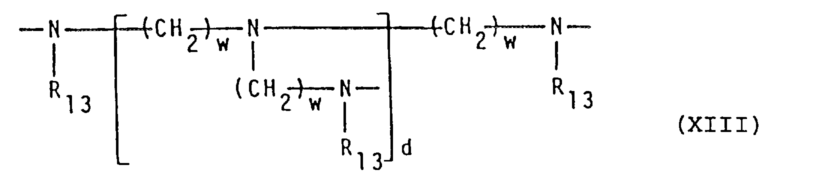 where:
R₁₃ has the meaning previously defined;
w is an integer of from 2 to 4;
d is 1 or 2;
n is a number from greater than 0 to 3;
R₅ is selected from H; OH; C₁-C₈ alkoxy; C₆-C₁₂ aryloxy, optionally substituted by a C₁-C₈ alkyl group; C₇-C₁₂ aralkyl, optionally substituted by a C₁-C₄ alkyl group; C₁-C₄ alkyl, optionally substituted by a carboxylic group; and C₆-C₁₂ aryl; R₆ is selected from H; OH; C₁-C₈ alkoxy; C₆-C₁₂ aryloxy; C₁-C₄ alkyl; C₆-C₁₂ aryl; a group of formula 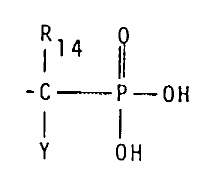 wherein:
R₁₄ is H or C₁-C₁₂ alkyl; and
Y is OH or R₁₄;
a group of formula 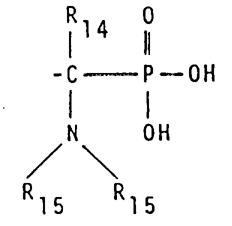 wherein:
R₁₄ is as defined above and the groups R₁₅, alike or different from each other, represent H or C₁-C₄ alkyl; or the moiety N(R₁₅)₂ is replaced by an N-heterocyclic radical which, optionally, contains another heteroatom and is linked to the carbon atom through the nitrogen atom;
a group of formula 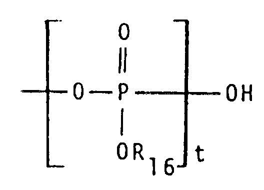 wherein:
R₁₆ is H or C₁-C₈ alkyl and t is an integer of from 1 to 3;
a group of formula 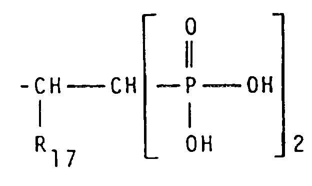 wherein:
R₁₇ is H or OH;
and groups of the following formulae: 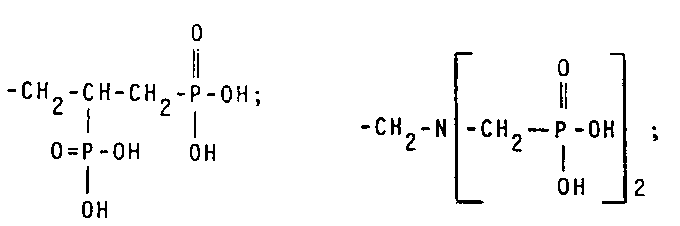 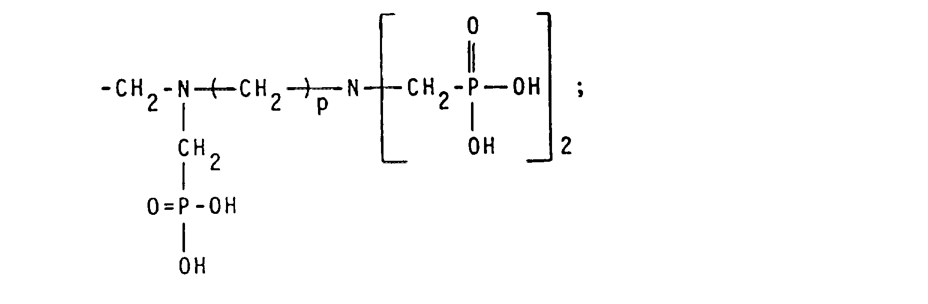 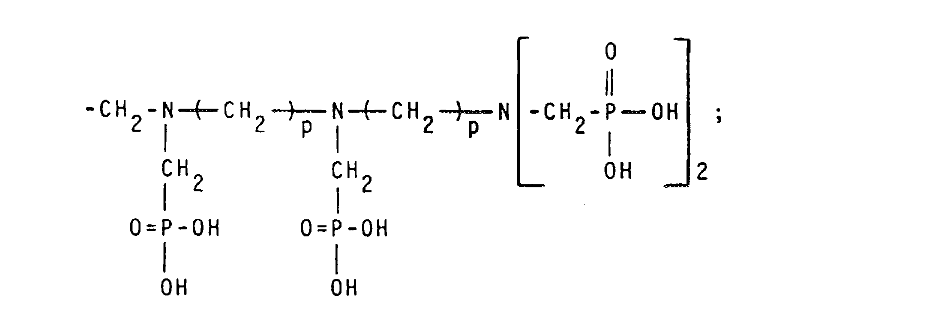 wherein:
p is an integer of from 2 to 6;
or R₅ and R₆ together form a cyclic structure of one
of the following formulae:

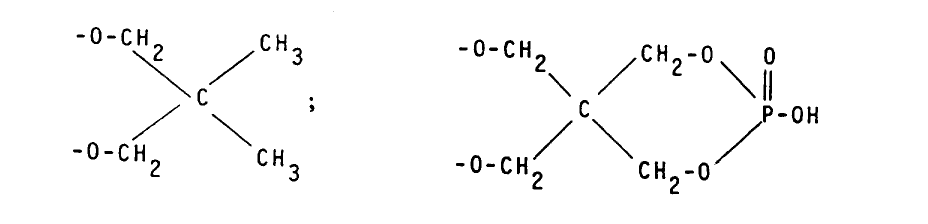

2. Salts according to claim 1, wherein at least one of the groups R, R₁, R₂ and R₃ in general formula (I) is replaced by a group of formula
⁅CₘH₂ₘ⁆O-R₇
where m is an integer of from 2 to 4 and R₇ is hydrogen or C₁-C₄ alkyl.

3. Salts according to any one of claims 1 and 2, wherein the acid of phosphorus is selected from
hypophosphorous acid; phosphorous acid; phosphoric acid; pyrophosphoric acid; tripolyphosphoric acid; ethane-1,1,2-triphosphonic acid; 2-hydroxyethane-1,1,2-triphosphonic acid; propane-1,2,3-triphosphonic acid; isopropylphosphoric acid; n-butylphosphoric acid; di-isopropylphosphoric acid; d-n-butylphosphoric acid; di-n-pentylphosphoric acid; isooctylphosphoric acid; hexylphosphoric acid; 2-ethylhexylphosphoric acid; ethylphosphoric acid; methylphosphonic acid; ethylphosphonic acid; n-propylphosphonic acid; n-butylphosphonic acid; aminomethylphosphonic acid; phenylphosphoric acid; phenylphosphonic acid; phenylphosphinic acid; di-n-butylpyrophosphoric acid; di(2-ethylhexyl)pyrophosphoric acid; octylphenylphosphoric acid; 2-methylbenzylphosphonic acid; 1-aminoethane-1,1-diphosphonic acid; 1-hydroxyethane-1,1-diphosphonic acid; 1-hydroxydodecane-1,1-diphosphonic acid; 1-(N-methylamino)ethane-1,1-diphosphonic acid; N,N-dimethylaminomethane-1,1-diphosphonic acid; N-butylaminomethane-1,1-diphosphonic acid; phosphonacetic acid; 2-phosphonopropionic acid; 3-phosphonopropionic acid; 2-phosphonobutyric acid; 4-phosphonobutyric acid; 2-hydroxy-5,5-dimethyl-2-oxo-1,3,2-dioxophosphorinane; 3,9-dihydroxy-2,4,8,10-tetroxo-3,9-diphosphaspiro[5.5]undecano-3,9-dioxide; amino-tris (methylene phosphonic) acid; ethylene diaminotetra(methylene phosphonic) acid; hexamethylene diaminotetra(methylene phosphonic) acid; diethylene triaminopenta(methylene phosphonic) acid.

4. Process for the preparation of salts of general formula (I) according to any one of claims 1 to 3, comprising the reaction of n moles of a derivative of 2,4,6-triamino-1,3,5-triazine of general formula (XIV): 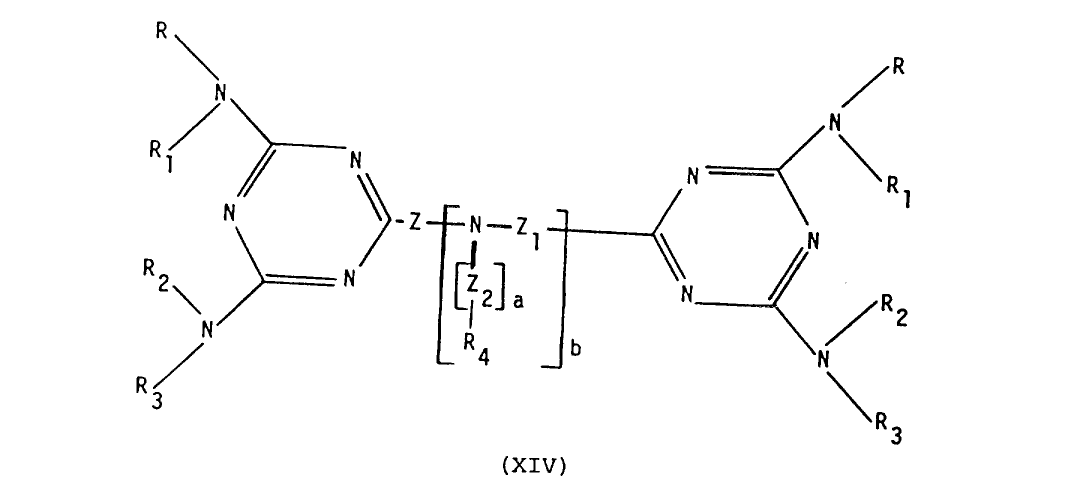 where n, the groups R, R₁, R₂, R₃ and R₄ and the radical 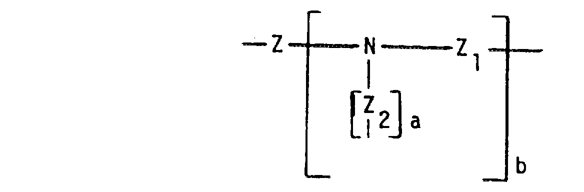 have the meanings given in claim 1, with one mole of a phosphorus-containing acid of general formula (XV): 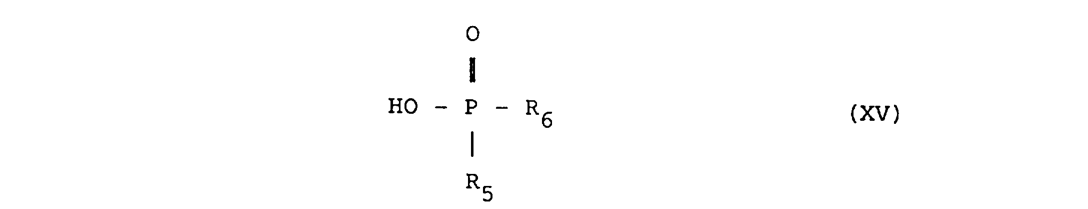 wherein R₅ and R₆ have the meanings given in claim 1.

5. Process according to claim 4, wherein the reaction between the derivative of general formula (XIV) and the phosphorus-containing acid of general formula (XV) is carried out in the presence of a solvent and at temperatures ranging from 0°C to the boiling point of the solvent used.

6. Self-extinguishing polymeric compositions, comprising
(a) from 90 to 40 parts by weight of a thermoplastic polymer and/or a polymer having elastomeric properties;
(b) from 10 to 60 parts by weight of one or more salts of 2,4,6-triamino-1,3,5-triazine derivatives with an oxygenated acid of phosphorus, said derivatives of 2,4,6-triamino-1,3,5-triazine having the general formula (XIV) 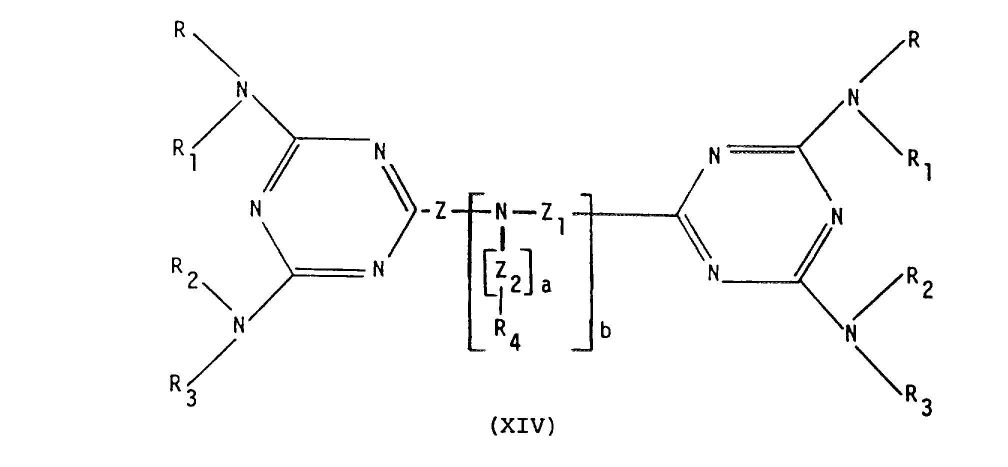 wherein:
the groups R, R₁, R₂, R₃ and R₄ and the radical 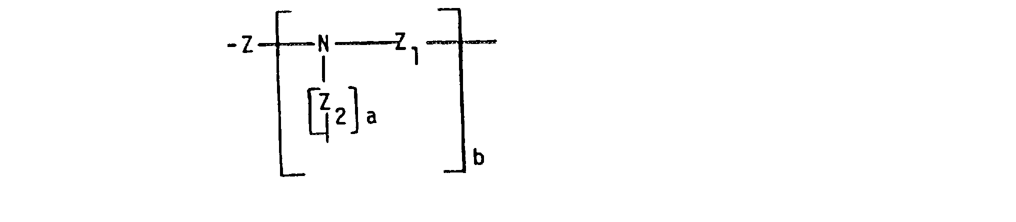
have the meanings given in any one of claims 1 and 2.

7. Compositions according to claim 6, wherein component (b) is selected from the salts of general formula (I): 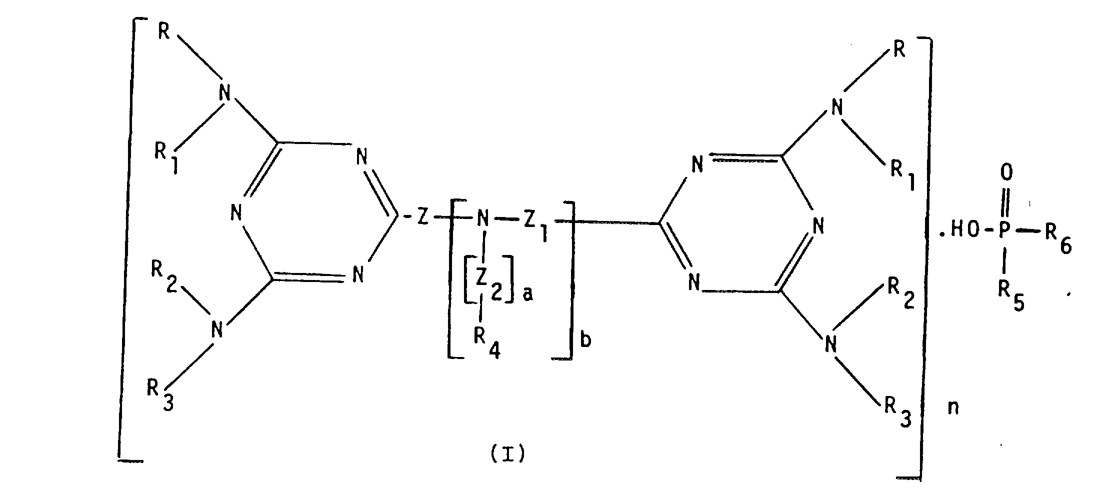 wherein:
n, the groups R to R₆ and the radical 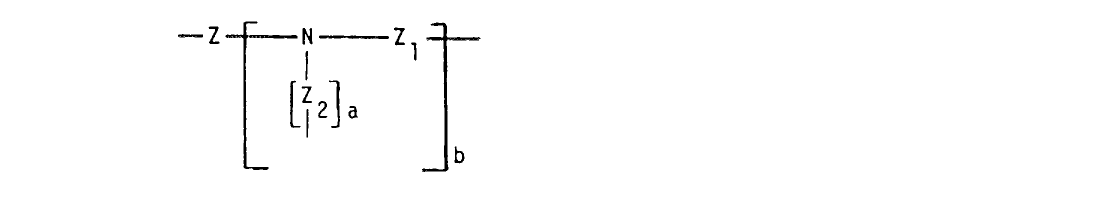 have the meanings given in any one of claims 1 and 2.

8. Self-extinguishing polymeric compositions comprising:
(a) from 90 to 40 parts by weight of component (a) as defined in claim 6;
(b) from 9 to 35 parts by weight of component (b) as defined in any one of claims 6 and 7;
(c) from 1 to 25 parts by weight of one or more compounds selected from ammonium and/or amine phosphates and/or phosphonates.

9. Compositions according to claim 8, wherein the ammonium phosphates (c) have the general formula (NH₄)ₙ₊₂PₙO₃ₙ₊₁, where n is an integer of at least 2; and/or the general formula (NH₄PO₃)ₙ, where n ranges from 50 to 500.

10. Compositions according to claim 8, wherein the amine phosphates (c) are selected from dimethylammonium or diethylammonium phosphate; ethylene diamine phosphate; melamine ortho- and pyrophosphate and mixtures thereof.

11. Compositions according to an one of claims 6 to 10,
wherein polymer (a) is selected from acrylonitrile/butadiene/styrene terpolymers (ABS); acrylonitrile/styrene copolymers (SAN); polyurethanes; poly(ethylene terephthalate); poly(butylene terephthalate); polyamides and polymers or copolymers of olefins of general formula R'-CH=CH₂, where R' is a hydrogen atom or a C₁-C₈ alkyl or aryl radical; particularly:
(1) Isotactic or prevailingly isotactic polypropylene;
(2) HDPE, LLDPE and LDPE polyethylene;
(3) Crystalline copolymers of propylene and minor amounts of ethylene and/or other alpha-olefins, such as 1-butene, 1-hexene, 1-octene and 4-methyl-1-pentene;
(4) Heterophasic compositions comprising
(A) a homopolymeric fraction of propylene and/or a fraction of one of the copolymers specified under (3) above and (B) a copolymeric fraction composed of elastomeric copolymers of ethylene with an alpha-olefin, optionally containing minor amounts of a diene, wherein the alpha-olefin is preferably selected from propylene and 1-butene; and
(5) Elastomeric copolymers of ethylene with alphaolefins, optionally containing minor amounts of a diene.

12. Moulded articles, obtained from the compositions of any one of claims 6 to 11.

## Patentansprüche

1. Salze von Triazin-Derivaten mit Sauerstoffsäuren von Phosphor, die allgemeine Formel (I) aufweisend: 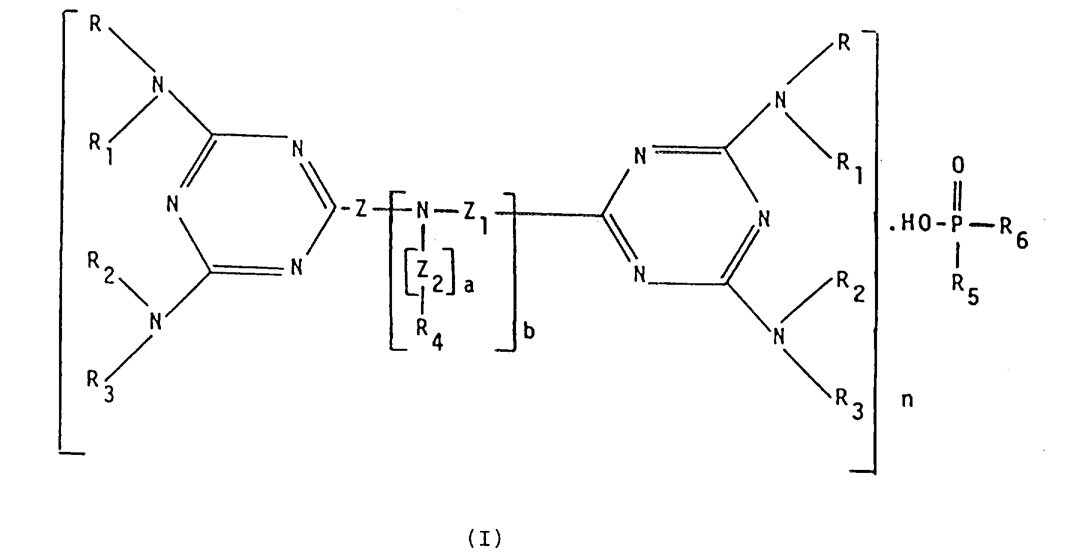 worin:
die Reste R, R₁, R₂ und R₃, gleich oder verschieden voneinander und in jedem Triazin-Ring dieselben oder unterschiedliche Bedeutungen aufweisend, ausgewählt sind aus H; C₁-C₁₈-Alkyl; C₂-C₈-Alkenyl; C₆-C₁₆-Cycloalkyl und C₆-C₁₆-Alkylcycloalkyl, gegebenenfalls substituiert mit einer oder mehreren Hydroxy- und/oder C₁-C₄-Hydroxyalkylgruppen; mit der Maßgabe, daß wenn die unten definierten zweiwertigen oder mehrwertigen Reste zu denjenigen der allgemeinen Formeln (III) bzw. (XII) gehören, das unten definierte Reste-Paar R₅/R₆ von H/OH verschieden ist;
⁅CₘH₂ₘ⁆O-R₇;
und 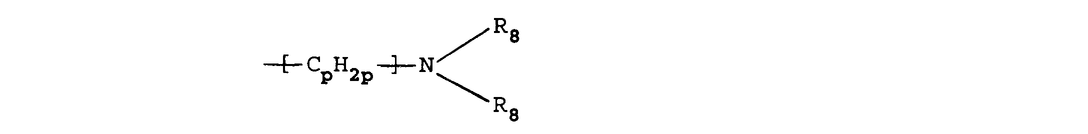 worin:
m = eine ganze Zahl von 2 bis 8;
p = eine ganze Zahl von 2 bis 6;
R₇ = H; C₁-C₈-Alkyl; C₂-C₆-Alkenyl; -[-C_{q}H_{2q}-]-O-R₉, wobei q eine ganze Zahl von 1 bis 4 ist und R₉ für H oder eine C₁-C₄-Alkylgruppe steht; C₆-C₁₂-Cycloalkyl oder C₆-C₁₂-Alkylcycloalkyl;
die Reste R₈, gleich oder verschieden voneinander, ausgewählt sind aus H; C₁-C₈-Alkyl; C₂-C₆-Alkenyl;
C₆-C₁₂-Cycloalkyl oder C₆-C₁₂-Alkylcycloalkyl; und
C₁-C₄-Hydroxyalkyl; mit der Maßgabe, daß wenn die unten definierten zweiwertigen oder mehrwertigen Reste zu denjenigen der allgemeinen Formeln (III) bzw. (XII) gehören, das unten definierte Reste-Paar R₅/R₆ von H/OH verschieden ist;
oder die Einheit N(R₈)₂ durch einen N-heterocyclischen Rest ersetzt ist, der aus Aziridinyl; Pyrrolidinyl; Piperidinyl; Morpholinyl; Thiomorpholinyl; Piperazinyl; 4-Methylpiperazinyl; und 4-Ethylpiperazinyl ausgewählt ist und über das Stickstoffatom an die Alkylkette gebunden ist;
oder in der allgemeinen Formel (I) mindestens eine der Einheiten NRR₁ und NR₂R₃ durch einen N-heterocyclischen Rest ersetzt ist, der aus Aziridinyl; Pyrrolidinyl; Piperidinyl; Morpholinyl; Thiomorpholinyl; Piperazinyl; 4-Methylpiperazinyl; 4-Ethylpiperazinyl; 2-Methylpiperazinyl; 2,5-Dimethylpiperazinyl; 2,3,5,6-Tetramethylpiperazinyl; 2,2,5,5-Tetramethylpiperazinyl; 2-Ethylpiperazinyl; und 2,5-Diethylpiperazinyl ausgewählt ist und über das Stickstoffatom an den Triazin-Ring gebunden ist;
a 0 oder 1 ist
b 0 oder eine ganze Zahl von 1 bis 5 ist;
R₄ Wasserstoff oder eine Gruppe der allgemeinen Formel 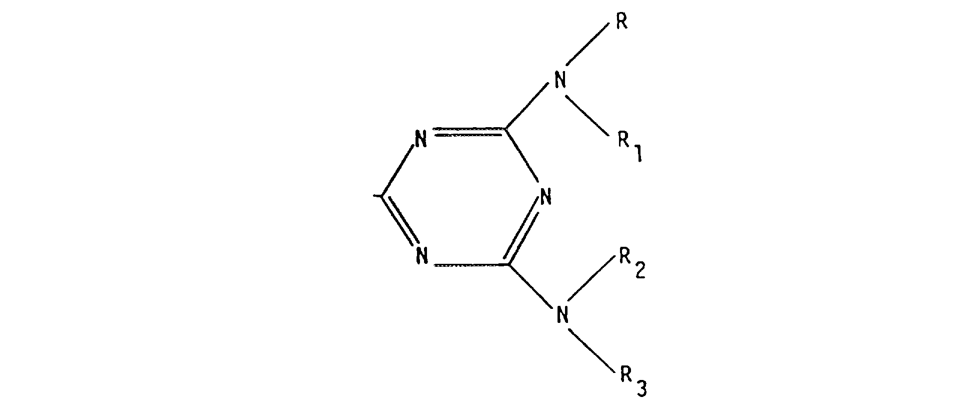 ist und seine Bedeutung in jeder wiederkehrenden Einheit unterschiedlich sein kann;
wenn b für 0 steht:
Z ausgewählt ist aus zweiwertigen Resten der folgenden Formeln: 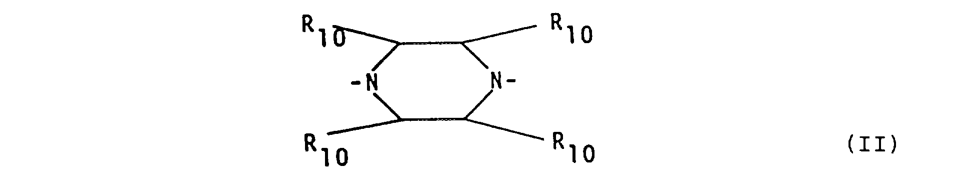 worin die Gruppen R₁₀, gleich oder verschieden voneinander, für Wasserstoff oder C₁-C₄-Alkyl stehen; 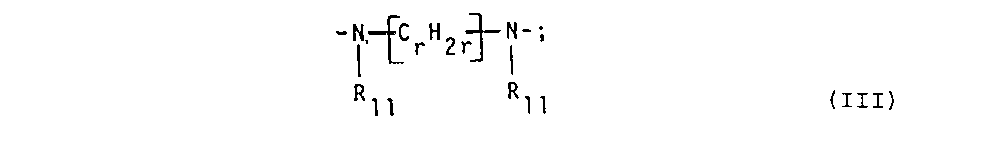 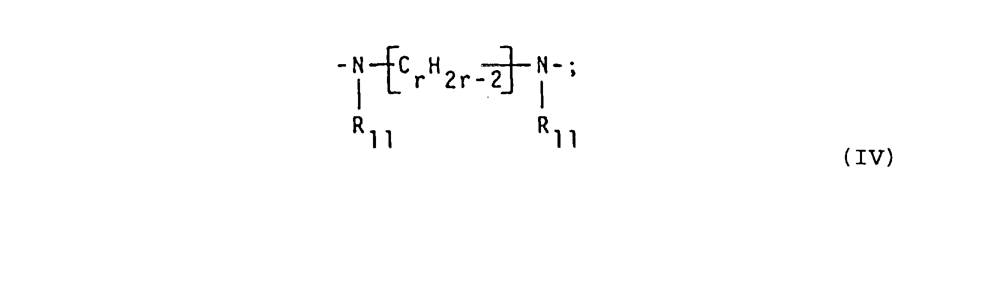 worin r eine ganze Zahl von 2 bis 14 ist und R₁₁ für Wasserstoff; C₁-C₄-Alkyl; C₂-C₆-Alkenyl; oder Cₗ-C₄-Hydroxyalkyl steht; 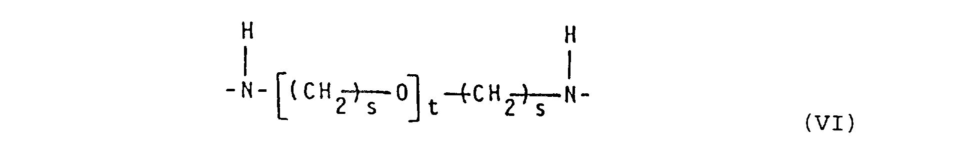 worin s eine ganze Zahl von 2 bis 5 ist und t eine ganze Zahl von 1 bis 3 ist; 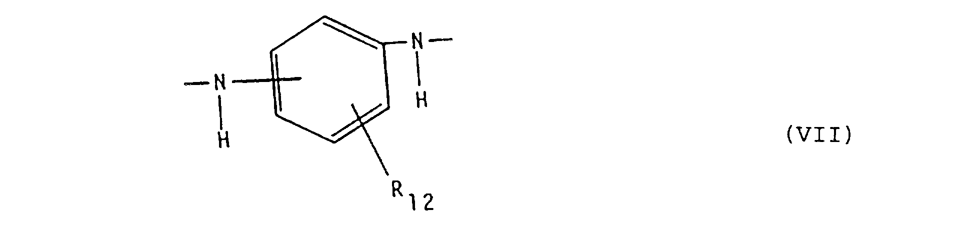 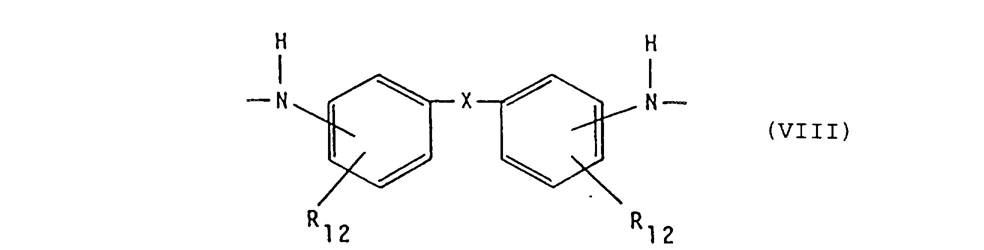 worin
X für eine direkte Bindung; O; S; S-S; SO; NH; NHSO₂; NHCO; N=N; oder CH₂ steht;
R₁₂ Wasserstoff; Hydroxy; C₁-C₄-Alkyl; oder C₁-C₄-Alkoxy ist; 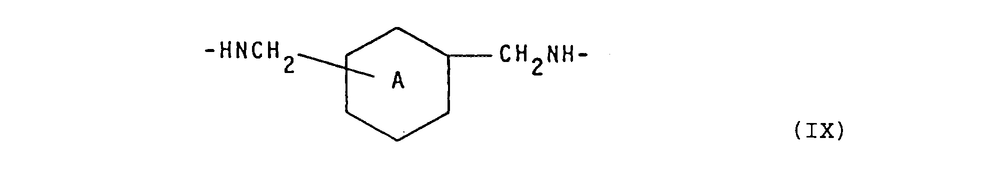 worin A ein gesättigter oder ungesättigter Ring ist; 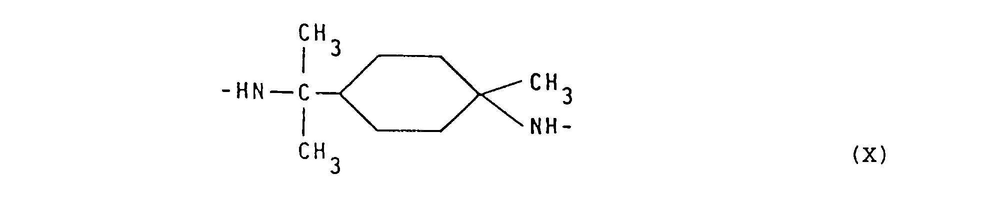 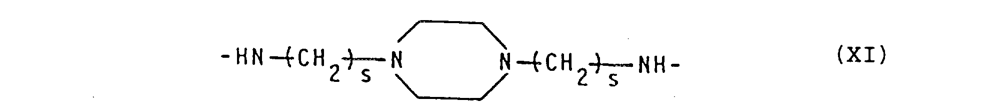 worin s dieselbe Bedeutung wie oben definiert aufweist;
wenn b für eine ganze Zahl von 1 bis 5 steht:
die Gruppe 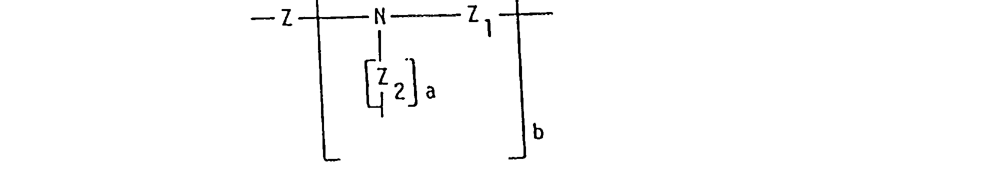 ein mehrwertiger Rest ist, der durch eine der folgenden Formeln dargestellt wird: 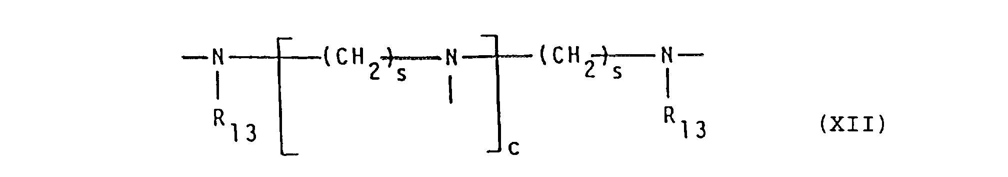 worin:
R₁₃ Wasserstoff oder C₁-C₄-Alkyl bedeutet;
c eine ganze Zahl von 1 bis 5 ist;
die Indices s, gleich oder verschieden voneinander, die oben definierte Bedeutung aufweisen; 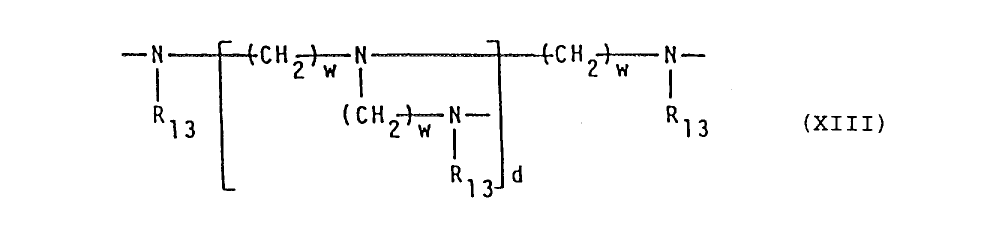 worin:
R₁₃ die oben definierte Bedeutung aufweist;
w eine ganze Zahl von 2 bis 4 ist;
d 1 oder 2 ist;
n eine ganze Zahl von größer als 0 bis 3 ist;
R₅ ausgewählt ist aus H; OH; C₁-C₈-Alkoxy; C₆-C₁₂-Aryloxy, gegebenenfalls mit einer C₁-C₈-Alkylgruppe substituiert; C₇-C₁₂-Aralkyl, gegebenenfalls mit einer C₁-C₄-Alkylgruppe substituiert; C₁-C₄-Alkyl, gegebenenfalls mit einer Carboxylgruppe substituiert; und
C₆-C₁₂ Aryl;
R₆ ausgewählt ist aus H; OH; C₁-C₈-Alkoxy; C₆-C₁₂-Aryloxy; C₁-C₄ Alkyl; C₆-C₁₂-Aryl; einer Gruppe der Formel 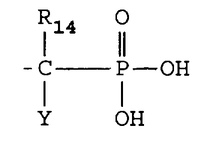 worin:
R₁₄ H oder C₁-Cₗ₂-Alkyl bedeutet; und
Y OH oder R₁₄ ist;
einer Gruppe der Formel 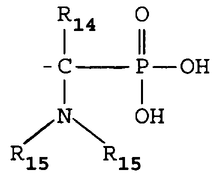 worin:
R₁₄ wie oben definiert ist und die Gruppen R₁₅, gleich oder verschieden voneinander, für H oder C₁-C₄-Alkyl stehen; oder die Einheit N(R₁₅)₂ durch einen N-heterocyclischen Rest, der gegebenenfalls ein weiteres Heteroatom enthält und über das Stickstoffatom an das Kohlenstoffatom gebunden ist, ersetzt ist;
einer Gruppe der Formel 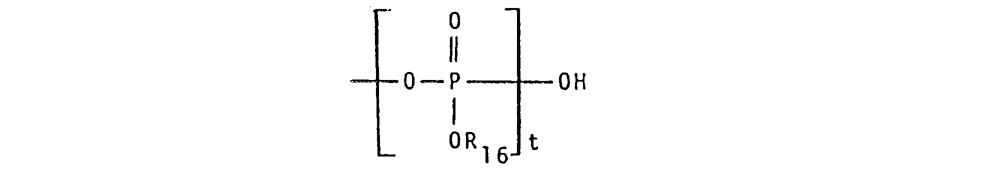 worin:
R₁₆ für H oder C₁-C₈-Alkyl steht und t eine ganze Zahl von 1 bis 3 ist;
einer Gruppe der Formel 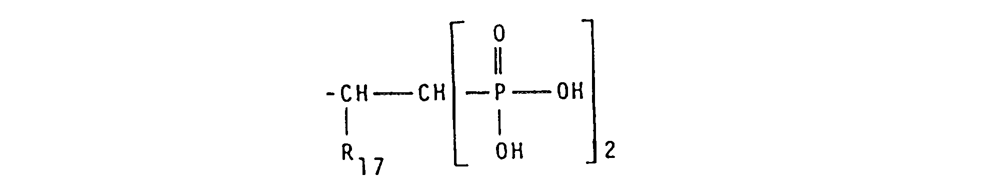 worin:
R₁₇ für H oder OH steht;
und Gruppen der folgenden Formeln: 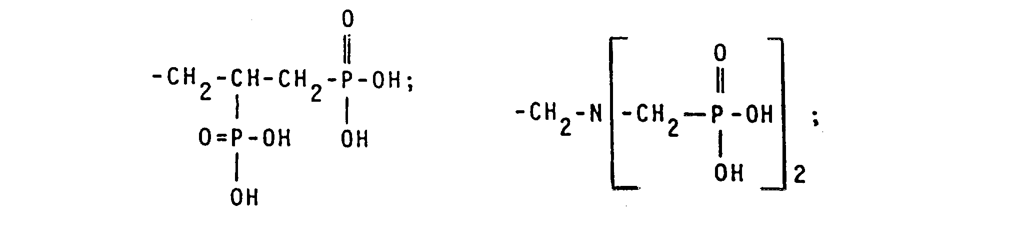 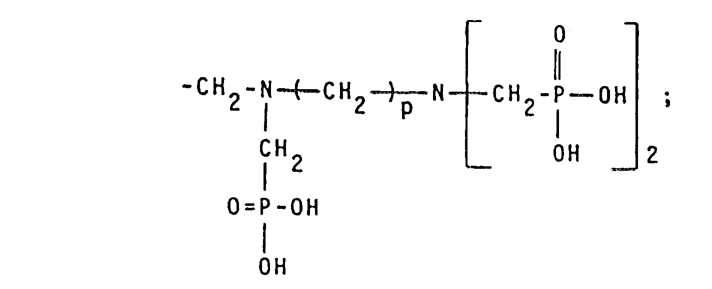 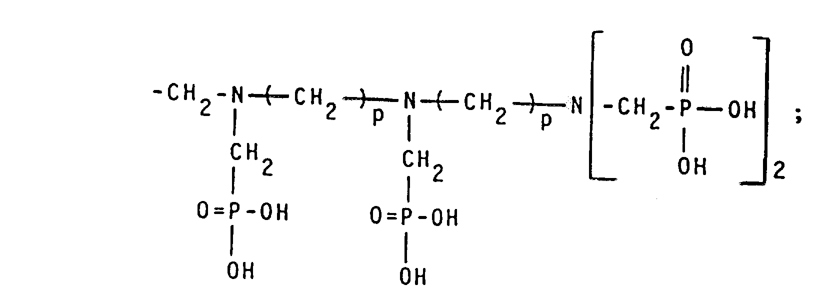 worin:
p eine ganze Zahl von 2 bis 6 ist;
oder R₅ und R₆ zusammen eine cyclische Struktur mit einer der folgenden Formeln bilden:

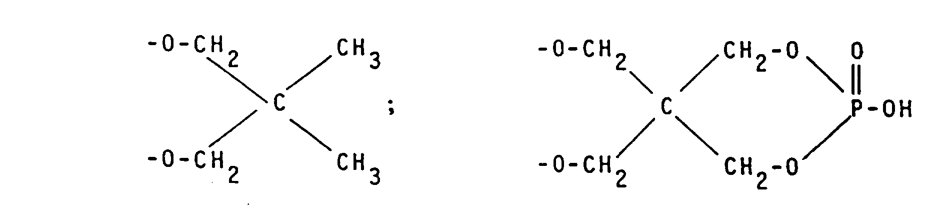

2. Salze nach Anspruch 1, in denen mindestens eine der Gruppen R, R₁, R₂ und R₃ in der allgemeinen Formel (I) durch eine Gruppe der Formel
⁅CₘH₂ₘ⁆O-R₇
ersetzt ist, worin m eine ganze Zahl von 2 bis 4 ist und R₇ Wasserstoff oder C₁-C₄-Alkyl bedeutet.

3. Salze nach irgendeinem der Ansprüche 1 und 2, in denen die Säure von Phosphor ausgewählt ist aus unterphosphoriger Säure; phosphoriger Säure; Phosphorsäure; Pyrophosphorsäure; Tripolyphosphorsäure; Ethan-1,1,2-triphosphonsäure; 2-Hydroxyethan-1,1,2-triphosphonsäure; Propan-1,2,3-triphosphonsäure; Isopropylphosphorsäure; n-Butylphosphorsäure; Diisopropylphosphorsäure; Di-n-butylphosphorsäure; Di-n-pentylphosphorsäure; Isooctylphosphorsäure; Hexylphosphorsäure; 2-Ethylhexylphosphorsäure; Ethylphosphorsäure; Methylphosphonsäure; Ethylphosphonsäure; n-Propylphosphonsäure; n-Butylphosphonsäure; Aminomethylphosphonsäure; Phenylphosphorsäure; Phenylphosphonsäure; Phenylphosphinsäure; Di-n-butylpyrophosphorsäure; Di (2-ethylhexyl) pyrophosphorsäure; Octylphenylphosphorsäure; 2-Methylbenzylphosphonsäure; 1-Aminoethan-1,1-diphosphonsäure; 1-Hydroxyethan-1,1-diphosphonsäure; 1-Hydroxydodecan-1,1-diphosphonsäure; 1-(N-Methylamino)ethan-1,1-diphosphonsäure; N,N-Dimethylaminomethan-1,1-diphosphonsäure; N-Butylaminomethan-1,1-diphosphonsäure; Phosphonoessigsäure; 2-Phosphonopropionsäure; 3-Phosphonopropionsäure; 2-Phosphonobuttersäure; 4-Phosphonobuttersäure; 2-Hydroxy-5,5-dimethyl-2-oxo-1,3,2-dioxophosphorinan; 3,9-Dihydroxy-2,4,8,10-tetroxo-3,9-diphosphaspiro[5.5]undecano-3,9-dioxid; Aminotris(methylenphosphon)säure; Ethylendiaminotetra(methylenphosphon)säure; Hexamethylendiaminotetra(methylenphosphon)säure; Diethylentriaminopenta(methylenphosphon)säure.

4. Verfahren zur Herstellung von Salzen der allgemeinen Formel (I) nach irgendeinem der Ansprüche 1 bis 3, umfassend die Umsetzung von n Molen eines Derivats von 2,4,6-Triamino-1,3,5-triazin der allgemeinen Formel (XIV) : 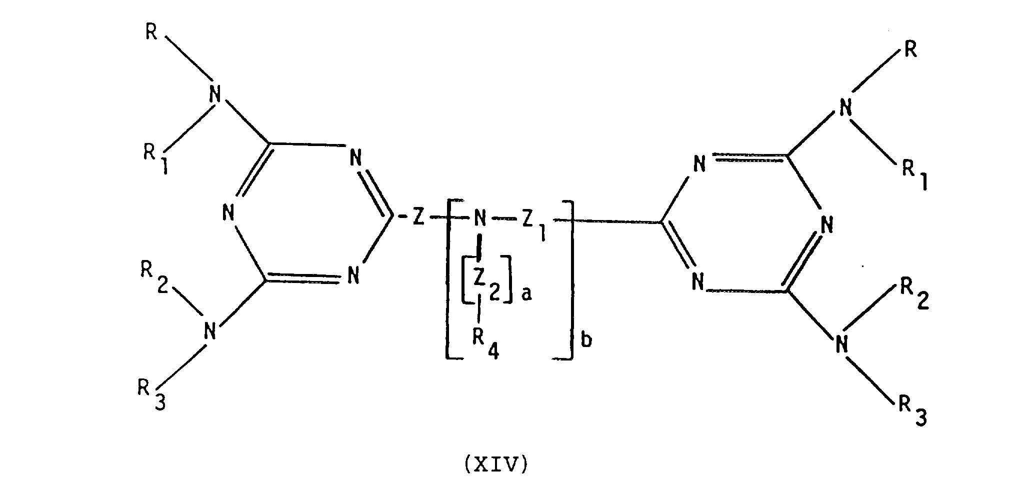 worin n, die Gruppen R, R₁, R₂, R₃ und R₄ und der Rest 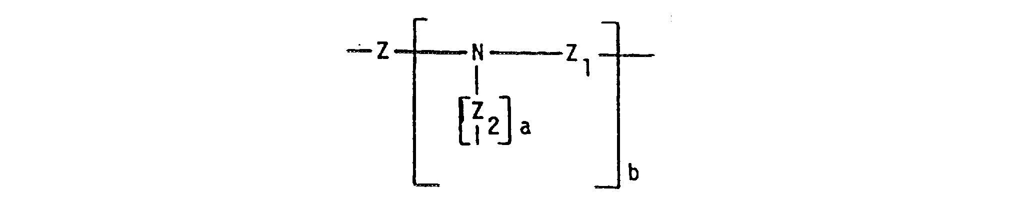 dieselben Bedeutungen wie in Anspruch 1 angegeben aufweisen, mit einem Mol einer Phosphor-haltigen Säure der allgemeinen Formel (XV): 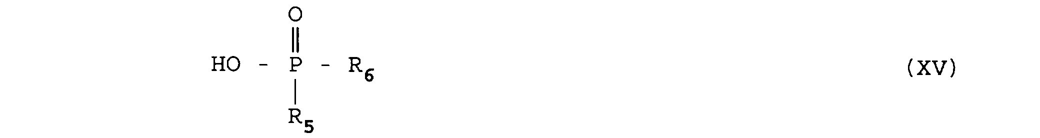 worin R₅ und R₆ dieselbe Bedeutung wie in Anspruch 1 angegeben aufweisen.

5. Verfahren nach Anspruch 4, in welchem die Umsetzung zwischen dem Derivat der allgemeinen Formel (XIV) und der Phosphor-haltigen Säure der allgemeinen Formel (XV) in Anwesenheit eines Lösungsmittels und bei Temperaturen im Bereich von 0°C bis zum Siedepunkt des eingesetzten Lösungsmittels durchgeführt wird.

6. Selbstverlöschende polymere Zusammensetzungen, umfassend
(a) 90 bis 40 Gewichtsteile eines thermoplastischen Polymeren und/oder eines Polymeren mit elastomeren Eigenschaften;
(b) 10 bis 60 Gewichtsteile eines oder mehrerer Salze von 2,4,6-Triamino-1,3,5-triazin-Derivaten mit einer Sauerstoffsäure von Phosphor, wobei diese Derivate von 2,4,6-Triamino-1,3,5-triazin die allgemeine Formel (XIV) aufweisen 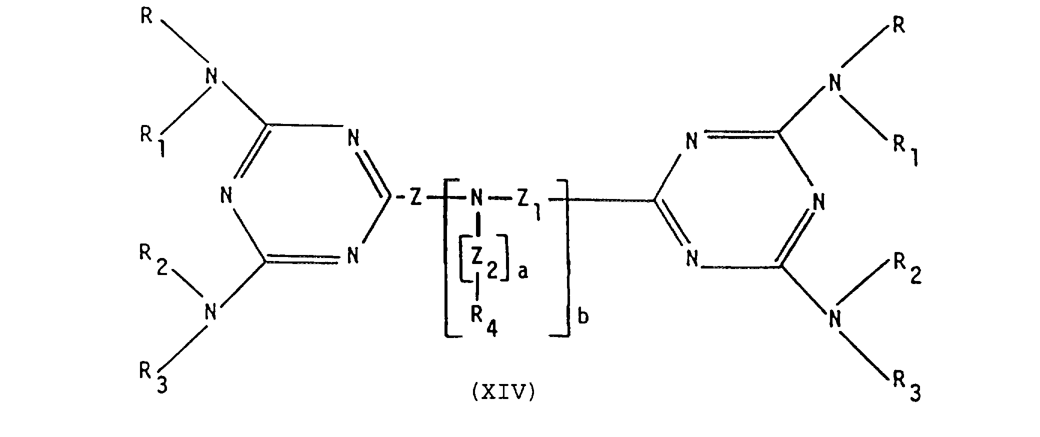 worin die Gruppen R, R₁, R₂, R₃ und R₄ und der Rest 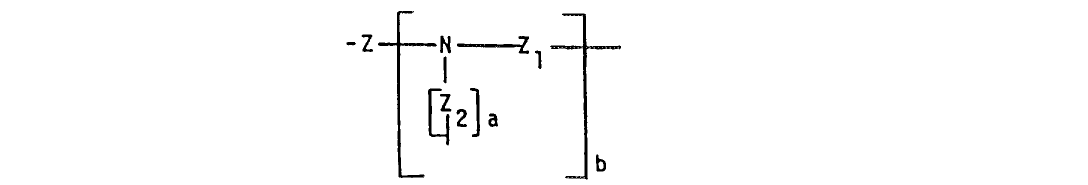 dieselben Bedeutungen wie in irgendeinem der Ansprüche 1 und 2 angegeben aufweisen.

7. Zusammensetzungen nach Anspruch 6, in welchen die Komponente (b) ausgewählt ist aus den Salzen der allgemeinen Formel (I) : 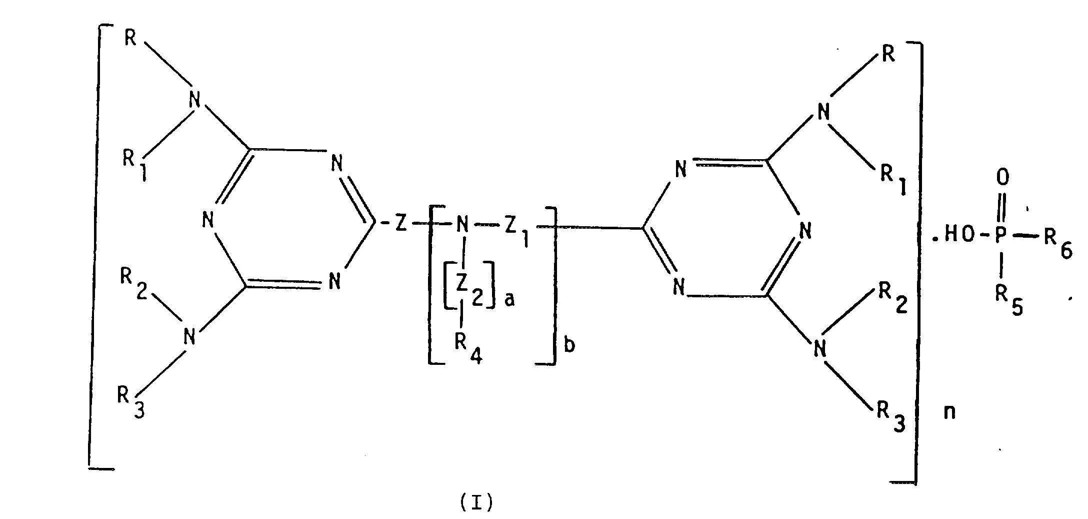 worin:
n, die Gruppen R bis R₆ und der Rest 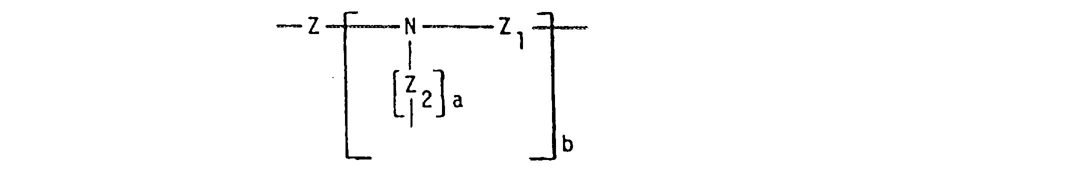 dieselben Bedeutungen wie in irgendeinem der Ansprüche 1 und 2 angegeben aufweisen.

8. Selbstverlöschende polymere Zusammensetzungen, umfassend:
(a) 90 bis 40 Gewichtsteile der Komponente (a) wie in Anspruch 6 definiert;
(b) 9 bis 35 Gewichtsteile der Komponente (b) wie in irgendeinem der Ansprüche 6 und 7 definiert;
(c) 1 bis 25 Gewichtsteile einer oder mehrerer Verbindungen, die aus Ammonium- und/oder Aminphosphaten und/oder -phosphonaten ausgewählt sind.

9. Zusammensetzungen nach Anspruch 8, in welchen die Ammoniumphosphate (c) die allgemeine Formel (NH₄)ₙ₊₂PₙO₃ₙ₊₁, worin n eine ganze Zahl von mindestens 2 ist; und/oder die allgemeine Formel (NH₄PO₃)ₙ, worin n im Bereich von 50 bis 500 liegt, aufweisen.

10. Zusammensetzungen nach Anspruch 8, in welchen die Aminphosphate (c) aus Dimethylammonium- oder Diethylammoniumphosphat; Ethylendiaminphosphat; Melaminortho-und -pyrophosphat und Mischungen davon ausgewählt sind.

11. Zusammensetzungen nach irgendeinem der Ansprüche 6 bis 10, worin Polymer (a) ausgewählt ist aus Acrylnitril/Butadien/Styrol-Terpolymeren (ABS); Acrylnitril/Styrol-Copolymeren (SAN); Polyurethanen; Polyethylenterephthalat; Polybutylenterephthalat; Polyamiden und Polymeren oder Copolymeren von Olefinen der allgemeinen Formel R'-CH=CH₂, worin R' ein Wasserstoffatom oder ein C₁-C₈-Alkyl- oder ein Arylrest ist; insbesondere:
(1) isotaktischem oder überwiegend isotaktischem Polypropylen;
(2) HDPE-, LLDPE- und LDPE-Polyethylen;
(3) kristallinen Copolymeren von Propylen und kleineren Mengen an Ethylen und/oder anderen alpha-Olefinen, wie beispielsweise 1-Buten, 1-Hexen, 1-Octen und 4-Methyl-1-penten;
(4) heterophasigen Zusammensetzungen, die umfassen (A) eine homopolymere Fraktion von Propylen und/oder eine Fraktion von einem der oben unter (3) angegebenen Copolymeren und (B) eine copolymere Fraktion, zusammengesetzt aus elastomeren Copolymeren von Ethylen mit einem alpha-Olefin, gegebenenfalls kleinere Mengen an Dien enthaltend, worin das alpha-Olefin vorzugsweise aus Propylen und 1-Buten ausgewählt ist; und
(5) elastomeren Copolymeren von Ethylen mit alpha-Olefinen, gegebenenfalls kleinere Mengen eines Diens enthaltend.

12. Formgegenstände, erhalten aus den Zusammensetzungen von irgendeinem der Ansprüche 6 bis 11.

## Revendications

1. Sels de dérivés de triazine et d'acides oxygénés dérivés du phosphore, correspondant à la formule générale (I) : 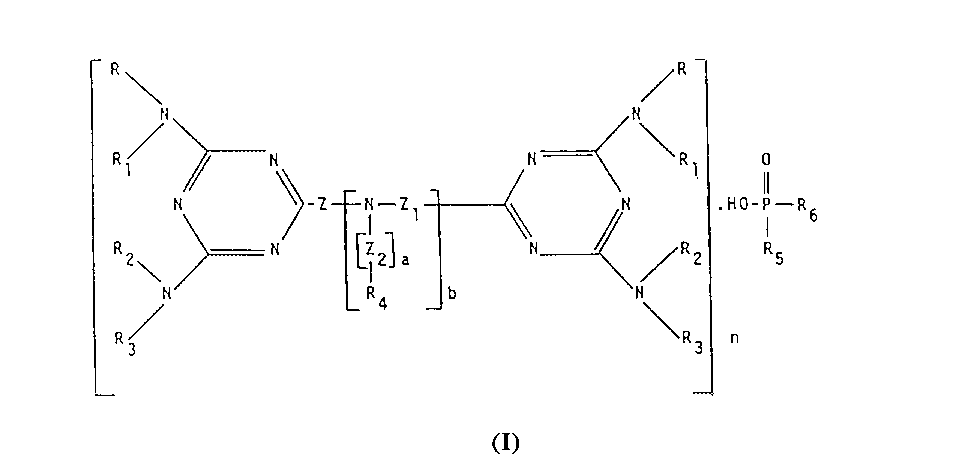 dans laquelle :
les radicaux symbolisés par R, R₁, R₂ et R₃, qui peuvent être identiques ou différents les uns des autres et qui peuvent être identiques sur les deux cycles triazine ou différents d'un cycle triazine à l'autre, sont choisis parmi l'atome d'hydrogène ; les groupes alkyle en C₁₋₁₈; les groupes alcényle en C₂₋₈; les groupes cycloalkyle en C₆₋₁₆ et alkylcycloalkyle en C₆-₁₆, portant éventuellement un ou plusieurs substituants hydroxyle et/ou hydroxyalkyle en C₁₋₄ ;
sous réserve que, si les radicaux bivalents ou polyvalents définis plus loin font respectivement partie de ceux de formule générale (III) ou (XII), la paire de radicaux R₅/R₆ définie plus loin n'est pas une paire H/OH;
-[CₘH₂ₘ]-O-R₇ ;
et 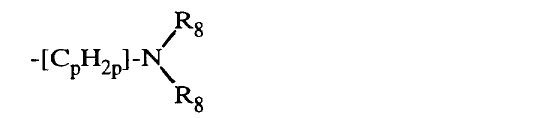 où :
m représente un nombre entier valant de 2 à 8 ;
p représente un nombre entier valant de 2 à 6 ;
R₇ représente un atome d'hydrogène, un groupe alkyle en C₁₋₈, un groupe alcényle en C₂₋₆ un groupe -[C_{q}H_{2q}]-O-R₉ (où q représente un nombre entier valant de 1 à 4 et R₉ représente un atome d'hydrogène ou un groupe alkyle en C₁₋₄), un groupe cycloalkyle en C₆₋₁₂ ou un groupe alkylcycloalkyle en C₆₋₁₂;
les radicaux symbolisés par R₈, qui peuvent être identiques ou différents l'un de l'autre, sont choisis parmi l'atome d'hydrogène et les groupes alkyle en C₁₋₈ alcényle en C₂₋₆, cycloalkyle en C₆₋₁₂ alkylcycloalkyle en C₆₋₁₂ et hydroxyalkyle en C₁₋₄, sous réserve que, si les radicaux bivalents ou polyvalents définis plus loin font respectivement partie de ceux de formule générale (III) ou (XII), la paire de radicaux R₅/R₆ définie plus loin n'est pas une paire H/OH ;
ou bien le fragment -N(R₈)₂ est remplacé par un groupe N-hétérocyclique choisi parmi les groupes aziridinyle, pyrrolidinyle, pipéridinyle, morpholinyle, thiomorpholinyle, pipérazinyle, 4-méthylpipérazinyle et 4-éthylpipérazinyle, et lié au groupe alkyle par l'atome d'azote ;
ou bien, dans la formule générale (I), au moins l'un des fragments NR₁ et NR₂R₃ est remplacé par un groupe N-hétérocyclique choisi parmi les groupes aziridinyle, pyrrolidinyle, pipéridinyle, morpholinyle, thiomorpholinyle, pipérazinyle, 4-méthylpipérazinyle, 4-éthylpipérazinyle, 2-méthylpipérazinyle, 2,5-diméthylpipérazinyle, 2,3,5,6-tétraméthylpipérazinyle, 2,2,5,5-tétraméthylpipérazinyle, 2-éthylpipérazinyle et 2,5-diéthylpipérazinyle, et lié au cycle triazine par l'atome d'azote ;
a vaut 0 ou 1 ;
b vaut 0 ou un nombre entier de 1 à 5 ;
R₄ représente un atome d'hydrogène ou un groupe de formule générale 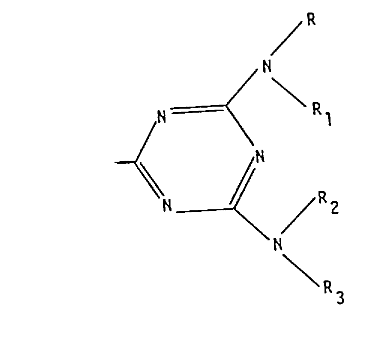 et sa signification peut différer d'un motif à l'autre ;
si b vaut 0:
Z est choisi parmi les radicaux divalents présentant les formules suivantes : 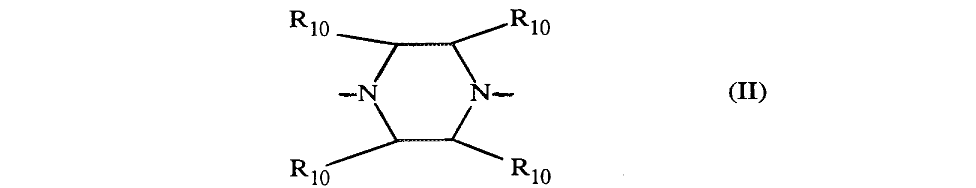 où les radicaux symbolisés par les R₁₀, qui peuvent être identiques ou différents les uns des autres, sont des atomes d'hydrogène ou des groupes alkyle en C₁₋₄, 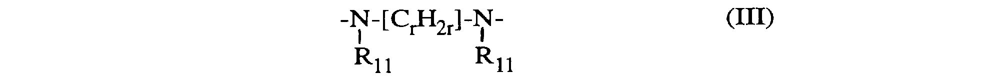 ou 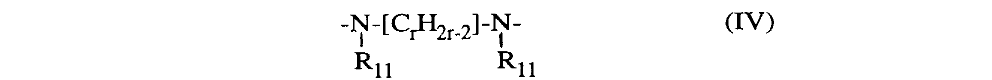 où r représente un nombre entier valant de 2 à 14 et R₁₁ représente un atome d'hydrogène ou un groupe alkyle en C₁₋₄, alcényle en C₂₋₆ ou hydroxyalkyle en C₁₋₄,
-NH-[(CH₂)ₛ-O]ₜ-(CH₂)ₛ-NH- (VI)
où s représente un nombre entier valant de 2 à 5 et t représente un nombre entier valant de 1 à 3, 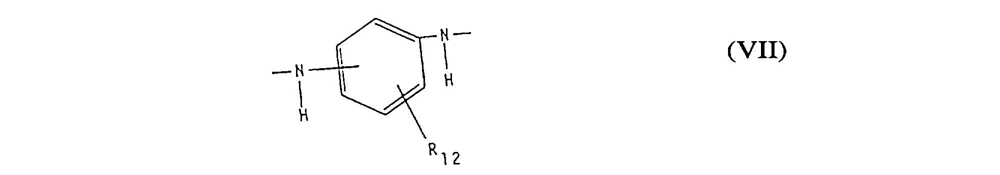 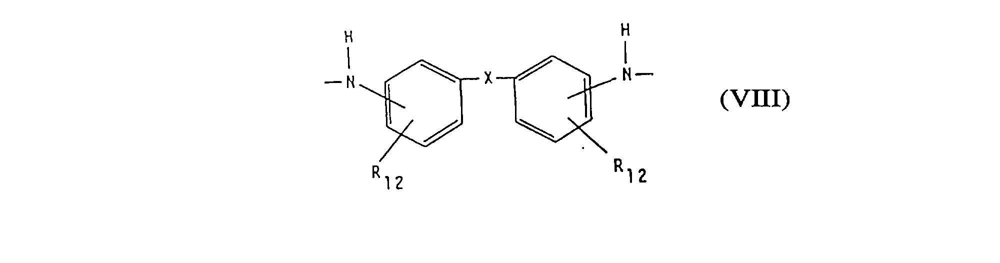 où X représente une liaison directe ou un chaînon -O-, -S-, -S-S-, -SO-, -NH-, -NHSO₂-, -NHCO-, -N=N- ou -CH₂-, et R₁₂ représente un atome d'hydrogène ou un groupe hydroxy, alkyle en C₁₋₄ ou alcoxy en C₁₋₄, 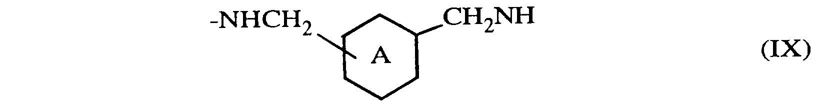 où A représente un cycle saturé ou insaturé, 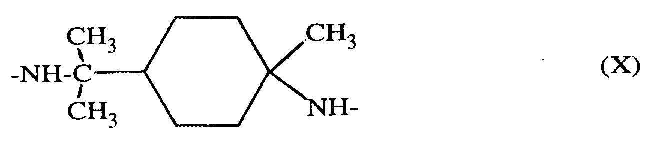 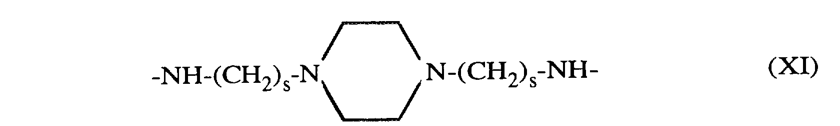 où s a la signification indiquée plus haut ;
si b représente un nombre entier valant de 1 à 5 : le fragment 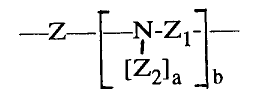 est un fragment polyvalent représenté par l'une des formules suivantes : 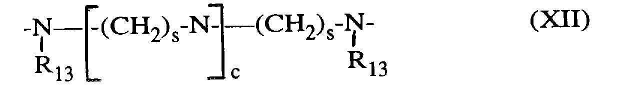 où R₁₃ représente un atome d'hydrogène ou un groupe alkyle en C₁₋₄, c représente un nombre entier valant de 1 à 5, et les indices s, qui peuvent être identiques ou différents les uns des autres, ont la signification indiquée plus haut, 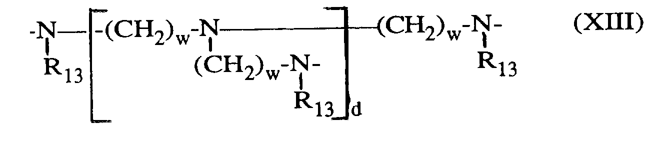 où R₁₃ a la signification indiquée plus haut, w représente un nombre entier valant de 2 à 4, et d vaut 1 ou 2 ;
n représente un nombre supérieur à 0 et valant au plus 3 ;
R₅ représente un atome d'hydrogène, un groupe hydroxy, un groupe alcoxy en C₁₋₈, un groupe aryloxy en C₆₋₁₂ portant éventuellement un substituant alkyle en C₁₋₈, un groupe aralkyle en C₇₋₁₂ portant éventuellement un substituant alkyle en C₁₋₄, un groupe alkyle en C₁₋₄ portant éventuellement un substituant carboxy, ou un groupe aryle en C₆₋₁₂ ;
R₆ représente un atome d'hydrogène, un groupe hydroxy, un groupe alcoxy en C₁₋₈, un groupe aryloxy en C₆₋₁₂ un groupe alkyle en C₁₋₄, un groupe aryle en C₆₋₁₂, un groupe de formule 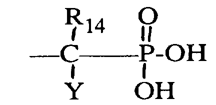 (où R₁₄ représente un atome d'hydrogène ou un groupe alkyle en C₁₋₁₂, et Y représente R₁₄ ou un groupe hydroxy), un groupe de formule 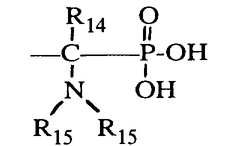 (où R₁₄ a la signification indiquée plus haut et les groupes symbolisés par R₁₅, qui peuvent être identiques ou différents l'un de l'autre, représentent chacun un atome d'hydrogène ou un groupe alkyle en C₁₋₄, ou bien le fragment -N(R₁₅)₂ est remplacé par un radical N-hétérocyclique qui comporte éventuellement un autre hétéroatome et qui est rattaché par l'atome d'azote à l'atome de carbone), un groupe de formule 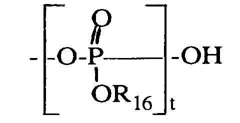 (où R₁₆ représente un atome d'hydrogène ou un groupe alkyle en C₁₋₈, et t représente un nombre entier valant de 1 à 3), un groupe de formule 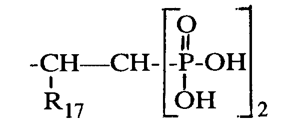 (où R₁₇ représente un atome d'hydrogène ou un groupe hydroxy), ou l'un des groupes dont les formules suivent : 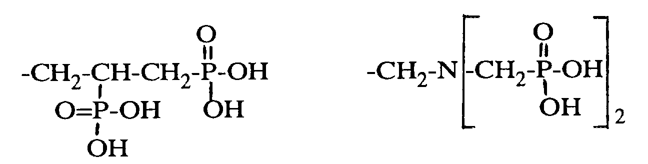 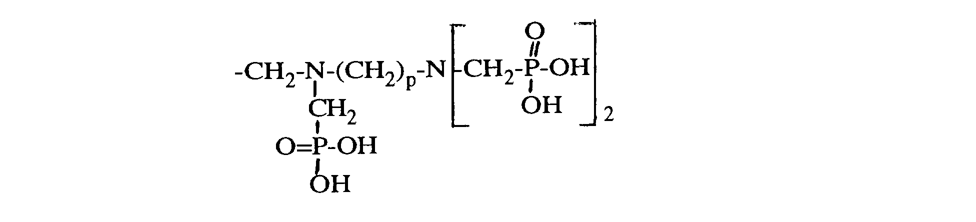 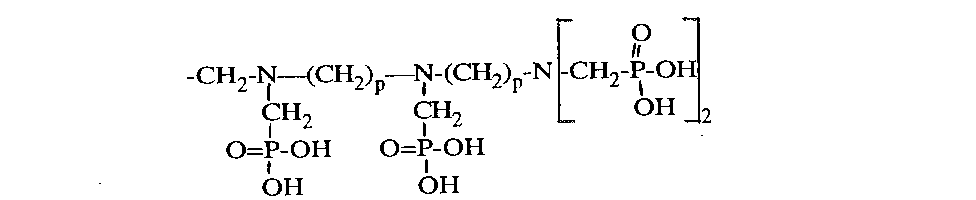 (où p représente un nombre entier valant de 2 à 6) ;
ou bien R₅ et R₆ forment conjointement une structure cyclique dont la formule est l'une des suivantes :

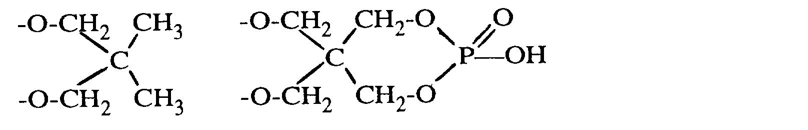

2. Sels conformes à la revendication 1, dans lesquels au moins l'un des groupes R, R₁, R₂ et R₃ de la formule générale (I) est remplacé par un groupe de formule -(CₘH₂ₘ)-O-R₇ où m représente un nombre entier valant de 2 à 4 et R₇ représente un atome d'hydrogène ou un groupe alkyle en C₁₋₄.

3. Sels conformes à la revendication 1 ou 2, dans lesquels l'acide dérivé du phosphore est choisi parmi les suivants : acide hypophosphoreux, acide phosphoreux, acide phosphorique, acide pyrophosphorique, acide tripolyphosphorique, acide éthane-1,1,2-triphosphonique, acide 2-hydroxyéthane-1,1,2-triphosphorique, acide propane-1,2,3-triphosphonique, acide isopropylphosphorique, acide n-butylphosphorique, acide di-isopropylphosphorique, acide di-n-butylphosphorique, acide di-n-pentylphosphorique, acide isooctylphosphorique, acide hexylphosphorique, acide 2-éthylhexylphosphorique, acide éthylphosphorique, acide méthylphosphonique, acide éthylphosphonique, acide n-propylphosphonique, acide n-butylphosphonique, acide aminométhylphosphonique, acide phénylphosphorique, acide phénylphosphonique, acide phénylphosphinique, acide di-n-butylpyrophosphorique, acide di-(2-éthylhexyl)pyrophosphorique, acide octylphénylphosphorique, acide 2-méthylbenzylphosphonique, acide 1-aminoéthane-1,1-di-phosphonique, acide 1-hydroxyéthane-1,1-diphosphonique, acide 1-hydroxydodécane-1,1-diphosphonique, acide 1-(N-méthylamino)éthane-1,1-diphosphonique, acide N,N-diméthylaminométhane- 1,1-diphosphonique, acide N-butylaminométhane- 1,1-diphosphonique, acide phosphonoacétique, acide 2-phosphonopropionique, acide 3-phosphonopropionique, acide 2-phosphonobutyrique, acide 4-phosphonobutyrique, 2-hydroxy-5,5-diméthyl-2-oxo-1,3,2-dioxophosphorinane, 3,9-dihydroxy-2,4,8,10-tétroxo-3,9-diphosphaspiro[5.5]undécano-3,9-dioxyde, acide amino-tris(méthylènephosphonique), acide éthylène-diamino-tétra(méthylènephosphonique), acide hexaméthylène-diamino-tétra(méthylènephosphonique), acide diéthylène-triamino-penta(méthylènephosphonique).

4. Procédé de préparation de sels de formule générale (I), conformes à l'une des revendications 1 à 3, qui comporte le fait de faire réagir n moles d'un dérivé de 2,4,6-triamino-1,3,5-triazine de formule générale (XIV) 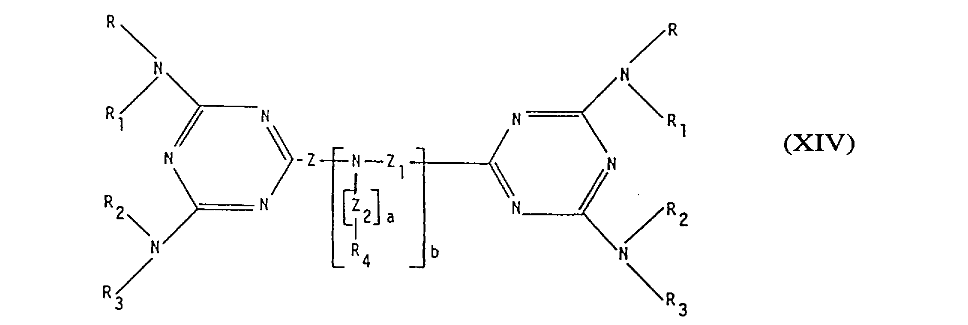 où n et les groupes symbolisés par R₁, R₂, R₃ et R₄, ainsi que le groupe de formule 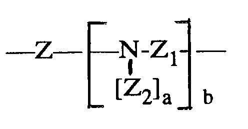 ont les définitions indiquées dans la revendication 1,
avec 1 mole d'un acide dérivé du phosphore, de formule générale (XV) : 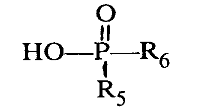 où R₅ et R₆ ont les significations indiquées dans la revendication 1.

5. Procédé conforme à la revendication 4, dans lequel on fait réagir le dérivé de formule générale (XIV) et l'acide dérivé du phosphore de formule générale (XV) en présence d'un solvant et à une température située entre 0°C et le point d'ébullition du solvant utilisé.

6. Compositions auto-extinguibles de polymères, comprenant
a) de 90 à 40 parties en poids d'un polymère thermoplastique et/ou d'un polymère possédant des propriétés d'élastomère, et
b) de 10 à 60 parties en poids d'un ou de plusieurs sels de dérivés de 2,4,6-triamino-1,3,5-triazine et d'acides oxygénés dérivés du phosphore, lesdits dérivés de 2,4,6-triamino-1,3,5-triazine correspondant à la formule générale (XIV) 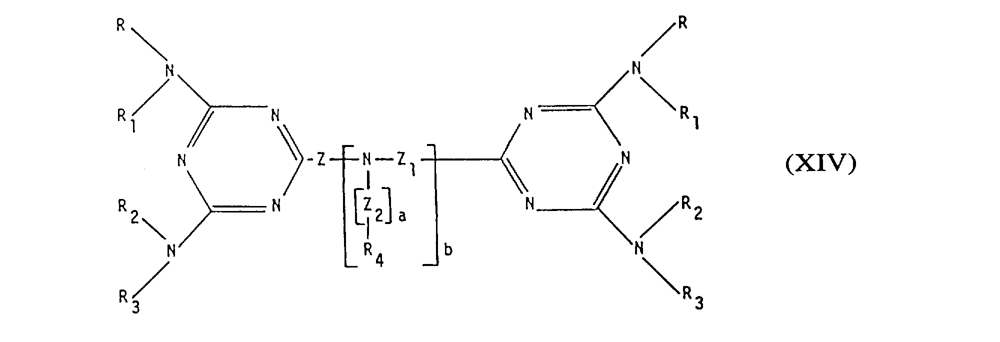 où n et les groupes symbolisés par R₁, R₂, R₃ et R₄, ainsi que le groupe de formule 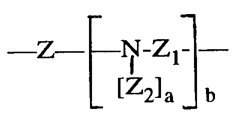 ont les définitions indiquées dans l'une des revendications 1 et 2.

7. Compositions conformes à la revendication 6, dans lesquelles le composant (b) est choisi parmi les sels de formule générale (I): 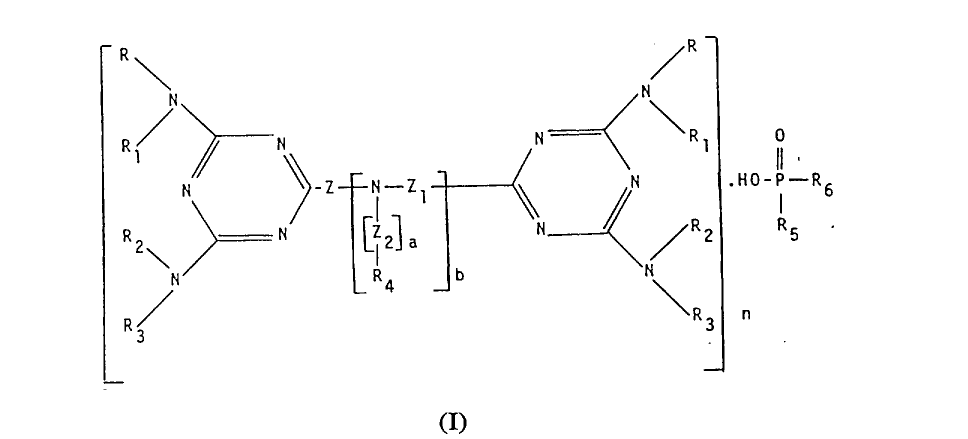 où n et les groupes symbolisés par R à R₆, ainsi que le groupe de formule 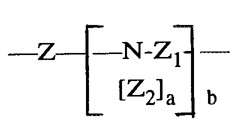 ont les définitions indiquées dans l'une des revendications 1 et 2.

8. Compositions auto-extinguibles de polymères, comprenant
a) de 90 à 40 parties en poids du composant (a) défini dans la revendication 6,
b) de 9 à 35 parties en poids du composant (b) défini dans l'une des revendications 6 et 7, et
c) de 1 à 25 parties en poids d'un ou de plusieurs composés choisis parmi les phosphates et/ou phosphonates d'ammonium et/ou d'amine.

9. Compositions conformes à la revendication 8, dans lesquelles les phosphates d'ammonium (c) correspondent à la formule générale (NH₄)ₙ₊₂PₙO₃ₙ₊₁ où n représente un nombre entier valant au moins 2, et/ou à la formule générale (NH₄PO₃)ₙ où n vaut de 50 à 500.

10. Compositions conformes à la revendication 8, dans lesquelles les phosphates d'amine (c) sont choisis parmi le phosphate de diméthylammonium, le phosphate de diéthylammonium, le phosphate d'éthylènediamine, l'orthophosphate de mélamine, le pyrophosphate de mélamine, et leurs mélanges.

11. Compositions conformes à l'une des revendications 6 à 10, dans lesquelles le polymère (a) est choisi parmi les terpolymères acrylonitrile/butadiène/styrène (ABS), les copolymères acrylonitrile/styrène (SAN), les polyuréthanes, le poly(éthylène téréphtalate), le poly(butylène téréphtalate), les polyamides, et les polymères et copolymères d'oléfines de formule générale R'-CH=CH₂ où R' représente un atome d'hydrogène ou un groupe alkyle en C₁₋₈ ou aryle, et en particulier :
1) du polypropylène isotactique ou à dominante isotactique,
2) du polyéthylène PEhd, PEbd ou PEbd1,
3) des copolymères cristallins du propylène et de quantités mineures d'éthylène et/ou d'autres α-oléfines, comme 1-butène, 1-hexène, 1-octène et 4-méthyl-1-pentène,
4) des compositions en phases hétérogènes, comprenant
(A) une fraction homopolymère de polypropylène et/ou une fraction de l'un des copolymères indiqués en (3) ci-dessus, et
(B) un fraction copolymère constituée de copolymères élastomères de l'éthylène et d'une α-oléfine, contenant éventuellement une petite quantité d'un diène, cette α-oléfine étant choisie de préférence parmi le propylène et le 1-butène, et
5) des copolymères élastomères de l'éthylène et d'α-oléfines, contenant éventuellement une petite quantité d'un diène.

12. Pièces moulées obtenues à partir des compositions conformes à l'une des revendications 6 à 11.
